# EUROPEAN PATENT SPECIFICATION

(11) **EP 1 720 871 B1**
(45) Date of publication and mention of the grant of the patent: **19.05.2010**
(21) Application number: 05717857.6
(22) Date of filing: 03.03.2005
(51) Int. Cl.: C07D 471/04, A61K 31/435, A61P 29/00, A61P 25/00

(54) **3,5-DISUBSTITUTED 1H-PYRROLO [2,3-B] PYRIDINES AS JNK INHIBITORS**
3,5-DISUBSTITUIERTE 1H-PYRROLO [2,3-B] PYRIDINE ALS JNK-INHIBITOREN
1H-PYRROLO [2,3-B] PYRIDINES 3,5 DISUBSTITUEES UTILISES COMME INHIBITEURS DE JNK

(30) Priority: 05.03.2004 GB 0405055
(43) Date of publication of application: 15.11.2006
(73) Proprietor: Eisai R&D Management Co., Ltd., Tokyo 112-8088 (JP)
(72) Inventor: MEDLAND, Darren, Peter Eisai London Res. Lab. Ltd., Gower Street London WC1E 6BT (GB); GRACZYK, Piotr, Pawel Eisai London Res. Lab. Ltd., Gower Street London WC1E 6BT (GB); BHATIA, Gurpreet, Singh Eisai London Res.Lab. Ltd., Gower Street London WC1E 6BT (GB)
(74) Representative: Crooks, Elizabeth Caroline
(86) International application number: PCT/GB2005/000779
(87) International publication number: WO 2005/085244

(56) References cited:
- WO-A-98/47899
- WO-A-03/082868
- WO-A-03/082869

## Description

The present invention relates to novel compounds that can be used in the inhibition of c-Jun N-terminal kinases, in medicine and particularly in the prevention and/or treatment of neurodegenerative disorders related to apoptosis and/or inflammation. The invention also provides processes for manufacture of said compounds, compositions containing them and processes for manufacturing such compositions.

c-Jun N-terminal kinases (hereinafter referred to as "JNKs") are members of the mitogen-activated protein kinase (MAPK) family. JNKs are involved in response to various stimuli, including proinflammatory cytokines and environmental stress. JNKs, and JNK3 in particular, play an important role during apoptotic death of cells and therefore have been implicated in various disorders including stroke, traumatic brain injury and other neurodegenerative diseases such as Parkinson disease, Alzheimer disease and others. Since JNK activity is a physiological regulator of AP-1 transcriptional activity, JNK inhibitors are expected to reduce inflammatory response.

Apoptosis is a form of cell death in which the cell actively participates in its own destruction in a process involving a characteristic series of biochemical and morphological changes, which are regulated by specific cell death genes. The apoptotic cell death is a process that has been observed in the developing mammalian nervous system. In mice, the inactivation by homologous recombination of genes that encode proteins that promote apoptosis, such as the caspase-3 or the Bax protein, prevents developmental neuronal cell death. The destruction of genes that encode cell death suppressors such as Bcl-x, leads to enhanced neuronal cell death. There is increasing evidence that apoptosis plays an important role in the pathology of acute and chronic neurodegenerative diseases. For example, in transgenic mice overexpressing the anti-apoptotic Bcl-2 protein in the nervous system there is a decrease in infarct volume following cerebral ischemia. Similarly, injection of the caspase inhibitor BAF reduces neuronal cell death following hypoxia/ischaemia in neonatal rats. Another example is spinal muscular atrophy (a motor neuron disease) where loss of function mutations in the SMN gene is associated with the disease. Recent data has shown that the wild type SMN protein binds to Bcl-2 and cooperates with it to inhibit apoptosis. These results suggest that inhibitors of neuronal apoptosis could be beneficial in the treatment of human neurodegenerative diseases. There is increasing evidence that neuronal apoptosis is an important pathological feature of stroke, traumatic brain injury and other neurodegenerative diseases. Therefore, pharmacotherapy using inhibitors of neuronal apoptosis may provide a therapeutic benefit in neurodegenerative conditions.

A number of groups have studied the mechanisms of neuronal cell death using in vitro cell culture systems and the results suggest that in some systems the transcription factor c-Jun is activated by the removal of survival signals and promotes cell death.

Antibodies specific for c-Jun protected NGF-deprived rat sympathetic neurones from apoptosis. Analogous neuroprotection due to expression of a c-Jun dominant negative mutant has been demonstrated, whereas overexpression of wild type c-Jun protein was sufficient to induce apoptosis in the presence of NGF. Estus and co-workers recently showed that an increase in c-Jun RNA levels occurs in cortical neurones undergoing apoptosis after treatment with β-amyloid peptide. It has also been shown that c-Jun is required for apoptosis in cerebellar granule neurones deprived of survival signals. c-Jun is activated by JNKs, which phosphorylate its transcriptional activation domain. In humans there are three JNK genes: JNK1, JNK2 and JNK3. The RNAs encoding JNK1 and JNK2 are expressed in many tissues, including the brain, but JNK3 is restricted to the nervous system and to a smaller extent the heart and testes.

JNKs are strongly activated in cellular responses to various stresses such as UV radiation, heat shock, osmotic shock, DNA-damaging agents, and proinflammatory cytokines such as TNFα, IL-1β and others. Upstream regulators of the JNK pathway include kinases such as SEK1, MKK7 and MEKK1. There is evidence that Jun kinase activity is required for neuronal apoptosis in vitro. Overexpression of MEKK1 in sympathetic neurones increased c-Jun protein levels and phosphorylation and induced apoptosis in the presence of NGF indicating that activation of the Jun kinase pathway can trigger neuronal cell death. The Jun kinase pathway has been shown to be necessary for the death of differentiated PC12 cells deprived of NGF. Furthermore, compound CEP-1347, which inhibits the c-Jun pathway (upstream of Jun kinase), protects motor neurones against cell death induced by survival factor withdrawal.

In JNK3 homozygous (-/-) knockout mice, epileptic seizures and death of hippocampal CA3 neurones induced by injection of kainic acid is blocked. This indicates that JNK3 is involved in certain forms of neuronal cell death in vivo. It is also a critical component of GluR6-mediated excitotoxicity. Furthermore, JNK3 (-/-) mice appear to develop normally and are viable suggesting that JNK3 is not essential for development or viability.

Strong nuclear JNK3 immunoreactivity in the brain CA1 neurones of patients with acute hypoxia suggests that JNK3 is involved in hypoxia-related neurodegeneration. Transient hypoxia may also trigger apoptosis through JNK signaling pathway in developing brain neurones.

Furthermore, JNK3 immunoreactivity is colocalized with Alzheimer disease-affected neurones. Moreover JNK3 is related to neurofibrillary pathology of Alzheimer disease. In particular, JNK3 induces robust phosphorylation of amyloid precursor protein (APP) thus affecting its metabolism in disease state.

WO 03/082869 discloses derivatives of 1H-pyrrole[2,3-b]pyridine substituted at the 5-position only for use as JNK inhibitors.

WO 03/082868 discloses derivatives of 1H-pyrrole[2,3-b]pyridine substituted at the 2-,3- and 5-position for use as JNK inhibitors.

The present inventors have provided compounds, which are inhibitors of c-Jun N-terminal kinases.

The first aspect of the invention therefore relates to a compound of formula (I) as illustrated below: 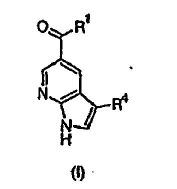 wherein R¹ is a C₃₋₁₂ carbocyclyl or C₃₋₁₂ heterocyclyl group or a group of formula (II) 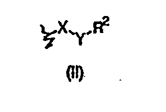
wherein X is NR³, O, S or (CR²²R²²)ₙ, Y is absent or is NR²³, O, or (CR²³R²³)ₙ, R² is optionally substituted C₁₋₁₂ alkyl, C₂₋₁₂ alkenyl, C₂₋₁₂ alkynyl, C₃₋₁₂ carbocyclyl or C₃₋₁₂ heterocyclyl, and R⁴ is an optionally substituted five or six membered heterocyclyl group or an optionally substituted six membered carbocyclyl group;
wherein the C₃₋₁₂ carbocyclyl or C₃₋₁₂ heterocyclyl group of R¹ is optionally fused to a partially saturated, unsaturated or fully saturated five to seven membered ring containing zero to three heteroatoms, and each substitutable carbon atom in R¹, including the optional fused ring, is optionally and independently substituted by one or more of halogen, C₁₋₁₂ alkyl, C₂₋₁₂ alkenyl, C₂₋₁₂ alkynyl, haloC₁₋₁₂alkyl, C₃₋₁₂ carbocyclyl, C₃₋₁₂ heterocyclyl, (CH₂)ₙOR⁵, (CH₂)ₙNR⁵₂(CH₂)ₙSR⁵, OR⁵, SR⁵, NO₂, CN, NR⁵₂, NR⁵COR⁵, NR⁵CONR⁵₂, NR⁵COR⁵, NR⁵CO₂R⁵, CO₂R⁵, COR⁵, CONR⁵₂, S(O)₂R⁵, SONR⁵2, S(O)R⁵, SO₂NR⁵₂, or NR⁵S(O)₂R⁵ wherein the C₁₋₁₂ alkyl group optionally contains one or more insertions selected from -O-, -N(R⁵)- -S-, - S(O)- and -S(O₂)-; and each saturated carbon in the optional fused ring is further optionally and independently substituted by =O, =S, NNR⁶₂, =N-OR⁶, =NNR⁶COR⁶, =NNR⁶CO₂R⁶, =NNSO₂R⁶, or =NR⁶; and each substitutable nitrogen atom in R¹ is optionally substituted by R⁷, COR⁷, SO₂R⁷ or CO₂R⁷; wherein n is 1 to 6, preferably n is 1, 2 or 3;
wherein R⁵ is hydrogen , C₁₋₁₂ alkyl, C₃₋₁₂ carbocyclyl or C₃₋₁₂ heterocyclyl, optionally substituted by one or more of C₁₋₆ alkyl, C₃₋₁₂ carbocyclyl, C₃₋₁₂ heterocyclyl, halogen, C₁₋₆ haloalkyl, OR⁸, SR³, NO₂, CN, NR⁸R⁸, NR⁸COR⁸, NR⁸CONR⁸R⁸, NR⁸COR³, NR⁸CO₂R⁸, CO₂R⁸, COR⁸, CONR⁸₂, S(O)₂R⁸, SONR⁸₂, S(O)R⁸, SO₂NR⁸R⁸, MR⁸S(O)₂R⁸, wherein the C₁₋₁₂ alkyl group optionally incorporates one or two insertions selected from the group consisting of -O-, -N(R⁸)-, -S(O)- and -S(O₂)-, wherein each R⁸ may be the same or different and is as defined below;
wherein two R⁵ in NR⁵₂ may optionally form a partially saturated, unsaturated or fully saturated three to seven membered ring containing one to three heteroatoms, optionally and independently substituted by one or more of C₁₋₆ alkyl, halogen, C₁₋₆ haloalkyl, OR⁸, SR⁸, NO₇, CN, NR⁸R⁵, NR⁸COR⁸, NR⁸CONR⁸R⁸, NR⁸COR⁸, NR⁸CO₂R⁸, CO₂R⁸, COR⁸, CONR⁸₂, S(O)₂R⁸, SONR⁸₂, S(O)R⁸, SO₂NR⁸R⁸, NR⁸S(O)₂R⁸,
   wherein the C₁₋₆ alkyl group optionally incorporates one or two insertions selected from the group consisting of -O-, -N(R⁸)-. -S(O)- and -S(O₂)-, wherein each R⁸ may be the same or different and is as defined below;
wherein R⁶ is hydrogen, C₁₋₁₂ alkyl, C₃₋₁₂ carbocyclyl or C₃₋₁₂ heterocyclyl, optionally substituted by one or more of C₁₋₆ alkyl, halogen, C₁₋₆ haloalkyl, OR⁸, SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S(O)₂R⁸, S(O)R⁸, SO₂NR⁸R⁸, NR⁸S(O)₂R⁸, wherein the C₁₋₁₂ alkyl group optionally incorporates one or two insertions selected from the group consisting of -O-, -N(R⁸)-, -S(O)- and -S(O₂)-, wherein each R⁸ may be the same or different and is as defined below;
wherein R⁷ is hydrogen, C₆₋₁₂ aryl, C₁₋₆ alkyl or C₁₋₆ haloalkyl;
wherein R⁸ is hydrogen, C₁₋₆ alkyl, or C₁₋₆ haloalkyl;
Y is absent or is NR²³, O, or (CR²³R²³)ₙ, wherein each R²³ may be the same or different and is H, C₁₋₄ alkyl, C₁₋₄ alkoxy or C₁₋₄ haloalkyl;
   and n is 1 to 6, preferably n is 1, 2, 3 or 4.

R² is C₁₋₁₂ alkyl, C₂₋₁₂ alkenyl, C₂₋₁₂ alkynyl, C₃₋₁₂ carbocyclyl or C₃₋₁₂ heterocyclyl, each of which is optionally substituted, wherein:
the optionally substituted carbocyclyl or heterocyclyl group is optionally fused to one to three unsaturated, partially unsaturated or fully saturated five to seven membered rings containing zero to three heteroatoms;
each substitutable carbon atom in R², including the optional fused ring, is optionally and independently substituted by one or more of C₁₋₁₂ alkyl, C₃₋₁₂ cycloalkyl, C₃₋₁₂ heterocycloalkyl, C₃₋₁₂ aryl, C₃₋₁₂ heteroaryl halogen, C₁₋₁₂ haloalkyl, OR⁹, SR⁹, NO₂, CN, NR⁹R⁹, NR⁹COR⁹, NR⁹CONR⁹R⁹, NR⁹COR⁹, NR⁹CO₂R⁹, CO₂R⁹, COR⁹, CONR⁹R⁹, S(O)₂R⁹, SONH₂, S(O)R⁹, SO₂NR⁹R⁹, NR⁹S(O)₂R⁹, wherein each R⁹ may be the same or different and is as defined below and wherein:
   the C₁₋₁₂ alkyl group optionally incorporates one or two insertions selected from the group consisting of -O-, -C(O)-, -N(R⁹)-, -S(O)- and -S(O₂)-, wherein each R⁹ may be the same or different and is as defined above;
   the C₁₋₁₂ alkyl, C₃₋₁₂ cycloalkyl, C₃₋₁₂ heterocycloalkyl, C₃₋₁₂ aryl, or C₃₋₁₂ heteroaryl groups are optionally substituted by one or more of halogen, C₁₋₁₂ haloalkyl, OR⁹, SR⁹, NO₂, CN, NR⁹R⁹, NR⁹COR⁹, NR⁹CONR⁹R⁹, NR⁹COR⁹, NR⁹CO₂R⁹, CO₂R⁹, COR⁹, CONR⁹R⁹, S(O)₂R⁹, SONH₂, S(O)R⁹, SO₂NR⁹R⁹, NR⁹S(O)₂R⁹, wherein each R⁹ may be the same or different and is as defined below; and
   the C₃₋₁₂ cycloalkyl, C₃₋₁₂ heterocycloalkyl, C₃₋₁₂ aryl, or C₃₋₁₂ heteroaryl groups are optionally substituted by one or more C₁₋₁₂ alkyl groups;
   each saturated carbon in R², including the optional fused ring, is further optionally and independently substituted by =O, =S, NNR⁹R⁹, =N-OR⁹, =NNHCOR⁹, =NNHCO₂R⁹, =NNSO₂R⁹, or =NR⁹, wherein each R⁹ may be the same or different and is as defined below; and
   each substitutable nitrogen atom in R² is optionally substituted by R¹⁰, COR⁹, SO₂R⁹ or CO₂R⁹ wherein each R⁹ and R¹⁰ may be the same or different and is as defined below;
wherein two R⁹ in NR⁹₂ may optionally form a partially saturated, unsaturated or fully saturated three to seven membered ring containing one to three heteroatoms, optionally and independently substituted by one or more of C₁₋₆ alkyl, halogen, C₁₋₆ haloalkyl, OR¹¹, SR¹¹, NO₂, CN, NR¹¹R¹¹, NR¹¹COR¹¹, NR¹¹CONR¹¹R¹¹, NR¹¹COR¹¹, NR¹¹CO₂R¹¹, CO₂R¹¹, COR¹¹, CONR¹¹₂, S(O)₂R¹¹, SONR¹¹₂, S(O)R¹¹, SO₂NR¹¹R¹¹, NR¹¹S(O)₂R¹¹,
wherein the C₁₋₆ alkyl group optionally incorporates , one or two insertions selected from the group consisting of -O-, -N(R¹¹)-, -S(O)- and -S(O₂)-, wherein each R¹¹ may be the same or different and is as defined below; wherein R¹¹ is hydrogen, C₁₋₆ alkyl, or C₁₋₆ haloalkyl;
wherein R⁹ is hydrogen , C₁₋₁₂ alkyl or C₃₋₁₂, aryl, optionally substituted by one or more of C₁₋₄ alkyl, halogen, C₁₋₄ haloalkyl, OR¹², SR¹², NO₂, CN, NR¹²R¹², NR¹²COR¹², NR¹²CONR¹²R¹², NR¹²COR¹², NR¹²CO₂R¹², CO₂R¹², COR¹², CONR¹²₂, S(O)₂R¹², SONH₂, S(O)R¹², SO₂ NR¹²R¹², NR¹²S(O)₂R¹², wherein the C₁₋₁₂ alkyl group optionally incorporates one or two insertions selected from the group consisting of -O-, -N(R¹²)-, -S(O)- and -S(O₂)-, wherein each R¹² may be the same or different and is as defined below;
wherein R¹⁰ is C₁₋₁₂ alkyl or C₃₋₁₂ aryl, optionally substituted by one or more of C₁₋₄ alkyl, halogen, C₁₋₄haloalkyl, OR¹², SR¹², NO₂, CN, NR¹²R¹², NR¹²COR¹², NR¹²CONR¹²R¹², NR¹²COR¹², NR¹²CO₂R¹², CO₂R¹², COR¹², CONR¹²₂, S(O)₂R¹², SONH₂, S(O)R¹², SO₂NR¹²R¹², NR¹²S(O)₂R¹², wherein the C₁₋₁₂ alkyl group optionally incorporates one or two insertions selected from the group consisting of -O-, -N(R¹²)-, -S(O)- and -S(O₂)-, wherein each R¹² may be the same or different and is as defined below;
wherein R¹² is hydrogen, C₁₋₄ alkyl, or C₁₋₄ haloalkyl;
X is NR³; O, S or (CR²²R²²)ₙ wherein R²² is independently one or more of halogen, C₁₋₁₂ alkyl, C₂₋₁₂ alkenyl, C₂₋₁₂ alkynyl, C₁₋₁₂ haloalkyl, C₆₋₁₂ carbocyclyl, C₅₋₁₂ heterocyclyl, (CH₂)ₙOR⁵, (CH₂)ₙNR⁵₂, OR⁵, SR⁵, NO₂, CN, NR⁵₂, NR⁵COR⁵, NR⁵CONR⁵₂, NR⁵COR⁵, NR⁵CO₂R⁵, CO₂R⁵, COR⁵, CONR⁵₂, S(O)₂R⁵, SONR⁵₂, S(O)R⁵, SO₂NR⁵₂, or NR⁵S(O)₂R⁵ wherein each R⁵ may be the same or different and is as defined above; and
wherein n is 1 to 6, preferably n is 1, 2, 3 or 4;
wherein R³ is a lone electron pair, hydrogen, C₁₋₁₂ alkyl, C₂₋₁₂ alkenyl, C₂₋₁₂ alkynyl, C₃₋₁₂ carbocyclyl or C₃₋₁₂ heterocyclyl, each of which is optionally substituted, wherein:
   the optionally substituted carbocyclyl or heterocyclyl group is optionally fused to one to three unsaturated, partially unsaturated or fully saturated five to seven membered rings containing zero to three heteroatoms,
   each substitutable carbon atom in R³, including the optional fused ring, is optionally and independently substituted by one or more of C₁₋₁₂ alkyl, C₃₋₁₂ cycloalkyl, C₃₋₁₂ heterocycloalkyl, C₃₋₁₂ aryl, C₃₋₁₂ heteroaryl halogen, C₁₋₁₂ haloalkyl, OR¹³, SR¹³, NO₂, CN, NR¹³R¹³, NR¹³COR¹³, NR¹³CONR¹³R¹³, NR¹³COR¹³, NR¹³CO₂R¹³, CO₂R¹³, COR¹³, CONR¹³R¹³, S(O)₂R¹³, SONH₂, S(O)R¹³, SO₂NR¹³R¹³, NR¹³S(O)₂R¹³, wherein each R¹³ may be the same or different and is as defined above and wherein:
      the C₁₋₁₂ alkyl group optionally incorporates one or two insertions selected from the group consisting of -O-, -C(O)-, -N(R¹³)-, -S(O)- and -S(O₂)-, wherein each R¹³ may be the same or different and is as defined above;
      the C₁₋₁₂ alkyl, C₃₋₁₂ cycloalkyl, C₃₋₁₂ heterocycloalkyl, C₃₋₁₂ aryl, or C₃₋₁₂ heteroaryl groups are optionally substituted by one or more of halogen, C₁₋₁₂ haloalkyl, OR¹³, SR¹³, NO₂, CN, NR¹³R¹³, NR¹³COR¹³, NR¹³CONR¹³R¹³, NR¹³COR¹³ NR¹³CO₂R¹³, CO₂R¹³, COR¹³, CONR¹³R¹³ S(O)R¹³ SONH₂, S(O)R¹³, SO₂NR¹³R¹³, NR¹³S(O)₂R¹³, wherein each R¹³ may be the same or different and is as defined below; and
      the C₃₋₁₂ cycloalkyl, C₃₋₁₂ heterocycloalkyl, C₃₋₁₂ aryl, or C₃₋₁₂ heteroaryl groups are optionally substituted by one or more C₁₋₁₂ alkyl groups;
   each saturated carbon in R², including the optional fused ring, is further optionally and independently substituted by =O, =S, NNR¹³R¹³, =N-OR¹³, =NNHCOR¹³, =NNHCO₂R¹³, =NNSO₂R¹³, or =NR¹³, wherein each R¹³ may be the same or different and is as defined below; and
   each substitutable nitrogen atom in R³ is optionally substituted by R¹⁴, COR¹³, SO₂R¹³ or CO₂R¹³ wherein each R¹³ and R¹⁴ may be the same or different and is as defined below;
   wherein two R¹³ in NR¹³₂ may optionally form a partially saturated, unsaturated or fully saturated three to seven membered ring containing one to three heteroatoms, optionally and independently substituted by one or more of C₁₋₆ alkyl, halogen, C₁₋₆ haloalkyl, OR¹⁵, SR¹⁵, NO₂, CN, NR¹⁵R¹⁵, NR¹⁵COR¹⁵, NR¹⁵CONR¹⁵R¹⁵, NR¹⁵COR¹⁵, NR¹⁵CO₂R¹⁵, CO₂R¹⁵, COR¹⁵, CONR¹⁵₂, S(O)₂R¹⁵, SONR¹⁵₂, S(O)R¹⁵, SO₂NR¹⁵R¹⁵, NR¹⁵S(O)₂R¹⁵,
   wherein the C₁₋₆ alkyl group optionally incorporates one or two insertions selected from the group consisting of -O-, -N(R¹⁵)-, -S(O)- and -S(O₂)-, wherein each R¹⁵ may be the same or different and is as defined below; wherein R¹⁵ is hydrogen, C₁₋₆ alkyl, or C₁₋₆ haloalkyl;
   wherein R¹³ is hydrogen , C₁₋₁₂ alkyl or C₃₋₁₂ aryl, optionally substituted by one or more of C₁₋₄ alkyl, halogen, C₁₋₄ haloalkyl, OR¹⁶, SR¹⁶, NO₂, CN, NR¹⁶R¹⁶, NR¹⁶COR¹⁶, NR¹⁶CONR¹⁶R¹⁶, NR¹⁶COR¹⁶, NR¹⁶CO₂R¹⁶, CO₂R¹⁶, COR¹⁶, CONR¹⁶₂, S(O)₂R¹⁶, SONH₂, S(O)R¹⁶, SO₂ NR¹⁶R¹⁶, NR¹⁶S(O)₂R¹⁶, wherein the C₁₋₁₂ alkyl group optionally incorporates one or two insertions selected from the group consisting of -O-, -N(R¹⁶)-, -S(O)- and -S(O₂)-, wherein each R¹⁶ may be the same or different and is as defined below;
   wherein R¹⁴ is C₁₋₁₂ alkyl or C₃₋₁₂ aryl, optionally substituted by one or more of C₁₋₄ alkyl, halogen, C₁₋₄ haloalkyl, OR¹⁶, SR¹⁶, NO₂, CN, NR¹⁶R¹⁶, NR¹⁶COR¹⁶, NR¹⁶CONR¹⁶R¹⁶, NR¹⁶COR¹⁶, NR¹⁶CO₂R¹⁶, CO₂R¹⁶, COR¹⁶, CONR¹⁶₂, S(O)₂R¹⁶, SONH₂, S(O)R¹⁶, SO₂NR¹⁶R¹⁶, NR¹⁶S(O)₂R¹⁶, wherein the C₁₋₁₂ alkyl group optionally incorporates one or two insertions selected from the group consisting of -O-, -N(R¹⁶)-, -S(O)- and -S(O₂)-, wherein each R¹⁶ may be the same or different and is as defined below;
   wherein R¹⁶ is hydrogen, C₁₋₄ alkyl, or C₁₋₄ haloalkyl;
   wherein when X is NR², R² and R³ can form a 3 to 12 membered heterocyclyl ring, more preferably a 5, 6, 7, 8, 9, 10, 11 or 12 membered ring, wherein said ring can be partially saturated, unsaturated or fully saturated containing one to three heteroatoms; wherein the heterocyclyl group formed by R² and R³ can be optionally fused to one to three unsaturated, partially saturated or fully saturated 5 to 7 membered rings and contains from zero to three heteroatoms, any of said rings being optionally and independently substituted with one or more of C₁₋₆ alkyl, halogen, C₁₋₆ haloalkyl, OR²², SR²², NO₂, CN, NR²²R²², NR²²COR²², NR²²CONR²²R²², NR²²COR²², NR²²CO₂R²², CO₂R²², COR²², CONR²²₂, S(O)R²², SONR²²₂, S(O)R²², SO₂NR²²R²², NR²²S(O)₂R²², wherein the C₁₋₆ alkyl group optionally incorporates one or two insertions from -0-, - N(R²²)-, -S(O)- and -S(O₂)- and wherein each R²² may be the same or different;
   and wherein R⁴ is a six-membered carbocyclyl group or a five or six-membered heterocyclyl group containing from 1 to 4 heteroatoms independently selected from N, S or O, wherein the optionally substituted six-membered carbocyclyl or five or six-membered heterocyclyl group is optionally fused to a partially saturated, unsaturated or fully saturated five to seven membered ring containing zero to three heteroatoms, and each substitutable carbon or hetero-atom in R⁴ including the optional fused ring, is optionally and independently substituted by one or more of halogen, C₁₋₁₂ alkyl, C₂₋₁₂ alkenyl, C₂₋₁₂ alkynyl, C₁₋₁₂ haloalkyl, C₃₋₁₂ carbocyclyl, C₃₋₁₂ heterocyclyl, (CH₂)ₙOR¹⁷, (CH₂)ₙNR¹⁷₂, OR¹⁷, SR¹⁷, NO₂, CN, NR¹⁷₂, NR¹⁷COR¹⁷, NR¹⁷CONR¹⁷₂, NR¹⁷COR¹⁷, NR¹⁷CO₂R¹⁷ CO₂R¹⁷, COR¹⁷, CONR¹⁷₂, S(O)₂R¹⁷, SONR¹⁷₂, S(O)R¹⁷, SO₂NR¹⁷₂, or NR¹⁷S(O)₂R¹⁷, wherein the C₁₋₁₂ alkyl group optionally contains one or more insertions selected from -O-, -N(R¹²)- -S-, -S(O)- and -S(O₂)-; and each saturated carbon in the optional fused ring is further optionally and independently substituted by =O, =S, NNR¹⁸₂, =N-OR¹⁸, =NNR¹⁸COR¹⁸, =NNR¹⁸CO₂R¹⁸, =NNSO₂R¹⁸, or =NR¹⁸; and each substitutable nitrogen atom in R⁴ is optionally substituted by R¹⁹, COR¹⁹, SO₂R¹⁹ or CO₂R¹⁹; wherein n is 1 to 6, preferably n is 1, 2 or 3; preferably, wherein each substitutable carbon or hetero-atom in R⁴ is optionally and independently substituted by one or more of C₁₋₆ alkyl, OR²⁰, SR²⁰, NO₂, CN, NR²⁰₂, NR²⁰COR²⁰, NR²⁰CONR²⁰₂, NR²⁰COR²⁰, NHCO₂R²⁰, CO₂R²⁰, COR²⁰, CONR²⁰, S(O)₂R²⁰, SONR²⁰₂, S(O)R²⁰, SO₂NR²⁰₂, or NR²⁰S(O)₂R²⁰;
   wherein R²⁰ is hydrogen, C₁₋₆ alkyl, or C₁₋₆ haloalkyl;
   wherein R¹⁷ is hydrogen , C₁₋₁₂ alkyl, C₃₋₁₂ carbocyclyl or C₃₋₁₂ heterocyclyl, optionally substituted by one or more of C₁₋₆ alkyl, C₃₋₁₂ carbocyclyl, C₃₋₁₂ heterocyclyl, halogen, C₁₋₆ haloalkyl, OR²¹, SR²¹, NO₂, CN, NR²¹R²¹, NR²¹COR²¹, NR²¹CONR²¹R²¹, NR²¹COR²¹, NR²¹CO₂R²¹, CO₂R²¹, COR²¹, CONR²¹₂, S(O)₂R²¹, SONR²¹₂, S(O)R²¹, SO₂NR²¹R²¹, NR²¹S(O)₂R²¹, wherein the C₁₋₁₂ alkyl group optionally incorporates one or two insertions selected from the group consisting of -O-, -N(R²¹)-, -S(O)- and -S(O₂)-, wherein each R²¹ may be the same or different and is as defined below;
   wherein two R¹⁷ in NR¹⁷₂ may optionally form a partially saturated, unsaturated or fully saturated three to seven membered ring containing one to three heteroatoms, optionally and independently substituted by one or more of C₁₋₆ alkyl, halogen, C₁₋₆ haloalkyl, OR²¹, SR²¹, NO₂, CN, NR²¹R²¹, NR²¹COR²¹, NR²¹CONR²¹R²¹, NR²¹COR²¹, NR²¹CO₂R²¹, CO₂R²¹, COR²¹, CONR²¹₂, S(O)₂R²¹, SONR²¹₂, S(O)R²¹, SO₂NR²¹R²¹, NR²¹S(O)₂R²¹, wherein the C₁₋₆ alkyl group optionally incorporates one or two insertions selected from the group consisting of -O-, -N(R²¹)-, -S(O)- and -S(O₂)-, wherein each R²¹ may be the same or different and is as defined below;
   wherein R¹⁸ is hydrogen, C₁₋₁₂ alkyl, C₃₋₁₂ carbocyclyl or C₃₋₁₂ heterocyclyl, optionally substituted by one or more of C₁₋₆ alkyl, halogen, C₁₋₆ haloalkyl, OR²¹, SR²¹, NO₂, CN, NR²¹R²¹, NR²¹COR²¹, NR²¹CONR²¹R²¹, NR²¹COR²¹, NR²¹CO₂R²¹, CO₂R²¹, COR²¹, CONR²¹₂, S(O)₂R²¹, S(O)R²¹, SO₂NR²¹R²¹, NR²¹S(O)₂R²¹, wherein the C₁₋₁₂ alkyl group optionally incorporates one or two insertions selected from the group consisting of -O-, -N(R²¹)-, -S(O)- and-S(O₂)-, wherein each R²¹ may be the same or different and is as defined below;
   wherein R¹⁹ is hydrogen, C₆₋₁₂ aryl, C₁₋₆ alkyl or C₁₋₆ haloalkyl;
      wherein R²¹ is hydrogen, C₁₋₆ alkyl, or C₁₋₆ haloalkyl;
   and the pharmaceutically acceptable salts thereof.

For the avoidance of doubt when a group as defined above contains two or more radicals eg the radical R²¹ as for example in the groups SO₂NR²¹R²¹ and NR³COR³, the two or more radicals i.e. R²¹ may be the same or different.

For the purposes of this invention, alkyl relates to both straight chain and branched alkyl radicals of 1 to 12 carbon atoms, preferably 1 to 8 carbon atoms and most preferably 1 to 4 carbon atoms including but not limited to methyl, ethyl, n-propyl, isopropyl, n-butyl, sec-butyl, isobutyl, tert-butyl n-pentyl, n-hexyl, n-heptyl, n-octyl. In particular, alkyl relates to a group having 1, 2, 3, 4, 5, 6, 7, 8, 9, 10, 11 or 12 atoms. Haloalkyl relates to an alkyl radical as defined above preferably having 1 to 8 carbon atoms, preferably 1 to 4 carbon atoms substituted with one or more halide atoms for example one or more of F, Cl, Br or I, such as CH₂CH₂Br, CF₃ or CCl₃.

The term "alkenyl" means a straight chain or branched alkylenyl radical of 2 to 12 carbon atoms, preferably 2 to 6 carbon atoms and most preferably 2 to 4 carbon atoms, and containing one or more carbon-carbon double bonds and includes but is not limited to ethylene, n-propyl-1-ene, n-propyl-2-ene, isopropylene, etc. In particular, alkenyl relates to a group having 2, 3, 4, 5, 6, 7, 8, 9, 10, 11 or 12 carbon atoms. The term "alkynyl" means a straight chain or branched alkynyl radical of 2 to 12 carbon atoms, preferably 2 to 6 carbon atoms and most preferably 2 to 4 carbon atoms, and containing one or more carbon-carbon triple bonds and includes but is not limited to ethynyl, 2-methylethynyl etc. In particular, alkynyl relates to a group having 2, 3, 4, 5, 6, 7, 8, 9, 10, 11 or 12 carbon atoms.

"Carbocyclyl" relates to a saturated, partly unsaturated or unsaturated 3-12 membered hydrocarbon ring preferably a 6-12 membered hydrocarbon ring, including cycloalkyl and aryl.

"Aryl" means an aromatic 3-12 membered hydrocarbon preferably a 6-12 membered hydrocarbon containing one ring or being fused to one or more unsaturated rings including but not limited to phenyl, napthyl, anthracenyl or phenanthracenyl.

"Heteroaryl" means an aromatic 3-12 membered aryl preferably a 6-12 membered aryl containing one or more heteroatoms selected from N, O or S and containing one ring or being fused to one or more unsaturated rings and;

"Heterocyclyl" means a 3-12 membered ring system preferably a 6-12 membered ring system containing one or more heteroatoms selected from N, O or S and includes heteroaryl. In particular the terms "carbocyclyl", "aryl", "heteroaryl" and "heterocyclyl" relate to a group having 3, 4, 5, 6, 7, 8, 9, 10, 11 or 12 carbon atoms.

The heterocyclyl system can contain one ring or may be fused to one or more saturated or unsaturated rings; the heterocyclyl can be fully saturated, partially saturated or unsaturated and includes but is not limited to heteroaryl and heterocarbocyclyl. Examples of carbocyclyl or heterocyclyl groups include but are not limited to cyclohexyl, phenyl, acridine, benzimidazole, benzofuran, benzothiophene, benzoxazole, benzothiazole, carbazole, cinnoline, dioxin, dioxane, dioxolane, dithiane, dithiazine, dithiazole, dithiolane, furan, imidazole, imidazoline, imidazolidine, indole, indoline, indolizine, indazole, isoindole, isoquinoline, isoxazole, isothiazole, morpholine, napthyridine, oxazole, oxadiazole, oxathiazole, oxathiazolidine, oxazine, oxadiazine, phenazine, phenothiazine, phenoxazine, phthalazine, piperazine, piperidine, pteridine, purine, pyran, pyrazine, pyrazole, pyrazoline, pyrazolidine, pyridazine, pyridine, pyrimidine, pyrrole, pyrrolidine, pyrroline, quinoline, quinoxaline, quinazoline, quinolizine, tetrahydrofuran, tetrazine, tetrazole, thiophene, thiadiazine, thiadiazole, thiatriazole, thiazine, thiazole, thiomorpholine, thianaphthalene, thiopyran, triazine, triazole, and trithiane.

For the purpose of the present invention, the term "fused" includes a polycyclic compound in which one ring contains one or more atoms preferably one, two or three atoms in common with one or more other ring.

Halogen means F, Cl, Br or I, preferably F.

R¹ is preferably a group of formula (II) or an optionally substituted five or six membered carbocyclyl or heterocyclyl group wherein the carbocyclyl or heterocyclyl group is optionally fused to one or more unsaturated rings.

When R¹ is a substituted five or six membered carbocyclyl or heterocyclyl group it is preferably selected from optionally substituted phenyl, acridine, benzimidazole, benzofuran, benzothiophene, benzoxazole, benzothiazole, cyclohexyl furan, imidazole, indole, isoindole, isoquinolitne, isoxazole, isothiazole, morpholine, napthaline, oxazole, phenazine, phenothiazine, phenoxazine, piperazine, piperidine, pyrazole, pyridazine, pyridine, pyrrole, quinoline, quinolizine, tetrahydrofuran, tetrazine, tetrazole, thiophene, thiazole, thiomorpholine, thianaphthalene, thiopyran, triazine, triazole or trithiane.

When R¹ is a group of formula (II), X is preferably a group NR³, Y is preferably absent and one or more of R² and R³ are preferably hydrogen alkyl or cycloalkyl, in particular, the group of formula (II) is preferably an alkylamino or cycloalkylamino group preferably selected from optionally substituted methylamino, ethylamino, propylamino, isopropylamino, butylamino, cyclobutylamino, pentylamino, cyclopentylamino, hexylamino, cyclohexylamino, heptylamino, cycloheptylamino, octylamino and cyclooctylamino. In particular, X is an alkylamino or a cycloalkylamino group wherein the alkyl group has 1, 2, 3, 4, 5, 6, 7, 8, 9, 10, 11 or 12 carbon atoms and the cycloalkyl group has 3, 4, 5, 6, 7, 8, 9, 10, 11 or 12 carbon atoms. R¹ may additionally be a group of formula (II) wherein X is NR³ and R² and R³ form a 5, 6, 7 or 8 membered ring, said ring being partially, saturated, fully saturated or unsaturated and optionally substituted as previously discussed.

As discussed above, R¹ can be optionally substituted at any position on the alkylamino, cycloalkyl amino, carbocyclyl, heterocyclyl or optional fused ring.

R¹ is preferably substituted with one or more of OR²⁴, halogen, C₁₋₆ alkyl, C₂₋₆ alkenyl, C₂₋₆ alkynyl, C₁₋₆haloalkyl, C₁₋₆alkylaryl, C₁₋₆alkylheterocyclyl, (CH₂)ₙOR²⁴, (CH₂)ₙNR²⁴₂, SR²⁴, NO₂, CN, NR²⁴₂, CO₂R²⁴, NR²⁴C(O)R²⁴, NR²⁴S(O)₂R²⁴, COR²⁴, CONR²⁴₂, S(O)₂R²⁴, S(O)R²⁴ or SO₂NR²⁴₂;
wherein R²⁴ is hydrogen, C₁₋₄ alkyl or C₆₋₁₂ aryl preferably phenyl, or C₅₋₁₂ heterocyclyl preferably pyridine, and n is 1, 2, 3, 4, 5 or 6.
wherein two R²⁴ in NR²⁴₂ may optionally form a partially saturated, unsaturated or fully saturated three to seven membered ring containing one to three heteroatoms, said ring is preferably independently substituted with one or more of halogen, C₁₋₁₂ alkyl, C₂₋₁₂ alkenyl, C₂₋₁₂ alkynyl, C₁₋₁₂ haloalkyl, C₃₋₁₂ carbocyclyl, C₃₋₁₂ heterocyclyl, OR²⁵, SR²⁵, NO₂, CN, NR²⁵₂, NR²⁵COR²⁵, NR²⁵CONR²⁵₂, NR²⁵COR²⁵, NR²⁵CO₂R²⁵, CO₂R²⁵, COR²⁵, CONR²⁵₂, S(O)₂R²⁵, SONR²⁵₂, S(O)R²⁵, SO₂NR²⁵₂, or NR²⁵S(O)₂R²⁵; and each saturated carbon in the optional ring is further optionally and independently substituted by =O, =S, NNR²⁶₂, =N-OR²⁶, =NNR²⁶COR²⁶, =NNR²⁶CO₂R²⁶, =NNSO₂R²⁶, or =NR²⁶; and each substitutable nitrogen atom is optionally substituted by R²⁷, COR²⁷, SO₂R²⁷ or CO₂R²⁷;
wherein R²⁵ is hydrogen , C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR²⁸, SR²⁸, NO₂, CN, NR²⁸R²⁸, NR²⁸COR²⁸, NR²⁸CONR²⁸R²⁸, NR²⁸COR²⁸, NR²⁸CO₂R²⁸, CO₂R²⁸, COR²⁸, CONR²⁸₂, S(O)₂R²⁸, SONR²⁸₂, S(O)R²⁸, SO₂NR²⁸R²⁸, NR²⁸S(O)₂R²⁸, wherein the C₁₋₁₂ alkyl group optionally incorporates one or two insertions selected from the group consisting of -O-,-N(R²⁸)-, -S(O)- and -S(O₂)-, wherein each R²⁸ may be the same or different and is as defined below;
wherein R²⁶ is hydrogen, C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR²⁸, SR²⁸, NO₂, CN, NR²⁸R²⁸, NR²⁸COR²⁸, NR²⁸CONR²⁸R²⁸, NR²⁸COR²⁸, NR²⁸CO₂R²⁸, CO₂R²⁸, COR²⁸, CONR²⁸, S(O)₂R²⁸, S(O)R²⁸, SO₂NR²⁸R²⁸, NR²⁸S(O)₂R²⁸, wherein the C₁₋₁₂ alkyl group optionally incorporates one or two insertions selected from the group consisting of -O-, -N(R²⁸)-, -S(O)- and - S(O₂)-, wherein each R²⁸ may be the same or different and is as defined below;
wherein R²⁷ is hydrogen, C₁₋₆ alkyl, C₁₋₆ haloalkyl or C₆₋₁₂ aryl;
wherein R²⁸ is hydrogen, C₁₋₆alkyl, or C₁₋₆haloalkyl.
R⁴ is preferably selected from phenyl, cyclohexyl, acridine, benzimidazole, benzofuran, benzothiophene, benzoxazole, benzothiazole, indole, isoindole, indolizine, indazole, isoindole, isoquinoline, morpholine, napthalene, phenazine, phenothiazine, phenoxazine, piperazine, piperidine, pyridazine, pyridine, pyrimidinyl, pyrazinyl, quinoline, quinolizine tetrazine, thiomorpholine, thianaphthalene, thiopyran, triazine, trithiane, furan, imidazole, isoxazole, isothiazole, oxazole, oxadiazole, oxathiazole, pyrazole, pyrrole, tetrazole, thiophene, thiadiazole, thiatriazole, thiazole or triazole.

As discussed above, R⁴ can be optionally substituted at any position on the carbocyclyl, heterocyclyl or optional fused ring. Preferably, each substitutable carbon or hetero-atom in R⁴ is optionally and independently substituted by one or more of C₁₋₆ alkyl, OR²⁰, SR²⁰, NO₂, CN, NR²⁰₂, NR²⁰COR²⁰, NR²⁰CONR²⁰₂, NR²⁰COR²⁰, NHCO₂R²⁰, CO₂R²⁰, COR²⁰, CONR²⁰₂, S(O)₂R²⁰, SONR²⁰₂, S(O)R²⁰, SO₂NR²⁰₂, or NR²⁰S(O)₂R²⁰;
wherein R²⁰ is hydrogen, C₁₋₆ alkyl, or C₁₋₆ haloalkyl.
When R⁴ is a six-membered carbocyclyl or heterocyclyl group, R⁴ is preferably substituted with one or more of OR²⁹, NR²⁹₂, SR²⁹, (CH₂)ₙOR²⁹, (CH₂)ₙNR²⁹₂, halogen, C₁₋₆ alkyl, C₂₋₆ alkenyl, C₂₋₆ alkynyl, haloalkyl, NO₂, CN, NR²⁹C(O)R²⁹, NR²⁹S(O)R²⁹, CO₂R²⁹, COR²⁹, CONR²⁹, S(O)₂R²⁹, S(O)R²⁹ or SO₂NR²⁹₂;
wherein R²⁹ is hydrogen, C₁₋₄ alkyl, C₅₋₁₂ heterocyclyl or C₆₋₁₂ aryl preferably phenyl, and n is 1, 2, 3, 4, 5 or 6.
wherein two R²⁹ in NR²⁹₂ may optionally form a partially saturated, unsaturated or fully saturated five to seven membered ring containing one to three heteroatoms, optionally and independently substituted with one or more of halogen, C₁₋₁₂ alkyl, C₂₋₁₂ alkenyl, C₂₋₁₂ alkynyl, C₁₋₁₂ haloalkyl, C₆₋₁₂ carbocyclyl, C₅₋₁₂ heterocyclyl, OR³⁰, SR³⁰, NO₂, CN, NR³⁰₂, NR³⁰COR³⁰, NR³⁰CONR³⁰₂, NR³⁰COR³⁰, NR³⁰CO₂R³⁰, CO₂R³⁰, COR³⁰, CONR³⁰₂, S(O)₂R³⁰, SONR³⁰₂, S(O)R³⁰, SO₂NR³⁰₂, or NR³⁰S(O)₂R³⁰; and each saturated carbon in the optional ring is further optionally and independently substituted by =O, =S, NNR³¹₂, =N-OR³¹, =NNR³¹COR³¹, =NNR³¹CO₂R³¹, =NNSO₂R³¹, or =NR³¹; and each substitutable nitrogen atom is optionally substituted by R³², COR³², SO₂R³² or CO₂R³²;
wherein R³⁰ is hydrogen , C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR³³, SR³³, NO₂, CN, NR³³R³³, NR³³COR³³, NR³³CONR³³R³³, NR³³COR³³, NR³³CO₂R³³, CO₂R³³, COR³³, CONR³³₂, S(O)₂R³³, SONR³³₂, S(O)R³³, SO₂NR³³R³³, NR³³S(O)₂R³³, therein the C₁₋₁₂ alkyl group optionally incorporates one or two insertions selected from the group consisting of -0-, - N(R³³)-, -S(O)- and -S(O₂)-, wherein each R³³ may be the same or different and is as defined below;
wherein R³¹ is hydrogen, C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR³³, SR³³, NO₂, CN, NR³³R³³, NR³³COR³³, NR³³CONR³³R³³, NR³³COR³³, NR³³CO₂R³³, CO₂R³³, COR³³, CONR³³₂, S(O)²R³³, S(O)R³³, SO₂NR³³R³³, NR³³S(O)₂R³³, wherein the C₁₋₁₂ alkyl group optionally incorporates one or two insertions selected from the group consisting of -O-, -N(R³³)-, -S(O)- and - S(O₂)-, wherein each R²¹ may be the same or different and is as defined below;
wherein R³² is hydrogen, C₆₋₁₂ aryl, C₁₋₆ alkyl or C₁₋₆ haloalkyl;
wherein R³³ is hydrogen, C₁₋₆ alkyl, or C₁₋₆ haloalkyl.

When R⁴ is a five-membered heterocyclyl, it is preferably a group 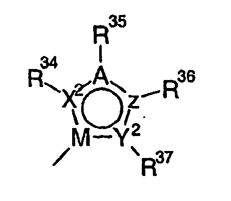
Wherein A, X², Y² or Z are independently selected from N, O, C, S and M is C or N, wherein one, two, three or four of A, X², Y², Z and M is other than C, preferably R⁴ is furan, imidazole, isoxazole, isothiazole, oxazole, oxadiazole, oxatriazole, pyrazole, pyrrole, tetrazole, thiophene, thiadiazole, thiatriazole, thiazole or triazole;
R³⁴, R³⁵, R³⁶ or R³⁷ are independently selected from a lone electron pair, hydrogen, halogen, C₁₋₁₂ alkyl, C₁₋₁₂ haloalkyl, OR³⁸, SR³⁸, NO₂, CN, NR³⁸₂, NR³⁸COR³⁸, NR³⁸CONR³⁸₂, NR³⁸COR³⁸, NR³⁸CO₂R³⁸, (CH₂)ₙOR³⁸, (CH₂)ₙNR³⁸_{2,} CO₂R³⁸, COR³⁸, CONR³⁸₂, S(O)₂R³⁸, SONR³⁸₂, S(O)R³⁸, SO₂NR³⁸₂, or NHS(O)₂R³⁸;
   wherein n is 1 to 6, preferably n is 1, 2 or 3;
or wherein any two of R³⁴, R³⁵, R³⁶ or R³⁷ may optionally form a partially saturated, unsaturated or fully saturated five to seven membered ring containing zero to three heteroatoms, each saturated carbon in the optional fused ring is further optionally and independently substituted with one or more of halogen, C₁₋₁₂ alkyl, C₂₋₁₂ alkenyl, C₂₋₁₂ alkynyl, C₁₋₁₂ haloalkyl, C₆₋₁₂ carbocyclyl, C₅₋₁₂ heterocyclyl, OR³⁸, SR³⁸, NO₂, CN, NR³⁸₂, NR³⁸CONR³⁸₂, NR³⁸COR³⁸, NR³⁸CO₂R³⁸, (CH₂)ₙOR³⁸, (CH₂)ₙNR³⁸₂, CO₂R³⁸, COR³⁸, CONR³⁸₂, S(O)₂R³⁸, SONR³⁸₂, S(O)R³⁸, SO₂NR³⁸₂, or NR³⁸S(O)₂R³⁸; and each saturated carbon in the optional fused ring is further optionally and independently substituted by =O, =S, NNR³⁹₂, =N-OR³⁹, =NNR³⁹COR³⁹, =NNR³⁹CO₂R³⁹, =NNSO₂R³⁹, or =NR³⁹; and each substitutable nitrogen atom in R⁴ is optionally substituted by R⁴⁰, COR⁴⁰, SO₂R⁴⁰ or CO₂R⁴⁰;
   wherein n is 1 to 6, preferably n is 1, 2 or 3;
wherein R³⁸ is hydrogen, C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR⁴¹, SR⁴¹, NO₂, CN, NR⁴¹R⁴¹, NR⁴¹CONR⁴¹R⁴¹, NR⁴¹COR⁴¹, NR⁴¹CO₂R⁴¹, CO₂R⁴¹, COR⁴¹, CONR⁴¹₂, S(O)₂R⁴¹, SONR⁴¹₂, S(O)R⁴¹, SO₂NR⁴¹R⁴¹, NR⁴¹S(O)₂R⁴¹, wherein the C₁₋₁₂ alkyl group optionally incorporates one or two insertions selected from the group consisting of -O-, -N(R⁴¹)-, -S(O)- and - S(O₂)-, wherein each R⁴¹ may be the same or different and is as defined below;
wherein R³⁹ is hydrogen, C₁₋₁₂ alkyl, carbocyclyl or heterocyclyl, optionally substituted by one or more of C₁₋₆ alkyl, halogen, C₁₋₆ haloalkyl, OR⁴¹, SR⁴¹, NO₂, CN, NR⁴¹R⁴¹, NR⁴¹COR⁴¹, NR⁴¹CONR⁴¹R⁴¹, NR⁴¹COR⁴¹, NR⁴¹CO₂R⁴¹, CO₂R⁴¹, COR⁴¹, CONR⁴¹₂, S(O)₂R⁴¹, S(O)R⁴¹, SO₂NR⁴¹R⁴¹, NR⁴¹S(O)₂R⁴¹, wherein the C₁₋₁₂ alkyl group optionally incorporates one or two insertions selected from the group consisting of -O-, -N(R⁴¹)-, -S(O)- and -S(O₂)-, wherein each R⁴¹ may be the same or different and is as defined below;
wherein R⁴⁰ is hydrogen, C₁₋₆ alkyl, C₁₋₆ haloalkyl or C₆₋₁₂ aryl.
wherein R⁴¹ is hydrogen, C₁₋₆ alkyl, or C₁₋₆ haloalkyl.

More preferably R³⁴, R³⁵, R³⁶ or R³⁷ are independently selected from a lone electron pair, hydrogen, halogen, C₁₋₆ alkyl, C₁₋₆ haloalkyl, OR⁴², SR⁴², CN, NR⁴²₂, NR⁴²COR⁴², CO₂R⁴², COR⁴², CONR⁴²₂, S(O)R⁴², or S(O)R⁴²; wherein R⁴² is hydrogen, C₁₋₄ alkyl, preferably methyl or ethyl or carbocyclyl, preferably phenyl.

Representative compounds according to the first aspect of the invention are illustrated below.

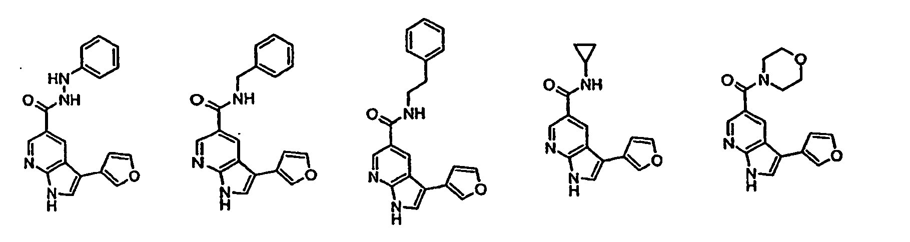

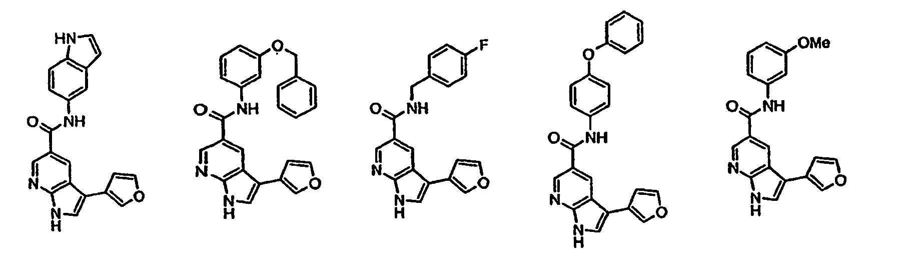

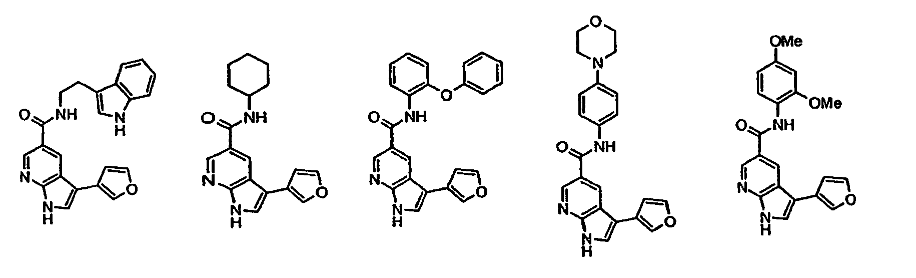

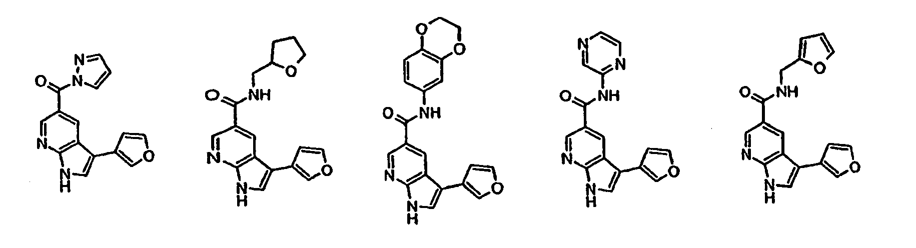

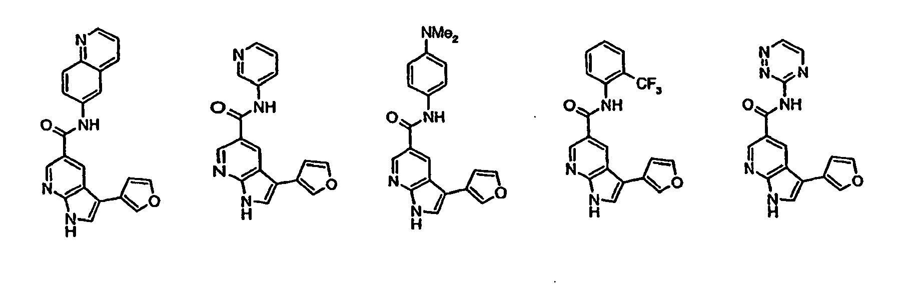

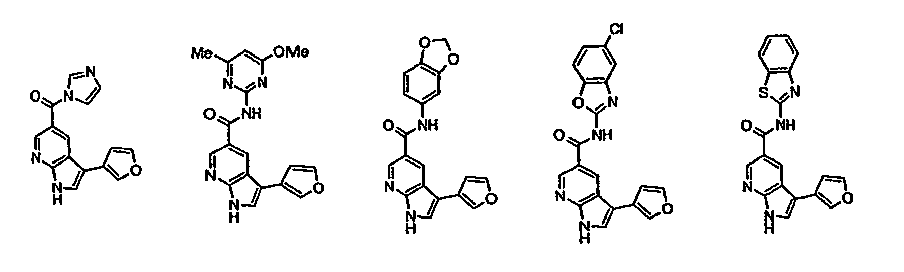

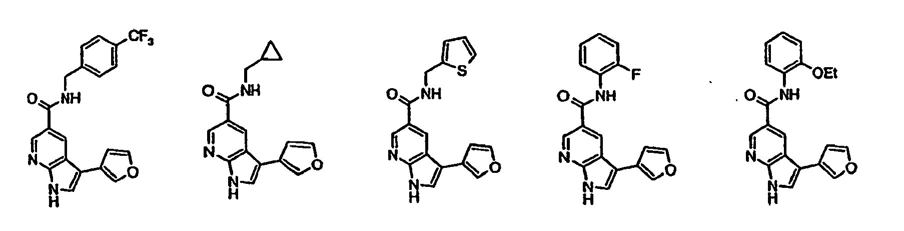

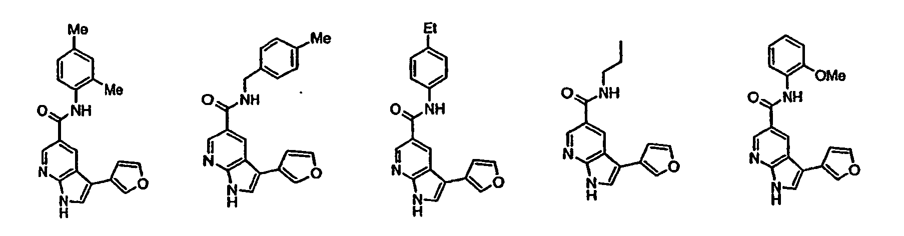

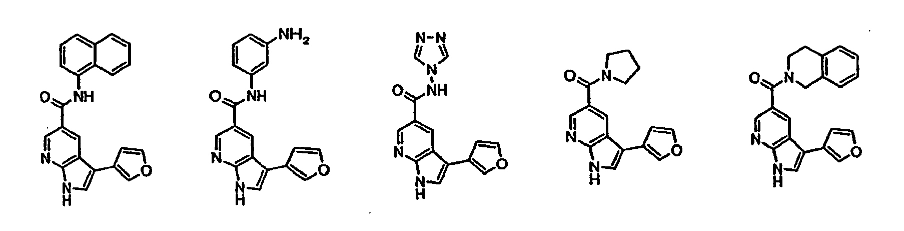

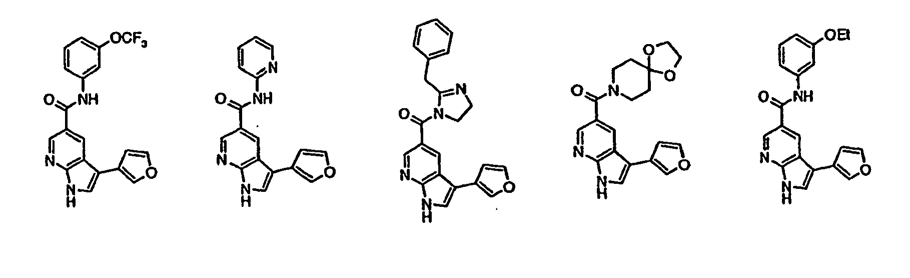

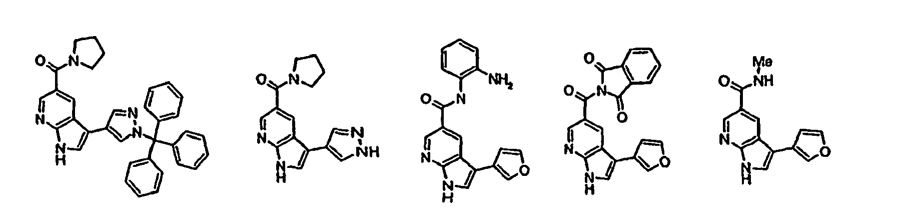

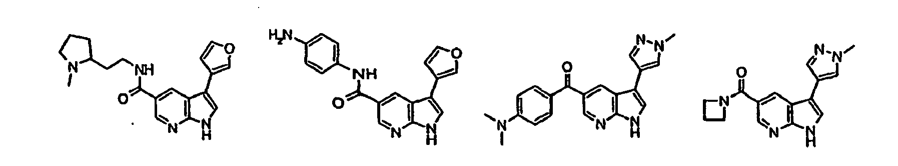

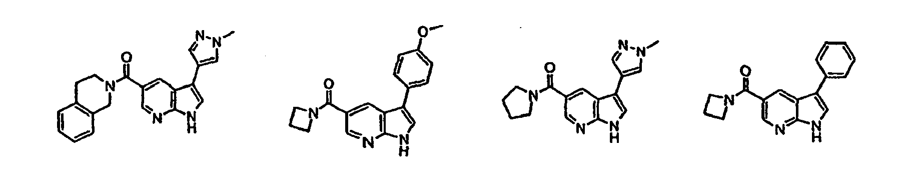

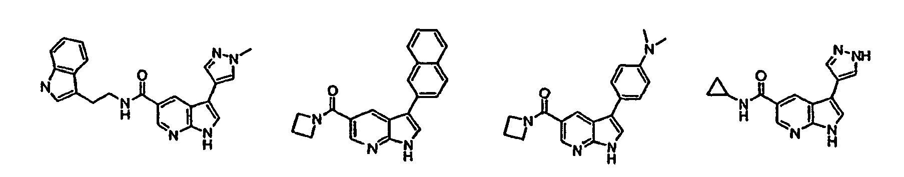

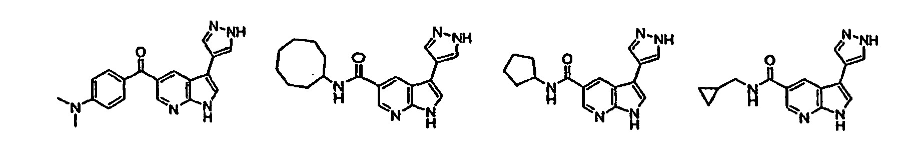

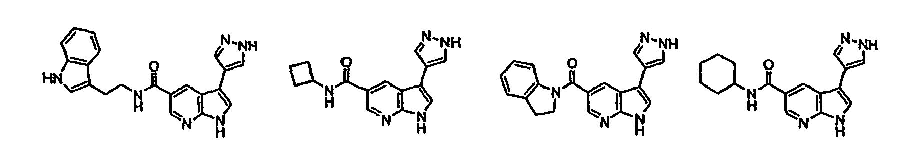

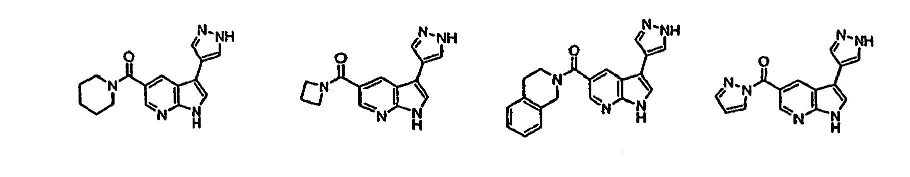

The compounds of the first aspect may be provided as a salt, preferably as a pharmaceutically acceptable salt of compounds of formula (I). Examples of pharmaceutically acceptable salts of these compounds include those derived from organic acids such as acetic acid, malic acid, tartaric acid, citric acid, lactic acid, oxalic acid, succinic acid, fumaric acid, maleic acid, benzoic acid, salicylic acid, phenylacetic acid, mandelic acid, methanesulphonic acid, benzenesulphonic acid and p-toluenesulphonic acid, mineral acids such as hydrochloric and sulphuric acid and the like, giving methanesulphonate, benzenesulphonate, p-toluenesulphonate, hydrochloride and sulphate, and the like, respectively or those derived from bases such as organic and inorganic bases. Examples of suitable inorganic bases for the formation of salts of compounds for this invention include the hydroxides, carbonates, and bicarbonates of ammonia, lithium, sodium, calcium, potassium, aluminium, iron, magnesium, zinc and the like. Salts can also be formed with suitable organic bases. Such bases suitable for the formation of pharmaceutically acceptable base addition salts with compounds of the present invention include organic bases, which are nontoxic and strong enough to form salts. Such organic bases are already well known in the art and may include amino acids such as arginine and lysine, mono-, di-, or trihydroxyalkylamines such as mono-, di-, and triethanolamine, choline, mono-, di-, and trialkylamines, such as methylamine, dimethylamine, and trimethylamine, guanidine; N-methylglucosamine; N-methylpiperazine; morpholine; ethylenediamine; N-benzylphenethylamine; tris(hydroxymethyl) aminomethane; and the like.

Salts may be prepared in a conventional manner using methods well known in the art. Acid addition salts of said basic compounds may be prepared by dissolving the free base compounds according to the first aspect of the invention in aqueous or aqueous alcohol solution or other suitable solvents containing the required acid. Where a compound of the invention contains an acidic function, a base salt of said compound may be prepared by reacting said compound with a suitable base. The acid or base salt may separate directly or can be obtained by concentrating the solution e.g. by evaporation. The compounds of this invention may also exist in solvated or hydrated forms.

The compounds of the invention may contain one or more asymmetric carbon atoms and may exist in racemic and optically active forms. The compounds of the invention may exist in trans or cis form. The first aspect of the invention covers all of these compounds.

The second aspect of the invention provides a process for the manufacture of a compound of formula (I) wherein R¹ is a group of formula (II) as defined in the first aspect of the invention comprising the condensation of an intermediate with an intermediate (IV). 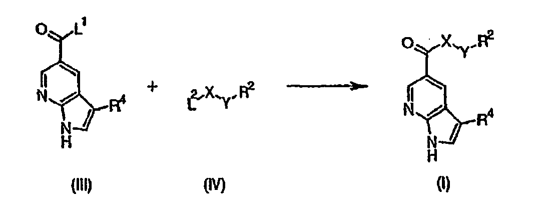 wherein R² and R⁴ are as defined in the first aspect of the invention; L¹ and L² are independently a leaving group wherein L¹ and L² together form a condensation product.

According to the process, a compound of the general formula (III), undergoes a condensation reaction with the compound of the general formula (IV), to form a compound of general formula I. In formulae (III) and (IV), L¹ and L² represent radicals that together form a condensation product, e. g. H and OH or H and Cl. Preferably L¹ is OH, OR⁵⁰, OM, Cl, Br or I wherein R⁵⁰ is C₁₋₆ alkyl, preferably methyl or ethyl and M is a metal, preferably Na, Li, K, Ca, Mg or Ba, and L² is preferably hydrogen or M. The condensation reaction occurs in a solution, preferably in a polar aprotic solvent such as e.g. dimethylformamide or dichloromethane. The condensation reaction may occur under the influence of coupling agents such as, for instance WSC·HCl, DCC, benzotriazole-1-yl-oxy-tris-(dimethylamino)-phosphonium hexafluorophosphate (BOP), PyBrOP, etc., and in the presence of a tertiary amine (e.g. triethylamine) and 1-hydroxybenzotriazole (HOBT). Alternatively, the acid (III) may be first converted to an acid chloride by treatment with, for example, oxalyl chloride or thionyl chloride, and then without purification, reacted with, e.g. amines of formula (IV).

A compound of formula (III) may undergo one or more further reactions to provide a different compound of formula (III). For example, a compound may undergo a hydrolysis, reduction, oxidation, elimination, substitution and/or addition reaction.

A compound of the first aspect can be prepared by a process comprising providing a starting material, which is commercially available or can be produced by a method known in the art, converting the starting material to form an intermediate compounds of formula (III),
using a process as described above or a process known in the art (and optionally converting the intermediate compound so formed into another intermediate compound) and then converting the intermediate compound into a compound of the first aspect using a process as described above or a process known in the art (and optionally converting the compound of the first aspect so formed into another compound of the first aspect).

The third aspect of the invention provides a composition comprising a compound according to the first aspect of the invention in combination with a pharmaceutically acceptable carrier, diluent or excipient.

The composition may also comprise one or more additional active agent, such as an anti-inflammatory agent (for example a p38 inhibitor, glutamate receptor antagonist, or a calcium channel antagonist), AMPA receptor antagonist, a chemotherapeutic agent and/or an antiproliferative agent.

Suitable carriers and/or diluents are well known in the art and include pharmaceutical grade starch, mannitol, lactose, magnesium stearate, sodium saccharin, talcum, cellulose, glucose, sucrose, (or other sugar), magnesium carbonate, gelatin, oil, alcohol, detergents, emulsifiers or water (preferably sterile). The composition may be a mixed preparation of a composition or may be a combined preparation for simultaneous, separate or sequential use (including administration).

The composition according to the invention for use in the aforementioned indications may be administered by any convenient method, for example by oral (including by inhalation), parenteral, mucosal (e.g. buccal, sublingual, nasal), rectal or transdermal administration and the compositions adapted accordingly.

For oral administration, the composition can be formulated as liquids or solids, for example solutions, syrups, suspensions or emulsions, tablets, capsules and lozenges.

A liquid formulation will generally consist of a suspension or solution of the compound or physiologically acceptable salt in a suitable aqueous or non-aqueous liquid carrier(s) for example water, ethanol, glycerine, polyethylene glycol or oil. The formulation may also contain a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and microcrystalline cellulose.

A composition in the form of a capsule can be prepared using routine encapsulation procedures. For example, powders, granules or pellets containing the active ingredient can be prepared using standard carriers and then filled into a hard gelatine capsule; alternatively, a dispersion or suspension can be prepared using any suitable pharmaceutical carrier(s), for example aqueous gums, celluloses, silicates or oils and the dispersion or suspension then filled into a soft gelatine capsule.

Compositions for oral administration may be designed to protect the active ingredient against degradation as it passes through the alimentary tract, for example by an outer coating of the formulation on a tablet or capsule.

Typical parenteral compositions consist of a solution or suspension of the compound or physiologically acceptable salt in a sterile aqueous or non-aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions for nasal or oral administration may conveniently be formulated as aerosols, drops, gels and powders. Aerosol formulations typically comprise a solution or fine suspension of the active substance in a physiologically acceptable aqueous or non-aqueous solvent and are usually presented in single or multidose quantities in sterile form in a sealed container, which can take the form of a cartridge or refill for use with an atomising device. Alternatively the sealed container may be a unitary dispensing device such as a single dose nasal inhaler or an aerosol dispenser fitted with a metering valve, which is intended for disposal once the contents of the container have been exhausted. Where the dosage form comprises an aerosol dispenser, it will contain a pharmaceutically acceptable propellant. The aerosol dosage forms can also take the form of a pump-atomiser.

Compositions suitable for buccal or sublingual administration include tablets, lozenges and pastilles, wherein the active ingredient is formulated with a carrier such as sugar and acacia, tragacanth, or gelatin and glycerin.

Compositions for rectal or vaginal administration are conveniently in the form of suppositories (containing a conventional suppository base such as cocoa butter), pessaries, vaginal tabs, foams or enemas.

Compositions suitable for transdermal administration include ointments, gels, patches and injections including powder injections.

Conveniently the composition is in unit dose form such as a tablet, capsule or ampoule.

The fourth aspect of the invention provides a process for the manufacture of a composition according to the third aspect of the invention. The manufacture can be carried out by standard techniques well known in the art and comprises combining a compound according to the first aspect of the invention and the pharmaceutically acceptable carrier or diluent and optionally one or more additional active agents. The composition may be in any form including a tablet, a liquid, a capsule, and a powder or in the form of a food product, e.g. a functional food. In the latter case the food product itself may act as the pharmaceutically acceptable carrier.

The fifth aspect of the present invention relates to a compound of the first aspect, or a composition of the third aspect, for use in medicine.

The compounds of the present invention are inhibitors of JNK, such as JNK1, JNK2, or JNK3. In particular, the compounds of the present invention are inhibitors of JNK3. Preferably, the compounds of the present invention inhibit JNK3 selectively (i.e. the compounds of the invention preferably show greater activity against JNK3 than JNK1 and 2). For the purpose of this invention, an inhibitor is any compound, which reduces or prevents the activity of the JNK enzyme.

The compounds are therefore useful for conditions for which inhibition of JNK activity is beneficial. Thus, preferably, this aspect provides a compound of the first aspect, or a composition of the third aspect of the present invention, for the prevention or treatment of a JNK-mediated disorder. The compounds of the first aspect of the invention may thus be used for the inhibition of JNK, more preferably for the inhibition of JNK3.

A "JNK-mediated disorder" is any disease or deleterious condition in which JNK plays a role. Examples include neurodegenerative disorder (including dementia), inflammatory disease, a disorder linked to apoptosis, particularly neuronal apoptosis, autoimmune disease, destructive bone disorder, proliferative disorder, cancer, infectious disease, allergy, ischemia reperfusion injury, heart attack, angiogenic disorder, organ hypoxia, vascular hyperplasia, cardiac hypertrophy, thrombin induced platelet aggregation and any condition associated with prostaglandin endoperoxidase synthase-2. The compounds of the present invention may be used for any of these JNK-mediated disorders.

The compounds of the present invention are particularly useful for the prevention or treatment of a neurodegenerative disorder. In particular, the neurodegenerative disorder results from apoptosis and/or inflammation. Examples of neurodegenerative disorders are: dementia; Alzheimer's disease; Parkinson's disease; Amyotrophic Lateral Sclerosis; Huntington's disease; senile chorea; Sydenham's chorea; hypoglycemia; head and spinal cord trauma including traumatic head injury; acute and chronic pain; epilepsy and seizures; olivopontocerebellar dementia; neuronal cell death; hypoxia-related neurodegeneration; acute hypoxia; glutamate toxicity including glutamate neurotoxicity; cerebral ischemia; dementia linked to meningitis and/or neurosis; cerebrovascular dementia; or dementia in an HIV-infected patient.

The neurodegenerative disorder may be a peripheral neuropathy, including mononeuropathy, multiple mononeuropathy or polyneuropathy. Examples of peripheral neuropathy may be found in diabetes mellitus, Lyme disease or uremia; peripheral neuropathy caused by a toxic agent; demyelinating disease such as acute or chronic inflammatory polyneuropathy, leukodystrophies, or Guillain-Barré syndrome; multiple mononeuropathy secondary to a collagen vascular disorder (e.g. polyarteritis nodosa, SLE, Sjögren's syndrome); multiple mononeuropathy secondary to sarcoidosis; multiple mononeuropathy secondary to a metabolic disease (e.g. diabetes or amyloidosis); or multiple mononeuropathy secondary to an infectious disease (e.g Lyme disease or HIV infection).

The compounds of the invention can also be used to prevent or treat disorders resulting from inflammation. These include, for example, inflammatory bowel disorder, bronchitis, asthma, acute pancreatitis, chronic pancreatitis, allergies of various types, and possibly Alzheimer's disease. Autoimmune diseases which may also be treated or prevented by the compounds of the present invention include rheumatoid arthritis, systemic lupus erythematosus, glomerulonephritis scleroderma, chronic thyroiditis, Graves's disease, autoimmune gastritis, diabetes, autoimmune haemolytis anaemia, autoimmune neutropaenia, thrombocytopenia, atopic dermatitis, chronic active hepatitis, myasthenia gravis, multiple sclerosis, ulcerative colitis, Crohn's disease, psoriasis or graft vs host disease.

A compound of the present invention may be administered simultaneously, subsequently or sequentially with one or more other active agent, such as an anti-inflammatory agent e.g. p38 inhibitor, AMPA receptor antagonist, glutamate receptor antagonist, calcium channel antagonist, a chemotherapeutic agent or an antiproliferative agent. For example, for acute treatment, a p38 inhibitor may be administered to a patient prior to administering a compound of the present invention.

The compounds of the invention will normally be administered in a daily dosage regimen (for an adult patient) of, for example, an oral dose of between 1 mg and 2000 mg, preferably between 30 mg and 1000 mg, e.g. between 10 and 250 mg or an intravenous, subcutaneous, or intramuscular dose of between 0.1 mg and 100 mg, preferably between 0.1 mg and 50 mg, e.g. between 1 and 25 mg of the compound of the formula (I) or a physiologically acceptable salt thereof calculated as the free base, the compound being administered 1 to 4 times per day. Suitably the compounds will be administered for a period of continuous therapy, for example for a week or more.

The sixth aspect of the present invention provides the use of a compound of the first aspect in the manufacture of a medicament for the prevention or treatment of a JNK-mediated disorder. The medicament may be used for treatment or prevention of any of the JNK-mediated disorders listed above in relation to the fifth aspect. Again, the compound of the present invention may be administered simultaneously, subsequently or sequentially with one or more other active agent, preferably a p38 inhibitor for acute treatment.

An assay for determining the activity of the compounds of the present invention is described, comprising providing a system for assaying the activity and assaying the activity of the compound. Preferably the assay is for the JNK inhibiting activity of the compound, more preferably it is for the JNK3-specific inhibiting activity of the compounds. The compounds of the invention may be assayed in vitro, in vivo, in silico, or in a primary cell culture or a cell line. In vitro assays include assays that determine inhibition of either the kinase activity or ATPase activity of activated JNK. Alternatively, in vitro assays may quantitate the ability of a compound to bind JNK and may be measured either by radiolabelling the compound prior to binding, then isolating the inhibitor/JNK complex and determining the amount of the radiolabel bound or by running a competition experiment where new inhibitors are incubated with JNK bound to known radioligands. An example of an assay, which may be used, is Scintillation Proximity Assay (SPA), preferably using radiolabelled ATP. Another example is ELISA. Any type or isoform of JNK may be used in these assays.

A method for inhibiting the activity or function of a JNK, particularly JNK3 is described which method comprises exposing a
JNK to a compound or a composition of the first or third aspect of the present invention. The method may be performed in a research model, in vitro, in silico, or in vivo such as in an animal model. A suitable animal model may be a kainic acid model in rat or mice, traumatic brain injury model in rat, or MPTP in mice.

All features of each of the aspects apply to all other aspects *mutatis mutandis.*

The invention will now be illustrated by the following non-limiting examples.

### EXAMPLES

### Synthesis of example inhibitor 8

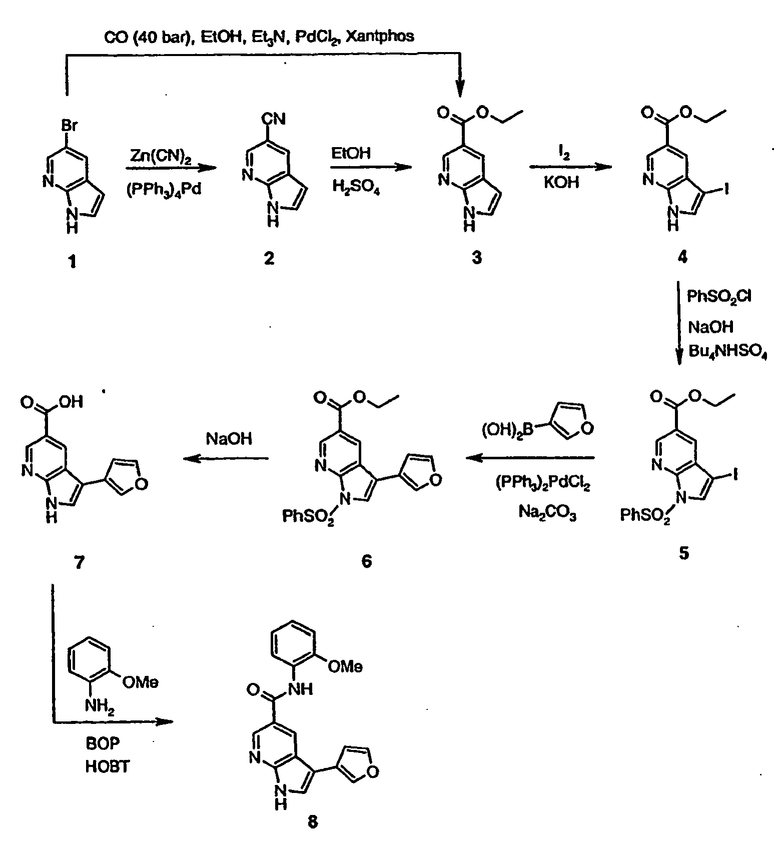

### 1H-Pyrrolo[2,3-b]pyridine-5-carbonitrile (2)

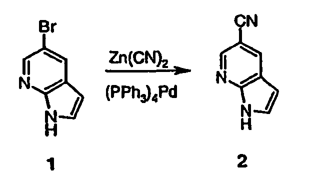

A mixture of bromide 1 (10.0 g, 50.8 mmol), ZnCl₂ (3.58 g, 30.5 mmol), and Pd(PPh₃)₄ (3.52 g, 3.05 mmol) in DMF (110 mL) was heated at 80 °C overnight. The solvent was evaporated and the residue separated by silicagel chromatography (100 g column) using hexane:ethyl acetate as eluent (gradient elution). The resulting solid was partitioned between water (200 mL)/CH₂Cl₂ (100 mL) and the aqueous phase extracted with more CH₂Cl₂ (4 x 100 mL). The combined organic extracts were dried (MgSO₄) and concentrated to give product 2 as a white solid (5.48 g, 75%), which was used for subsequent reactions without further purification.

### 1H-Pyrrolo[2,3-b]pyridine-5-carboxylic acid ethyl ester (3)

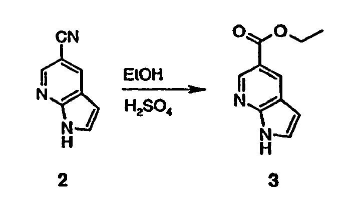

A solution of 2 (197.6 mg, 1.38 mmol) in a mixture of EtOH (4.2 mL) and concentrated H₂SO₄ (2.0 mL) was refluxed overnight. The reaction mixture was cooled and poured slowly onto a mixture of NaHCO₃ (8.2 g, solid), ice (50 g) and ethyl acetate (20 mL). The organic layer was separated. The aqueous layer was extracted with ethyl acetate (3x20 mL). Combined organic solutions were dried (MgSO₄), concentrated and dried in vacuum to afford ethyl ester 3 (262.5 mg, 100%) as white solid; ¹H NMR (400 MHz, CDCl₃) δ 1.44 (t, J = 7.2 Hz, 3H), 4.43 (q, J = 7.2 Hz, 2H), 6.62 (dd, J = 3.6, 2.0 Hz, 1H), 7.39 (dd, J = 3.6, 2.4 Hz, 1H), 8.63 (dd, J = 2.0, 0.8 Hz, 1H), 9.01 (d, J = 2.0 Hz, 1H), 9.21 (bs, 1H).

### 1H-Pyrrolo[2,3-b]pyridine-5-carboxylic acid ethyl ester (3) - an alternative method

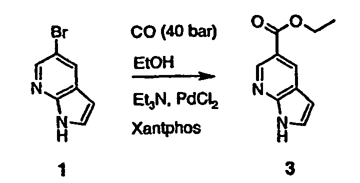

An autoclave charged with a mixture of 1 (9.85 g, 50.0 mmol), PdCl₂ (44 mg, 0.25 mmol), Xantphos (145 mg, 0.25 mmol), Et₃N (9.0 mL, 64.6 mmol) in EtOH (55 mL) was purged with CO. Then, CO was introduced to the pressure of 40 bar and the temperature of the reaction mixture was raised to 120 °C. The mixture was stirred at 120 °C overnight. The mixture was cooled to room temperature and CO was released. ¹H NMR of an aliquot showed conversion of 80%. New portion of PdCl₂ (44 mg, 0.25 mmol) and Xantphos (145 mg, 0.25 mmol) was added. The autoclave was pressurized with CO again and raised to 120 °C. After additional 3 days stirring at 120 °C the reaction was completed (¹H NMR). The reaction mixture was concentrated and separated between AcOEt - saturated aqueous NaHCO₃. The aqueous layer was extracted with AcOEt (5x100 mL). Combined organic solutions were dried (MgSO₄), and concentrated to afford 3 (7.55 g, 79%) as tan solid indistinguishable (¹H NMR) from the sample prepared via nitrile 2.

### 3-Iodo-1H-pyrrolo[2,3-b]pyridine-5-carboxylic acid ethyl ester (4)

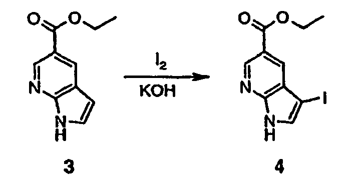

To a solution of 3 (100 mg, 0.526 mmol) in DMF (1.3 mL) was added I₂ (162.56 mg, 0.64 mmol) followed by KOH (43.0 mg, 0.77 mmol). The reaction mixture was stirred for 35 min, and treated with a mixture of 0.1 M phosphate buffer (2.0 mL): saturated aqueous Na₂S₂O₃ (0.5 mL). The suspension was stirred at r.t. for 15 min. The solid precipitate was filtered off, washed with water (2.0 mL), and dried under high vacuum to give 4 as white solid (123.0 mg, 74%); ¹H NMR (400 MHz, DMSO-*d₆*) δ 1.34 (t, J = 7.1 Hz, 3H), 4.35 (q, J = 7.1 Hz, 2H), 7.87 (s, 1H), 8.15 (d, J = 2.0 Hz, 1H), 8.80 (d, J = 2.0 Hz, 1H, 12.50 (bs, 1H).

### 1-Benzenesulfonyl-3-iodo-1H-pyrrolo[2,3-b]pyridine-5-carboxyüc acid ethyl ester (5)

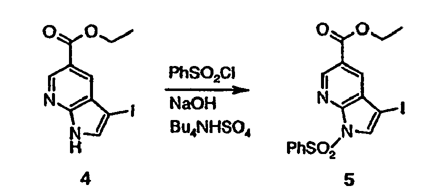

To a suspension of 4 (112.5 mg, 0.356 mmol) in CH₂Cl₂ (2.2 mL) was added benzenesulfonyl chloride (69.6 µL, 0.55 mmol), tetra-n-butylammonium hydrogen sulfate (14.9 mg, 0.044 mmol) and 50% aqueous NaOH (29 µL), and the reaction mixture was stirred for 1 h. The organic layer was separated and the residue was extracted with CH₂Cl₂ (2x3 mL). The combined organic solutions were dried (MgSO₄) and concentrated to give oil, which was triturated with methanol (1.0 mL) to give white solid. The solid was filtered off, washed with methanol (2x1 mL) and dried overnight in vacuum to give product 5 as a white solid (139.2 mg, 64%); ¹H NMR (400 MHz, CDCl₃) δ 1.42 (t, J = 7.1 Hz, 3H), 4.43 (q, J = 7.1 Hz, 2H), 7.49-7.53 (m, 2H), 7.59-7.64 (m, 1H), 7.94 (s, 1H), 8.21-8.25 (m, 2H), 8.31 (d, J = 2.0 Hz, 1H), 9.08 (d, J = 2.0 Hz, 1H).

### 1-Benzenesulfonyl-3-furan-3-yl-1H-pyrrolo[2,3-b]pyridine-5-carboxylic acid ethyl ester (6)

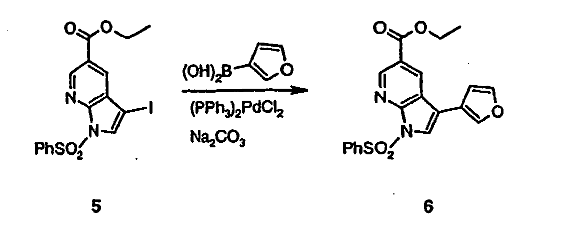

A mixture of 5 (100 mg, 0.219 mmol), EtOH (1.3 mL), toluene (1.3 mL), furan-3-boronic acid (37.4 mg, 0.33 mmol), 1M aq. Na₂CO₃ (0.55 mL, 0.55 mmol), LiCl (28 mg, 0.66 mmol) and PdCl₂(PPh₃)₂ (12.9 mg, 18.4 µmol) was refluxed for 17 min. The organic layer was separated, brine was added, and the aqueous layer was extracted with AcOEt. The combined organic solutions were concentrated and separated by means of silicagel chromatography using hexane:CH₂Cl₂ as eluent (in gradient up to 15% AcOEt) to give 6 as a tan solid (66.8 mg, 77%); ¹H NMR (400 MHz, CDCl₃) δ 1.42 (t, J = 7.1 Hz, 3H), 4.43 (q, J = 7.1 Hz, 2H), 6.71 (dd, J =1.8, 0.9 Hz, 1H), 7.48-7.54 (m, 2H), 7.56 (t, J =1.7 Hz, 1H), 7.58-7.63 (m, 1H), 7.85 (t, J = 0.9 Hz, 1H), 7.89 (s, 1H), 8.22-8.27 (m, 2H), 8.60 (d, J = 2.0 Hz, 1H), 9.12 (d, J = 2.0 Hz, 1H).

### 3-Furan-3-yl-1H-pyrrolo [2,3-b]pyridine-5-carboxylic acid (7)

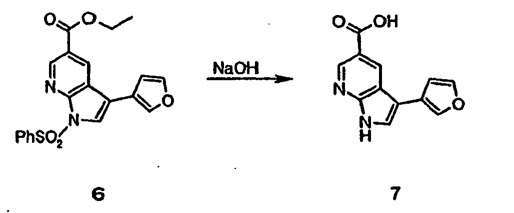

To a suspension of 6 (65.5 mg, 0.165 mmol) in EtOH (1.0 mL), was added 10% aqueous NaOH (0.5 mL, about 1.25 mmol), and the reaction mixture was refluxed for 0.5 h. The mixture was cooled to r.t. and EtOH was evaporated in vacuum. The residual solution was treated with glacial acetic acid (75 µL, 1.25 mmol). The suspension which formed was stirred at r.t. for 30 min. The solid was filtered off, washed with water, and dried under high vacuum to afford 7 as a tan powder (34.1 mg, 91 %); ¹H NMR (400 MHz, DMSO-*d₆*) δ 6.94 (d, J = 1.7 Hz, 1H), 7.74 (t, J =1.5 Hz, 1H), 7.88 (d, J = 2.2 Hz, 1H), 8.17 (s, 1H), 8.62 (d, J =1.8 Hz, 1H), 8.81 (d, J = 1.8 Hz, 1H), 12.16 (bs, 1H).

### 3-Furan-3-yl-1H-pyrrolo[2,3-b]pyridine-5-carboxylic acid (2-methoxyphenyl)-amide (8)

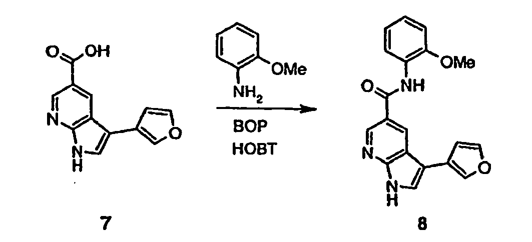

A mixture of 2-methoxy-phenylamine (21.55 mg, 0.175 mmol), carboxylic acid 7 (20 mg, 88 µmol), BOP (50.42 mg, 0.114 mmol), HOBT (17.8 mg, 0.132 mmol) and *i-*Pr₂NEt (30.7 µL, 0.176 mmol) in dry DMF (0.6 mL) was stirred at r.t. for 2 h. Separation of the crude reaction mixture by LCMS (column LUNA 10 µ C18(2) 00G-4253-V0 250x50 mm) using water - acetonitrile (0.1% AcOH) as eluent (in gradient; flow 80 mL/min) afforded amide 8 (13.11 mg, 45%) as a white solid. ¹H NMR (400 MHz, CDCl₃:5%CD₃OD) δ 3.93 (s, 3H), 6.71 (dd, J =1.8, 0.8 Hz, 1H), 6.94 (dd, J = 8.0, 1.5 Hz, 1H), 7.03 (ddd, J = 7.9, 7.7, 1.5 Hz, 1H), 7.10 (ddd, J = 8.0, 7.7, 1.7 Hz, 1H), 7.50 (s, 1H), 7.53 (t, J = 1.7 Hz, 1H), 7.84 (t, J=1.1 Hz, 1H), 8.48 (dd, J = 7.9, 1.7 Hz, 1H), 8.61 (d, J = 2.0 Hz, 1H), 8.81 (d, J=2.0 Hz, 1H); LCMS m/e 334 (M+H), 375 (M+MeCN+H).

### Synthesis of example inhibitor 16

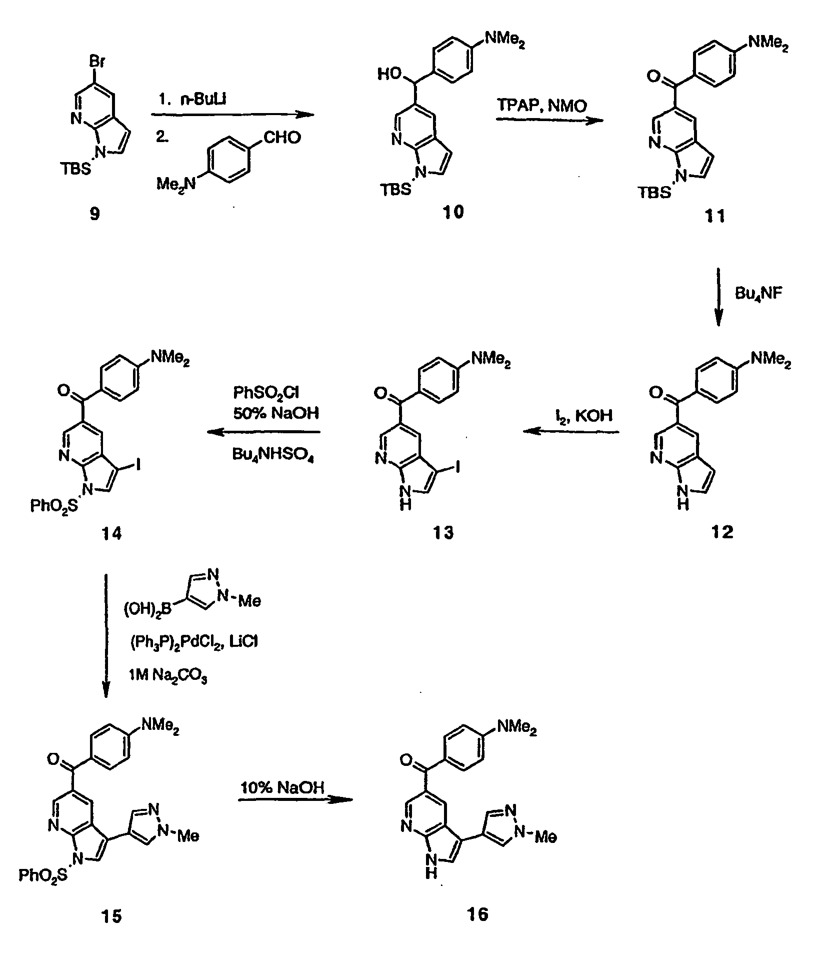

### [1-(tert-Butyl-dimethyl-silanyl)-1H-pyrrolo[2,3-b]pyridin-5-yl]-(4-dimethylamino-phenyl)-methanol (10)

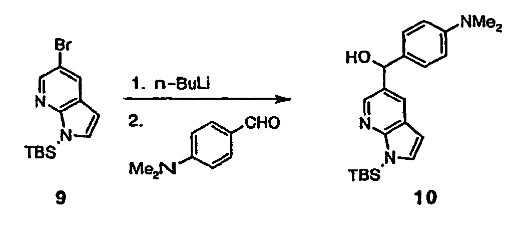

To a -78 °C stirred solution of the bromo-azaindole **9** (2 g, 6.4 mmol; preparation disclosed in W02004/078757) in THF (10 mL) was added a 2.5 M solution of *n*-butyllithium in hexanes (5.4 mL, 13.5 mmol) dropwise. The resulting yellow solution was stirred for 0.6 h at -78 °C and then 4-dimethylamino-benzaldehyde (1.25 g, 8.4 mmol) in THF (10 mL) was added slowly. The mixture was allowed to warm to room temperature and after a further 20 h diluted with EtOAc and saturated brine and partitioned. The aqueous layer was extracted with EtOAc (2x). The combined organic solutions were dried (MgSO₄) and concentrated *in vacuo.* The crude residue was purified by silica gel chromatography employing Et₃N-impregnated silica and AcOEt:hexane as eluent (gradient) to afford a 2.5:1 mixture of the alcohol **10** and 4-dimethylamino-benzaldehyde (1.63 g, 47%) as a yellow oil. The partially purified alcohol **10** was used directly for the next step without any further purification.

### [1-(tert-Butyl-dimethyl-silanyl)-1H-pyrrolo[2,3-b]pyridin-5-yl]-(4-dimethylamino-phenyl)-methanone (11)

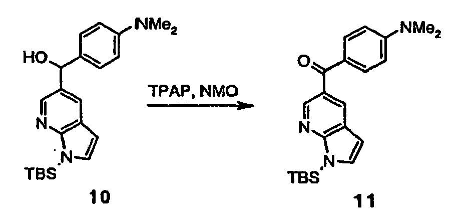

To a stirred solution of a 2.5:1 mixture of alcohol **11** and 4-dimethylamino-benzaldehyde (1.23 g, 2.3 mmol), 4-methylmorphohne N-oxide (0.41 g, 3.5 mmol), 4A powdered molecular sieves (1.2 g) in CH₂Cl₂ (12 mL) was added TPAP (82 mg, 0.23 mmol) in one portion. After 3 h the mixture was filtered through a pad of silica and the silica pad washed with CH₂Cl₂. The combined organic solutions were concentrated to afford **11** as black oil that was used directly in the next step without any purification. ¹H NMR (400 MHz; CDCl₃) δ 0.66 (s, 6H), 0.95 (s, 9H), 3.09 (s, 6H), 6.62 (*d*, J= 3.5 Hz, 1H), 6.70 (*d*, J=9.2 Hz, 2H), 7.31 (*d*, J= 3.5 Hz, 1H), 7.83 (d, J= 9.1 Hz, 2H), 8.27 (*d*, J= 2.1 Hz, 1H) and 8.71 (*d*, J= 2.1 Hz, 1H).

### (4-Dimethylamino-phenyl)-(1H-pyrrolo[2,3-b]pyridin-5-yl)-methanone (12)

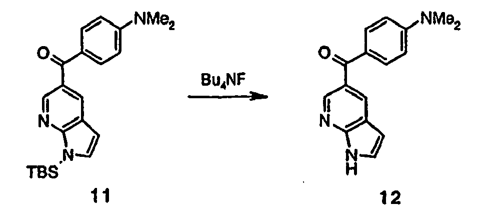

To a stirred solution of the crude ketone **11** (assumed 880 mg, 2.3 mmol) in THF (13 mL) was added 1M TBAF in THF (3.5 mL, 3.5 mmol) dropwise. After 3 h the mixture was concentrated to dryness and diluted with AcOEt and saturated brine, and partitioned. The aqueous layer was extracted with AcOEt (3x) and the combined organic extracts dried (MgSO₄), filtered and concentrated *in vacuo.* The residue was purified by silica gel chromatography using hexane:AcOEt (gradient elution) to afford ketone **12** (298 mg, 48% over 2 steps). ¹H NMR (400 MHz; CDCl₃) δ 3.10 (s, 6H), 6.62 (*d*, J= 3.4 Hz, 1H), 6.72 (*d*, J= 9.0 Hz, 2H), 7.42 (*d,* J= 3.3 Hz, 1H), 7.84 (*d,* J= 9.0 Hz, 2H), 8.38 (*d,* J= 1.9 Hz, 1H), 8.78 (*d,* J= 1.9 Hz, 1H) and 10.06 (brs, 1H).

### (4-Dimethylamino-phenyl)-(3-iodo-1H-pyrrolo[2,3-b]pyridin-5-yl)-methanone (13)

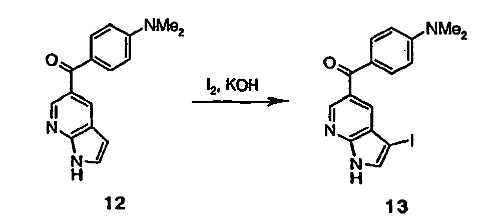

To a stirred solution of the ketone **12** (295 mg, 1.1 mmol) in DMF (7.5 mL) was added potassium hydroxide pellets (235 mg, 4.2 mmol). After 0.3 h, iodine (254 mg, 1.0 mmol) was added in one portion. Following a further 5 h the mixture was diluted with AcOEt and saturated sodium thiosulfate, and stirred vigoursly for 5 minutes. The mixture was partitioned and the aqueous layer extracted with AcOEt (3x). The combined organic extracts were dried (MgSO₄), filtered and concentrated *in vacuo* to afford iodide **13** as brown solid that was used directly in the next step without any purification.

### (1-Benzenesulfonyl-3-iodo-1H-pyrrolo[2,3-b]pyridin-5-yl)-(4-dimethylamino-phenyl)-methanone (14)

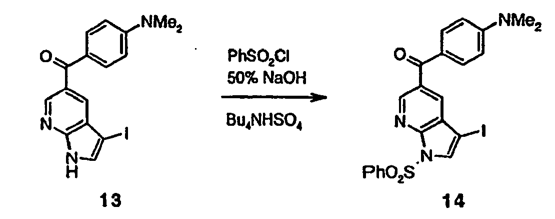

To a stirred solution of the iodide **13** (assumed 435 mg, 1.1 mmol) in CH₂Cl₂ (13 mL) was added benzenesulfonyl chloride (304 mg, 1.7 mmol), 50% NaOH (1 mL) and *n*-tetra-*n*-butyl ammonium sulfate (57 mg, 0.17 mmol). After 4.5 h the mixture was diluted with AcOEt and saturated sodium hydrogen carbonate solution and partitioned. The aqueous layer was extracted with EtOAc (3x) and the combined organic extracts were dried (MgSO₄), filtered and concentrated *in vacuo.* The residue was treated with MeOH and stirred vigoursly for 0.5 h and then vacuum filtered to afford the iodide **14** (410 mg, 69% over 2 steps) as a tan solid. ¹H NMR (400 MHz; CDCl₃) □ 3.10 (s, 6H), 6.69 (*J*= 9.1 Hz, 2H), 7.51 (m, 2H), 7.62 (tt, *J*= 1.2, 1.9 and 7.5 Hz, 1H), 7.77 (d, *J*= 9.1 Hz, 2H), 7.95 (s, 1H), 8.04 (d, *J*= 1.9 Hz, 1H), 8.23 (m, 2H), 8.79 (d, *J*= 1.9 Hz, 1H).

### [1-Benzenesulfonyl-3-(1-methyl-1H-pyrazol-4-yl)-1H-pyrrolo[2,3-b]pyridin-5-yl]-(4-dimethylamino-phenyl)-methanone (15)

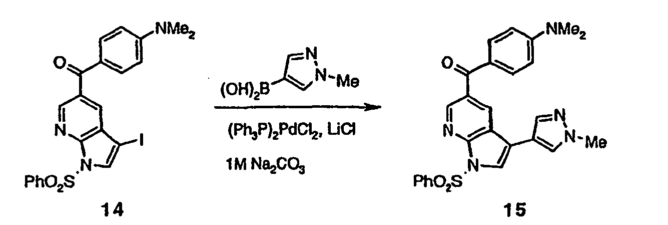

A mixture of iodide **14** (100 mg, 0.19 mmol), 1-methyl-1H-pyrazole-4-boronic acid (36 mg, 0.28 mmol), lithium chloride (24 mg, 0.56 mmol), dichlorobis(triphenylphosphine)-palladium (II) (7 mg, 0.01 mmol), 1M sodium carbonate (0.47 mL, 0.47 mmol) in toluene (2 mL) and ethanol (2 mL) was heated at 105 °C for 6 h. Then, the reaction mixture was cooled to room temperature and partitioned between AcOEt and saturated brine. The aqueous layer was extracted with AcOEt (3x). The combined organic extracts were dried (MgSO₄), filtered and concentrated. The residue was purified by preparative TLC using hexane:AcOEt=1:1 (v/v) as eluent to afford ketone **15** (51 mg, 56%). ¹H NMR (400 MHz; CDCl₃) δ 3.10 (s, 6H), 3.98 (s, 3H), 6.67 (d, *J*= 9.1 Hz, 2H), 7.51 (m, 2H), 7.59 (m, 1H), 7.69 (s, 1H), 7.79 (m, 3H), 7.86 (s, 1H), 8.23 (m, 2H), 8.36 (d, *J*=1.9 Hz, 1H) and 8.81 (d, *J*= 1.9 Hz, 1H).

### (4-Dimethylamino-phenyl)-[3-(1-methyl-1H-pyrazol-4-yl)-1H-pyrrolo[2,3-b]pyridin-5-yl]-methanone (16)

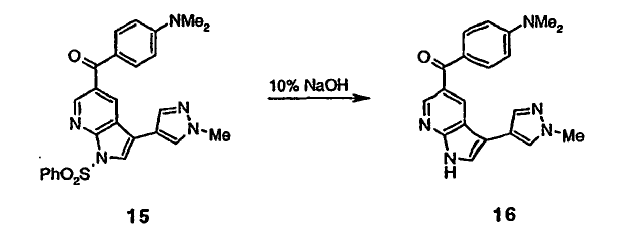

To a solution of the ketone **15** (51 mg, 0.11 mL) in ethanol (10 mL) was added 10% sodium hydroxide (1 mL) and the mixture stirred at 90 °C for 3 h. The mixture was concentrated to remove ethanol and partitioned between AcOEt and saturated brine. The aqueous layer was extracted with AcOEt (3x). The combined organic extracts were dried (MgSO₄), filtered and concentrated. The residual orange oil was purified by preparative TLC with AcOEt as eluent to afford inhibitor **16** (14 mg, 39%). ¹H NMR (400 MHz; CDCl₃) δ 3.10 (s, 6H), 3.98 (s, 3H), 6.72 (d, *J*=9.1 Hz, 2H), 7.50 (d, *J*=2.0 Hz, 1H), 7.68 (s, 1H), 7.76 (s, 1H), 7.86 (d, *J*= 9.1 Hz, 2H), 8.51 (d, *J*= 1.9 Hz, 1H), 8.78 (d, *J*= 1.9 Hz, 1H) and 10.28 (brs, 1H).

### Biological activity

### JNK1, JNK2, JNK3 - SPA assay

1. Compound is dissolved in DMSO to a convenient concentration and this is diluted in 10% DMSO to a five times concentrate of the desired starting concentration (frequently 1:100).
2. 10 µl of 500 mM EDTA is added to alternative wells of the Opti-plate row, which will receive kinase reaction plus DMSO. This creates the negative control.
3. For the JNK2 and JNK3 assay, compounds are prepared in six 2-fold dilutions with water and each concentration is tested in duplicate. For the JNK1 assay compounds are prepared in four 5-fold dilutions with water which are tested in triplicate. Controls are treated identically.
4. 20 µl per well of each compound concentration is transferred to an Opti-plate, in duplicate.
5. 30 µl (JNK2/3 SPA) or 50 µl (JNK1 SPA) of substrate solution (25 mM HEPES pH 7.5, 10mM magnesium acetate with 3.33µM ATP (JNK2/3) or 2µM ATP (JNK1), approximately 7.5 kBq [γ-³³P] ATP, GST-c-Jun, in water) is added to each well.
6. 50 µl (JNK2/3 SPA) or 30 µl (JNK1 SPA) of kinase solution (JNK in 25 mM HEPES pH 7.5, 10mM Mg Acetate) is added to each well.

| Kinase | Kinase per well (µg) | GST-c-Jun per well (µg) |
|---|---|---|
| JNK1 | 0.25 | 1 |
| JNK2 | 0.2 | 1.2 |
| JNK3 | 0.16 | 1.2 |

7. The plate is incubated for 30 minutes at room temperature.
8. 100 µl of bead/stop solution is added to each well (5 mg/ml glutathione-PVT-SPA beads, 40 mM ATP in PBS).
9. Plates are sealed and incubated for 30 minutes at room temperature, centrifuged for 10 minutes at 2500g and counted.
10. The IC₅₀ values are calculated as the concentration of the compound being tested at which the phosphorylation of c-Jun is decreased to 50% of the control value. Example IC₅₀ values for the compounds of this invention are given in Table 1.

### p38 ELISA

Active p38 kinase (100 ng; Upstate) was added to 2 µg GST-ATF2 substrate (NEB) in 250 mM Hepes pH 7.5/100 mM MgAc/50 µM ATP (final) in the presence or absence of compounds in 50 µl. The mixture was incubated at 30°C for 1 hour, and then diluted with 200 µl PBS-Tween (0.05 %). From this, duplicate volumes of 100 µl were added to a Reacti-Bind glutathione coated plate (Pierce) and incubated for 1 hour. After washing 3 times with PBS-Tween (0.05 %), rabbit anti-phospho-A'TF2 (Thr71) antibody (NEB) was added at 1:500, and incubated for another hour at room temperature. After 3 additional washes with PBS-Tween (0.05 %), 100 µl of anti-rabbit IgG alkaline phosphatase-conjugated secondary antibody (Sigma) was added at 1:1000, the reaction was incubated for a further hour, washed 3 times, and then phosphatase substrate (Sigma) was added (100 µl per well; 3 tablets in 5 ml water). After incubation in the dark at 37°C for 1 hour, the reaction mixture was transferred to a clear 96 well plate, and the absorbance at 405 nm was read. The IC₅₀ values are calculated as the concentration of the compound being tested at which the phosphorylation of ATF2 is decreased to 50% of the control value. Example IC₅₀ values for the compounds of this invention are given in Table 1 (last column).

**Table 1. IC₅₀ values for selected compounds against JNK1, JNK2, JNK3, and p38 MAP kinase**

| Compound | JNK3 IC₅₀ (nM) |
|---|---|
| 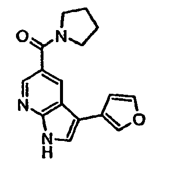 | <500 |
| 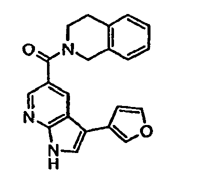 | <500 |
| 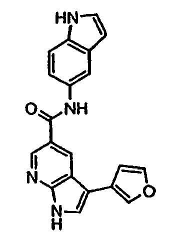 | <1000 |
| 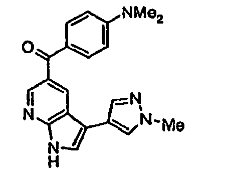 | <500 |
| 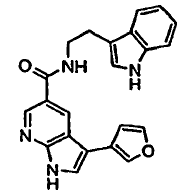 | <500 |

## Claims

1. A compound of formula (I): 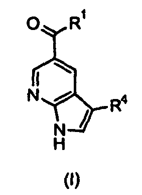 and the pharmaceutically acceptable salts;
wherein R¹ is a C₃₋₁₂ carbocyclyl or C₃₋₁₂ heterocyclyl group optionally fused to a partially saturated, unsaturated or fully saturated five to seven membered ring containing zero to three heteroatoms, and each substitutable carbon atom in R', including the optional fused ring, is optionally and independently substituted by one or more of halogen, C₁₋₁₂ alkyl, C₂₋₁₂ alkenyl, C₂₋₁₂ alkynyl, haloC₁₋₁₂alkyle, C₃₋₁₂ carbocyclyl, C₃₋₁₂ heterocyclyl, (CH₂)ₙOR⁵, (CH₂)ₙNR⁵₂(CH₂)ₙSR⁵, OR⁵, SR⁵, NO₂, CN, NR⁵₂, NR⁵COR⁵, NR⁵CONR⁵₂, NR⁵COR⁵, NR⁵CO₂R⁵, CO₂R⁵, COR⁵, CONR⁵₂, S(O)₂R⁵, SONR⁵₂, S(O)R⁵, SO₂NR⁵₂, or NR⁵S(O)₂R⁵ wherein the C₁₋₁₂ alkyl group optionally contains one or more insertions selected from -O-, -N(R⁵)- -S-, -S(O)- and -S(O₂)-; and each saturated carbon in the optional fused ring is further optionally and independently substituted by =O, =S, NNR⁶₂, =N-OR⁶, =NNR⁶COR⁶, =NNR⁶CO₂R⁶, =NNSO₂R⁶, or =NR⁶; and each substitutable nitrogen atom in R¹ is optionally substituted by R⁷, COR⁷, SO₂R⁷ or CO₂R⁷_{;}
wherein n is 1 to 6, preferably n is 1, 2 or 3;
wherein R⁵ is hydrogen , C₁₋₁₂ alkyl, C₃₋₁₂ carbocyclyl or C₃₋₁₂ heterocyclyl, optionally substituted by one or more of C₁₋₆ alkyl, C₃₋₁₂ carbocyclyl, C₃₋₁₂ heterocyclyl, halogen, C₁₋₆ haloalkyl, OR⁸, SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S(O)₂R⁸, SONR⁸₂, S(O)R⁸, SO₂NR⁸R⁸, NR⁸S(O)₂R⁸, wherein the C₁₋₁₂ alkyl group optionally incorporates one or two insertions selected from the group consisting of -O-, -N(R⁸)-, -S(O)- and -S(O₂)-, wherein each R⁸ may be the same or different and is as defined below;
wherein two R⁵ in NR⁵₂ may optionally form a partially saturated, unsaturated or fully saturated three to seven membered ring containing one to three heteroatoms, optionally and independently substituted by one or more of C₁₋₆ alkyl, halogen, C₁₋₆ haloalkyl, OR⁸, SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S(O)₂R⁸, SONR⁸₂, S(O)R⁸, SO₂NR⁸R⁸, NR⁸S(O)₂R⁸,
wherein the C₁₋₆ alkyl group optionally incorporates one or two insertions selected from the group consisting of -O-, -N(R⁸)-, -S(O)- and -S(O₂)-, wherein each R⁸ may be the same or different and is as defined below;
wherein R⁶ is hydrogen, C₁₋₁₂ alkyl, C₃₋₁₂ carbocyclyl or C₃₋₁₂ heterocyclyl, optionally substituted by one or more of C₁₋₆ alkyl, halogen, C₁₋₆ haloalkyl, OR⁸ SR⁸, NO₂, CN, NR⁸R⁸, NR⁸COR⁸ NR⁸CONR⁸R⁸, NR⁸COR⁸, NR⁸CO₂R⁸, CO₂R⁸, COR⁸, CONR⁸₂, S(O)₂R⁸, S(O)R⁸, SO₂NR⁸R⁸, NR⁸S(O)₂R⁸, wherein the C₁₋₁₂ alkyl group optionally incorporates one or two insertions selected from the group consisting of -O-, -N(R⁸)-, -S(O)- and -S(O₂)-, wherein each R⁸ may be the same or different and is as defined below;
wherein R⁷ is hydrogen, C₆₋₁₂ aryl, C₁₋₆ alkyl or C₁₋₆ haloalkyl;
wherein R⁸ is hydrogen, C₁₋₆ alkyl, or C₁₋₆ haloalkyl or wherein R¹ is a group of formula (II) 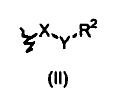
wherein X is NR³, O, S or (CR²²R²²)ₙ, Y is absent or is NR²³, O, or (CR²³R²³)ₙ, wherein each R²³ is H, C₁₋₄ alkyl, C₁₋₄ alkoxy or C₁₋₄ haloalkyl; and n is 1 to 6, preferably n is 1, 2, 3 or 4; and
R² is optionally substituted C₁₋₁₂ alkyl, C₂₋₁₂ alkenyl, C₂₋₁₂ alkynyl, C₃₋₁₂ carbocyclyl or C₃₋₁₂ heterocyclyl, , wherein the optionally substituted carbocyclyl or heterocyclyl group is optionally fused to one to three unsaturated, partially unsaturated or fully saturated five to seven membered rings containing zero to three heteroatoms;
each substitutable carbon atom in R², including the optional fused ring, is optionally and independently substituted by one or more of C₁₋₁₂ alkyl, C₃₋₁₂ cycloalkyl, C₃₋₁₂ heterocycloalkyl, C₃₋₁₂ aryl, C₃₋₁₂ heteroaryl halogen, C₁₋₁₂ haloalkyl, OR⁹, SR⁹, NO₂, CN, NR⁹R⁹, NR⁹COR⁹, NR⁹CONR⁹R⁹, NR⁹COR⁹, NR⁹CO₂R⁹, CO₂R⁹, COR⁹, CONR⁹R⁹, S(O)₂R⁹, SONH₂, S(O)R⁹, SO₂NR⁹R⁹, NR⁹S(O)₂R⁹, wherein each R⁹ may be the same or different and is as defined below and wherein:
the C₁₋₁₂ alkyl group optionally incorporates one or two insertions selected from the group consisting of -O-, -C(O)-, -N(R⁹)-, -S(O)- and -S(O₂)-, wherein each R⁹ may be the same or different and is as defined above;
the C₁₋₁₂ alkyl, C₃₋₁₂ cycloalkyl, C₃₋₁₂ heterocycloalkyl, C₃₋₁₂ aryl, or C₃₋₁₂ heteroaryl groups are optionally substituted by one or more of halogen, C₁₋₁₂ haloalkyl, OR⁹, SR⁹, NO₂, CN, NR⁹R⁹, NR⁹COR⁹, NR⁹CONR⁹R⁹, NR⁹COR⁹, NR⁹CO₂R⁹, CO₂R⁹, COR⁹, CONR⁹R⁹, S(O)₂R⁹, SONH₂, S(O)R⁹, SO₂NR⁹R⁹, NR⁹S(O)₂R⁹, wherein each R⁹ may be the same or different and is as defined below; and
the C₃₋₁₂ cycloalkyl, C₃₋₁₂ heterocycloalkyl, C₃₋₁₂ aryl, or C₃₋₁₂ heteroaryl groups are optionally substituted by one or more C₁₋₁₂ alkyl groups;
each saturated carbon in R², including the optional fused ring, is further optionally and independently substituted by =O, =S, NNR⁹R⁹, =N-OR⁹, =NNHCOR⁹, =NNHCO₂R⁹, =NNSO₂R⁹, or =NR⁹, wherein each R⁹ may be the same or different and is as defined below; and
each substitutable nitrogen atom in R² is optionally substituted by R¹⁰, COR⁹, SO₂R⁹ or CO₂R⁹ wherein each R⁹ and R¹⁰ may be the same or different and is as defined below;
wherein two R⁹ in NR⁹₂ may optionally form a partially saturated, unsaturated or fully saturated three to seven membered ring containing one to three heteroatoms, optionally and independently substituted by one or more of C₁₋₆ alkyl, halogen, C₁₋₆ haloalkyl, OR", SR¹¹, NO₂, CN, NR¹¹R¹¹, NR¹¹COR¹¹, NR¹¹CONR¹¹R¹¹, NR¹¹COR¹¹, NR¹¹CO₂R¹¹, CO₂R¹¹, COR¹¹, CONR¹¹₂, S(O)₂R¹¹, SONR¹¹₂, S(O)R¹¹, SO₂N¹¹R¹¹, NR¹¹S(O)₂R¹¹,
wherein the C₁₋₆ alkyl group optionally incorporates one or two insertions selected from the group consisting of -O-, -N(R¹¹⁾-, -S(O)- and -S(O₂)-, wherein each R¹¹ may be the same or different and is as defined below; wherein R¹¹ is hydrogen, C₁₋₆ alkyl, or C₁₋₆ haloalkyl;
wherein R⁹ is hydrogen , C₁₋₁₂ alkyl or C₃₋₁₂ aryl, optionally substituted by one or more of C₁₄ alkyl, halogen, C₁₄ haloalkyl, OR¹², SR¹², NO₂, CN, NR¹²R¹², NR¹²COR¹², NR¹²CONR¹²R¹², NR¹²COR¹², NR¹²CO₂R¹², CO₂R¹², COR¹², CONR¹²₂, S(O)₂R¹², SONH₂, S(O)R¹², SO₂ NR¹²R¹², NR¹²S(O)₂R¹², wherein the C₁₋₁₂ alkyl group optionally incorporates one or two insertions selected from the group consisting of -O-, -N(R¹²)-, -S(O)- and -S(O₂)-, wherein each R¹² may be the same or different and is as defined below;
wherein R¹⁰ is C₁₋₁₂ alkyl or C₃₋₁₂ aryl, optionally substituted by one or more of C₁₄ alkyl, halogen, C₁₋₄ haloalkyl, OR¹², SR¹² NO₂, CN, NR¹²R¹², NR¹²COR¹², NR¹²CONR¹²R¹², NR¹²COR¹² NR¹²CO₂R¹², CO₂R¹², COR¹², CONR¹²₂, S(O)₂R¹², SONH₂, S(O)R¹², SO₂NR¹²R¹², NR¹²S(O)₂R¹², wherein the C₁₋₁₂ alkyl group optionally incorporates one or two insertions selected from the group consisting of -O-, -N(R¹²)-, -S(O)- and -S(O₂)-, wherein each R¹² may be the same or different and is as defined below;
wherein R¹² is hydrogen, C₁₋₄ alkyl, or C₁₋₄ haloalkyl;
wherein X is NR³; O, S or (CR²²-R²²)ₙ wherein R²² is independently one or more of halogen, C₁₋₁₂ alkyl, C₂₋₁₂ alkenyl, Z₂₋₁₂ alkynyl, C₁₋₁₂ haloalkyl, C₆₋₁₂ carbocyclyl, C₅₋₁₂ heterocyclyl, (CH₂)ₙOR⁵, (CH₂)ₙNR⁵₂, OR⁵, SR⁵, NO₂, CN, NR⁵₂, NR⁵COR⁵, NR⁵CONR⁵₂, NR⁵COR⁵, NR⁵CO₂R⁵, CO₂R⁵, COR⁵, CONR⁵₂, S(O)₂R⁵, SONR⁵₂, S(O)R⁵, SO₂NR⁵₂, or NR⁵S(O)₂R⁵ wherein each R⁵ may be the same or different and is as defined above; and
wherein n is 1 to 6, preferably n is 1, 2, 3 or 4;
and wherein R³ is a lone electron pair, hydrogen, C₁₋₁₂ alkyl, C₂₋₁₂ alkenyl, C₂₋₁₂ alkynyl, C₃₋₁₂ carbocyclyl or C₃₋₁₂ heterocyclyl, each of which is optionally substituted, wherein:
the optionally substituted carbocyclyl or heterocyclyl group is optionally fused to one to three unsaturated, partially unsaturated or fully saturated five to seven membered rings containing zero to three heteroatoms,
each substitutable carbon atom in R³, including the optional fused ring, is optionally and independently substituted by one or more of C₁₋₁₂ alkyl, C₃₋₁₂ cycloalkyl, C₃₋₁₂ heterocycloalkyl, C₃₋₁₂ aryl, C₃₋₁₂ heteroaryl halogen, C₁₋₁₂ haloalkyl, OR¹³, SR¹³, NO₂, CN, NR¹³R¹³, NR¹³COR¹³, NR¹³CONR¹³R¹³ , NR¹³COR¹³, NR¹³CO₂R¹³, CO₂R¹³, COR¹³, CONR¹³R¹³, S(O)₂R¹³, SONH₂, S(O)R¹³, SO₂NR¹³R¹³, NR¹³S(O)₂R¹³, wherein each R¹³ may be the same or different and is as defined above and wherein:
the C₁₋₁₂ alkyl group optionally incorporates one or two insertions selected from the group consisting of -O-, -C(O)-, -N(R¹³)-, -S(O)- and -S(O₂)-, wherein each R¹³ may be the same or different and is as defined above;
the C₁₋₁₂ alkyl, C₃₋₁₂ cycloalkyl, C₃₋₁₂ heterocycloalkyl, C₃₋₁₂ aryl, or C₃₋₁₂ heteroaryl groups are optionally substituted by one or more of halogen, C₁₋₁₂ haloalkyl, OR¹³, SR¹³ NO₂, CN, NR¹³R¹³, NR¹³COR¹³, NR¹³CONR¹³R¹³, NR¹³COR¹³, NR¹³CO₂R¹³, CO₂R¹³, COR¹³, CONR¹³R¹³, S(O)R¹³, SONH₂, S(O)R¹³, SO₂NR¹³R¹³, NR¹³S(O)₂R¹³, wherein each R¹³ may be the same or different and is as defined below; and
the C₃₋₁₂ cycloalkyl, C₃₋₁₂ heterocycloalkyl, C₃₋₁₂ aryl, or C₃₋₁₂ heteroaryl groups are optionally substituted by one or more C₁₋₁₂ alkyl groups;
each saturated carbon in R², including the optional fused ring, is further optionally and independently substituted by =O, =S, NNR¹³R¹³, =N-OR¹³, =NNHCOR¹³, =NNHCO₂R¹³, =NNSO₂R¹³, or =NR¹³, wherein each R¹³ may be the same or different and is as defined below; and
each substitutable nitrogen atom in R³ is optionally substituted by R¹⁴, COR¹³, SO₂R¹³ or CO₂R¹³ wherein each R¹³ and R¹⁴ may be the same or different and is as defined below;
wherein two R¹³ in NR¹³₂ may optionally form a partially saturated, unsaturated or fully saturated three to seven membered ring containing one to three heteroatoms, optionally and independently substituted by one or more of C₁₋₆ alkyl, halogen, C₁₋₆ haloalkyl, OR¹⁵, SR¹⁵, NO₂, CN, NR¹⁵R¹⁵, NR¹⁵COR¹⁵, NR¹⁵CONR¹⁵R¹⁵, NR¹⁵COR¹⁵, NR¹⁵COR¹⁵, CO₂R¹⁵, COR¹⁵, CONR¹⁵₂, S(O)₂R¹⁵, SONR¹⁵₂, S(O)R¹⁵, SO₂NR¹⁵R¹⁵, NR¹⁵S(O)₂R¹⁵,
wherein the C₁₋₆ alkyl group optionally incorporates one or two insertions selected from the group consisting of -O-, -N(R¹⁵)-, -S(O)- and -S(O₂)-,
wherein each R¹⁵ may be the same or different and is as defined below;
wherein R¹⁵ is hydrogen, C₁₋₆ alkyl, or C₁₋₆ haloalkyl;
wherein R¹³ is hydrogen , C₁₋₁₂ alkyl or C₃₋₁₂ aryl, optionally substituted by one or more of C₁₋₄ alkyl, halogen, C₁₋₄ haloalkyl, OR¹⁶, SR¹⁶, NO₂, CN, NR¹⁶ R¹⁶, NR¹⁶COR¹⁶, NR¹⁶CONR¹⁶R¹⁶, NR¹⁶COR¹⁶, NR¹⁶CO₂R¹⁶, CO₂R¹⁶, COR¹⁶, CONR¹⁶₂, S(O)₂R¹⁶, SONH₂, S(O)R¹⁶, SO₂ NR¹⁶R¹⁶, NR¹⁶S(O)₂R¹⁶, wherein the C₁₋₁₂ alkyl group optionally incorporates one or two insertions selected from the group consisting of -O-, -N(R¹⁶)-, -S(O)- and -S(O₂)-, wherein each R¹⁶ may be the same or different and is as defined below;
wherein R¹⁴ is C₁₋₁₂ alkyl or C₃₋₁₂ aryl, optionally substituted by one or more of C₁₋₄ alkyl, halogen, C₁₋₄haloalkyl, OR¹⁶, SR¹⁶, NO₂, CN, NR¹⁶R¹⁶, NR¹⁶COR¹⁶, NR¹⁶CONR¹⁶R¹⁶, NR¹⁶COR¹⁶ , NR¹⁶COR¹⁶, COR¹⁶, COR¹⁶, CONR¹⁶₂, S(O)₂R¹⁶, SONH₂, S(O)R¹⁶, SO₂NR¹⁶R¹⁶, NR¹⁶S(O)₂R¹⁶, wherein the C₁₋₁₂ alkyl group optionally incorporates one or two insertions selected from the group consisting of -O-, -N(R¹⁶)-, -S(O)- and -S(O₂)-, wherein each R¹⁶ may be the same or different and is as defined below;
wherein R¹⁶ is hydrogen, C₁₋₄ alkyl, or C₁₋₄ haloalkyl;
wherein when X is NR², R² and R³ can form a 3 to 12 membered heterocyclyl ring, more preferably a 5, 6, 7, 8, 9, 10, 11 or 12 membered ring, wherein said ring can be partially saturated, unsaturated or fully saturated containing one to three heteroatoms; wherein the heterocyclylic group formed by R² and R³ can be optionally fused to one to three unsaturated, partially saturated or fully saturated 5 to 7 membered rings containing zero to three heteroatoms, any of said rings being optionally and independently substituted with one or more of C₁₋₆ alkyl, halogen, C₁₋₆ haloalkyl, OR²², SR²², NO₂, CN, NR²²R²², NR²²COR²², NR²²CONR²²R²², NR²²COR²², NR²²CO₂R²², CO₂R²², COR²², CONR²²₂, S(O)₂R²², SONR²²₂, S(O)R²², SO₂NR²²R²², NR²²S(O)₂R²², wherein the C₁₋₆ alkyl group optionally incorporates one or two insertions from -0-, -N(R²²)-, - S(O)- and -S(O₂)- and wherein each R²² may be the same or different,
wherein R⁴ is a six-membered carbocyclyl group or a five or six-membered heterocyclyl group containing from 1 to 4 heteroatoms independently selected from N, S or O, wherein the optionally substituted six-membered carbocyclyl or five or six-membered heterocyclyl group is optionally fused to a partially saturated, unsaturated or fully saturated five to seven membered ring containing zero to three heteroatoms, and each substitutable carbon or heteroatom in R⁴ including the optional fused ring, is optionally and independently substituted by one or more of halogen, C₁₋₁₂ alkyl, C₂₋₁₂ alkenyl, C₂₋₁₂ alkynyl, C₁₋₁₂ haloalkyl, C₃₋₁₂ carbocyclyl, C₃₋₁₂ heterocyclyl, (CH₂)ₙOR¹⁷, (CH₂)ₙNR¹⁷₂, OR¹⁷, SR¹⁷, NO₂, CN, NR¹⁷₂, NR¹⁷COR¹⁷ , NR¹⁷CONR¹⁷ 2, NR¹⁷COR¹⁷, NR¹⁷CO₂R¹⁷, CO₂R¹⁷, COR¹⁷, CONR¹⁷₂, S(O)₂R¹⁷, SONR¹⁷₂, S(O)R¹⁷, SO₂NR¹⁷₂, or NR¹⁷S(O)₂R¹⁷, wherein the C₁₋₁₂ alkyl group optionally contains one or more insertions selected from -O-, -N(R¹²)- -S-, -S(O)- and -S(O₂)-; and each saturated carbon in the optional fused ring is further optionally and independently substituted by =O, =S, NNR¹⁸₂, =N-OR¹⁸, =NNR¹⁸COR¹⁸, =NNR¹⁸CO₂R¹⁸, =NNSO₂R¹⁸, or =NR¹⁸; and each substitutable nitrogen atom in R⁴ is optionally substituted by R¹⁹, COR¹⁹, SO₂R¹⁹ or CO₂R¹⁹; wherein n is 1 to 6, preferably n is 1, 2 or 3; preferably, wherein each substitutable carbon or hetero-atom in R⁴ is optionally and independently substituted by one or more of C₁₋₆ alkyl, OR²⁰, SR²⁰, NO₂, CN, NR²⁰₂, NR²⁰COR²⁰, NR²⁰CONR²⁰₂, NR²⁰COR²⁰, NHCO₂R²⁰, CO₂R²⁰, COR²⁰, CONR²⁰, S(O)₂R²⁰, SONR²⁰₂, S(O)R²⁰, SO₂NR²⁰₂, or NR²⁰S(O)₂R²⁰;
wherein R²⁰ is hydrogen, C₁₋₆ alkyl, or C₁₋₆haloalkyl;
wherein R¹⁷ is hydrogen , C₁₋₁₂ alkyl, C₃₋₁₂ carbocyclyl or C₃₋₁₂ heterocyclyl, optionally substituted by one or more of C₁₋₆ alkyl, C₃₋₁₂ carbocyclyl, C₃₋₁₂ heterocyclyl, halogen, C₁₋₆ haloalkyl, OR²¹, SR²¹, NO₂, CN, NR²¹R²¹, NR²¹COR²¹, NR²¹CONR²¹R²¹, NR²¹COR²¹, NR²¹CO₂R²¹, CO₂R²¹, COR²¹, CONR²¹₂, S(O)₂R²¹, SONR²¹₂, S(O)R²¹, SO₂NR²¹R²¹, NR²¹S(O)₂R²¹, wherein the C₁₋₁₂ alkyl group optionally incorporates one or two insertions selected from the group consisting of -O-, -N(R²¹)-, -S(O)- and -S(O₂)-, wherein each R²¹ may be the same or different and is as defined below;
wherein two R¹⁷ in NR¹⁷ may optionally form a partially saturated, unsaturated or fully saturated three to seven membered ring containing one to three heteroatoms, optionally and independently substituted by one or more of C₁₋₆ alkyl, halogen, C₁₋₆, haloalkyl, OR²¹, SR²¹, NO₂, CN, NR²¹R²¹, NR²¹COR²¹, NR²¹CONR²¹R²¹ NR²¹COR²¹, NR²¹CO₂R²¹, COR²¹, COR²¹, CONR²¹₂, S(O)₂R²¹, SONR²¹₂, S(O)R²¹, SO₂NR²¹R²¹, NR²¹S(O)₂R²¹, wherein the C₁₋₆ alkyl group optionally incorporates one or two insertions selected from the group consisting of -O-, -N(R²¹)-, -S(O)- and -S(O₂)-, wherein each R²¹ may be the same or different and is as defined below;
wherein R¹⁸ is hydrogen, C₁₋₁₂ alkyl, C₃₋₁₂ carbocyclyl or C₃₋₁₂ heterocyclyl, optionally substituted by one or more of C₁₋₆ alkyl, halogen, C₁₋₆ haloalkyl, OR²¹, SR²¹, NO₂, CN, NR²¹R²¹, NR²¹COR²¹, NR²¹CONR²¹R²¹, NR²¹COR²¹ , NR²¹CO₂R²¹, CO₂R²¹, COR²¹, CONR²¹₂, S(O)₂R²¹, S(O)R²¹, SO₂NR²¹R²¹, NR²¹S(O)₂R²¹, wherein the C₁₋₁₂ alkyl group optionally incorporates one or two insertions selected from the group consisting of -O-, -N(R²¹)-, -S(O)- and - S(O₂)-, wherein each R²¹ may be the same or different and is as defined below;
wherein R¹⁹ is hydrogen, C₆₋₁₂ aryl, C₁₋₆ alkyl or C₁₋₆ haloalkyl;
wherein R²¹ is hydrogen, C₁₋₆ alkyl, or C₁₋₆ haloalkyl.

2. A compound as claimed in claim 1 wherein R¹ is an optionally substituted five or six membered carbocyclyl or heterocyclyl group selected from optionally substituted phenyl, acridine, benzimidazole, benzofuran, benzothiophene, benzoxazole, benzothiazole, cyclohexyl furan, imidazole, indole, isoindole, isoquinoline, isoxazole, isothiazole, morpholine, napthaline, oxazole, phenazine, phenothiazine, phenoxazine, piperazine, piperidine, pyrazole, pyridazine, pyridine, pyrrole, quinoline, quinolizine, tetrahydrofuran, tetrazine, tetrazole, thiophene, thiazole, thiomorpholine, thianaphthalene, thiopyran, triazine, triazole or trithiane.

3. A compound as claimed in claim 1 or claim 2 wherein R¹ is a group of formula (II), wherein X is a group NR³, Y is absent and one or more of R² and R³ is hydrogen, alkyl or cycloalkyl.

4. A compound as claimed in claim 3 wherein the group of formula (II) is an alkylamino or cycloalkylamino group preferably selected from optionally substituted methylamino, ethylamino, propylamino, isopropylamino, butylamino, cyclobutylamino, pentylamino, cyclopentylamino, hexylamino, cyclohexylamino, heptylamino, cycloheptylamino, octylamino and cyclooctylamino.

5. A compound as claimed in any one of claims 1 to 4 wherein R¹ is substituted with one or more of OR²⁴, halogen, C₁₋₆ alkyl, C₂₋₆ alkenyl, C₂₋₆ alkynyl, C₁₋₆haloalkyl, C₁₋₆alkylaryl, C₁₋₆alkylheterocyclyl, (CH₂)ₙOR²⁴, (CH₂)ₙNR²⁴₂, SR²⁴, NO₂, CN, NR²⁴2, CO₂R²⁴, NR²⁴C(O)R²⁴, NR²⁴S(O)₂R²⁴, COR²⁴, CONR²⁴₂, S(O)₂R²⁴₂, S(O)R²⁴ or SO₂NR²⁴₂;
wherein R²⁴ is hydrogen, C₁₋₄ alkyl or C₆₋₁₂ aryl preferably phenyl, or C₅₋₁₂ heterocyclyl preferably pyridine, and n is 1, 2, 3, 4, 5 or 6;
wherein two R²⁴ in NR²⁴₂ may optionally form a partially saturated, unsaturated or fully saturated three to seven membered ring containing one to three heteroatoms, said ring is preferably independently substituted with one or more of halogen, C₁₋₁₂ alkyl, C₂₋₁₂ alkenyl, C₂₋₁₂ alkynyl, C₁₋₁₂ haloalkyl, C₃₋₁₂ carbocyclyl, C₃₋₁₂ heterocyclyl, OR²⁵, SR²⁵, NO₂, CN, NR²⁵₂, NR²⁵COR²⁵, NR²⁵CONR²⁵₂, NR²⁵COR²⁵, NR²⁵CO₂R²⁵, CO₂R²⁵, COR²⁵, CONR²⁵₂, S(O)₂R²⁵, SONR²⁵₂, S(O)R²⁵, SO₂NR²⁵₂, or NR²⁵S(O)₂R²⁵; and each saturated carbon in the optional ring is further optionally and independently substituted by =O, =S, NNR²⁶₂, =N-OR²⁶, =NNR²⁶COR²⁶, =NNR²⁶CO₂R²⁶, =NNSO₂R²⁶, or =NR²⁶; and each substitutable nitrogen atom is optionally substituted by R²⁷, COR²⁷, SO₂R²⁷ or CO₂R²⁷;
wherein R²⁵ is hydrogen , C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR²⁸, SR²⁸, NO₂, CN, NR²⁸R²⁸, NR²⁸COR²⁸, NR²⁸CONR²⁸R²⁸, NR²⁸COR²⁸, NR²⁸CO₂R²⁸, CO₂R²⁸, COR²⁸, CONR²⁸₂, S(O)₂R²⁸, SONR²⁸₂, S(O)R²⁸, SO₂NR²⁸R²⁸, NR²⁸S(O)₂R²⁸, wherein the C₁₋₁₂ alkyl group optionally incorporates one or two insertions selected from the group consisting of -0-, - N(R²⁸)-, -S(O)- and -S(O₂)-, wherein each R²⁸ may be the same or different and is as defined below;
wherein R²⁶ is hydrogen, C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR²⁸, SR²⁸, NO₂, CN, NR²⁸R²⁸, NR²⁸COR²⁸, NR²⁸CONR²⁸R²⁸, NR²⁸COR²⁸, NR²⁸CO₂R²⁸, CO₂R²⁸, COR²⁸, CONR²⁸₂, S(O)₂R²⁸, S(O)R²⁸, SO₂NR²⁸R²⁸, NR²⁸S(O)₂R²⁸, wherein the C₁₋₁₂ alkyl group optionally incorporates one or two insertions selected from the group consisting of -O-, -N(R²⁸)-, -S(O)- and - S(O₂)-, wherein each R²⁸ may be the same or different and is as defined below;
wherein R²⁷ is hydrogen, C₁₋₆ alkyl, C₁₋₆ haloalkyl or C₆₋₁₂ aryl;
wherein R²⁸ is hydrogen, C₁₋₆ alkyl, or C₁₋₆ haloalkyl.

6. A compound as claimed in any one of claims 1 to 5 wherein R⁴ is selected from phenyl, cyclohexyl, acridine, benzimidazole, benzofuran, benzothiophene, benzoxazole, benzothiazole, indole, isoindole, indolizine, indazole, isoindole, isoquinoline, morpholine, napthalene, phenazine, phenothiazine, phenoxazine, piperazine, piperidine, pyridazine, pyridine, pyrimidinyl, pyrazinyl, quinoline, quinolizine, tetrazine,
thiomorpholine, thianaphthalene, thiopyran, triazine, trithiane, furan, imidazole, isoxazole, isothiazole, oxazole, oxadiazole, oxathiazole, pyrazole, pyrrole, tetrazole, thiophene, thiadiazole, thiatriazole, thiazole or triazole, wherein each substitutable carbon or hetero-atom in R⁴ is optionally and independently substituted by one or more of C₁₋₆ alkyl, OR²⁰, SR²⁰, NO₂, CN, NR²⁰₂, NR²⁰COR²⁰, NR²⁰CONR²⁰₂, NR²⁰COR²⁰, NHCO₂R²⁰, CO₂R²⁰, COR²⁰, CONR²⁰₂, S(O)₂R²⁰, SONR²⁰₂, S(O)R²⁰, SO₂NR²⁰₂, or NR²⁰S(O)₂R²⁰;
wherein R²⁰ is hydrogen, C₁₋₆ alkyl, or C₁₋₆ haloalkyl,

7. A compound as claimed in any one of claims 1 to 6 wherein R⁴ is a six-membered carbocyclyl or heterocyclyl group optionally substituted with one or more of OR²⁹, NR²⁹₂, SR²⁹, (CH₂)ₙOR²⁹, (CH₂)ₙNR²⁹₂, halogen, C₁₋₆ alkyl, C₂₋₆ alkenyl, C₂₋₆ alkynyl, haloalkyl, NO₂, CN, NR²⁹C(O)R²⁹, NR²⁹S(O)₂R²⁹, CO₂R²⁹, COR²⁹, CONR²⁹₂, S(O)₂R²⁹, S(O)R²⁹ or SO₂NR²⁹₂;
wherein R²⁹ is hydrogen, C₁₋₄ alkyl, C₅₋₁₂ heterocyclyl or C₆₋₁₂ aryl preferably phenyl, and n is 1, 2, 3, 4, 5 or 6;
wherein two R²⁹ in NR²⁹₂ may optionally form a partially saturated, unsaturated or fully saturated five to seven membered ring containing one to three heteroatoms, optionally and independently substituted with one or more of halogen, C₁₋₁₂ alkyl, C₂₋₁₂ alkenyl, C₂₋₁₂ alkynyl, C₁₋₁₂ haloalkyl, C₆₋₁₂ carbocyclyl, C₅₋₁₂ heterocyclyl, OR³⁰, SR³⁰, NO₂, CN, NR³⁰₂, NR³⁰COR³⁰, NR³⁰CONR³⁰₂, NR³⁰COR³⁰, NR³⁰CO₂R³⁰, CO₂R³⁰, COR³⁰, CONR³⁰₂, S(O)₂R³⁰, SONR³⁰₂, S(O)R³⁰, SO₂NR³⁰₂, or NR³⁰S(O)₂R³⁰; and each saturated carbon in the optional ring is further optionally and independently substituted by =O, =S, NNR³¹₂, =N-OR³¹, =NNR³¹COR³¹, =NNR³¹CO₂R³¹, =NNSO₂R³¹, or =NR³¹; and each substitutable nitrogen atom is optionally substituted by R³², COR³² SO₂R³² or CO₂R³²;
wherein R³⁰ is hydrogen , C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR³³, SR³³, NO₂, CN, NR³³R³³, NR³³COR³³, NR³³CONR³³R³³, NR³³COR³³, NR³³CO₂R³³, CO₂R³³, COR³³, CONR³³₂, S(O)₂R³³, SONR³³₂, S(O)R³³, SO₂NR³³R³³, NR³³S(O)₂R³³, wherein the C₁₋₁₂ alkyl group optionally incorporates one or two insertions selected from the group consisting of -0-, - N(R³³)-, -S(O)- and -S(O₂)-, wherein each R³³ may be the same or different and is as defined below;
wherein R³¹ is hydrogen, C₁₋₁₂ alkyl, C₆₋₁₂ carbocyclyl or C₅₋₁₂ heterocyclyl, optionally substituted by one or more of C₁₋₆ alkyl, halogen, C₁-₆ haloalkyl, OR³³, SR³³, NO₂, CN, NR³³R³³, NR³³COR³³, NR³³CONR³³R³³, NR³³COR³³, NR³³CO₂R³³, CO₂R³³, COR³³, CONR³³2, S(O)₂R³³, S(O)R³³, SO₂NR³³R³³, NR³³S(O)₂R³³, wherein the C₁₋₁₂ alkyl group optionally incorporates one or two insertions selected from the group consisting of -O-, -N(R³³)-, -S(O)- and - S(O₂)-, wherein each R²¹ may be the same or different and is as defined below;
wherein R³² is hydrogen, C₆₋₁₂ aryl, C₁₋₆ alkyl or C₁₋₆ haloalkyl;
wherein R³³ is hydrogen, C₁₋₆ alkyl, or C₁₋₆ haloalkyl.

8. A compound as claimed in any one of claims 1 to 7 wherein R⁴ is a five-membered heterocyclyl, 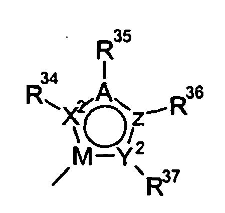
wherein A, X², Y² or Z are independently selected from N, O, C, S and M is C or N, wherein one, two, three or four of A, X², Y², Z and M is other than C; R³⁴, R³⁵, R³⁶ or R³⁷ are independently selected from a lone electron pair, hydrogen, halogen, C₁₋₁₂ alkyl, C₁₋₁₂ haloalkyl, OR³⁸, SR³⁸, NO₂, CN, NR³⁸₂, NR³⁸COR³⁸, NR³⁸CONR³⁸₂, NR³⁸COR³⁸, NR³⁸CO₂R³⁸, (CH)ₙOR³⁸, (CH₂)ₙNR³⁸, CO₂R³⁸, COR³⁸, CONR³⁸₂, S(O)₂R³⁸, SONR³⁸₂, S(O)R³⁸, SO₂NR³⁸₂, or NHS(O)₂R³⁸;
wherein n is 1 to 6, preferably n is 1, 2 or 3;
or wherein any two of R³⁴, R³⁸, R³⁶ or R³⁷ may optionally form a partially saturated, unsaturated or fully saturated five to seven membered ring containing zero to three heteroatoms, each saturated carbon in the optional fused ring is further optionally and independently substituted with one or more of halogen, C₁₋₁₂ alkyl, C₂₋₁₂ alkenyl, C₂₋₁₂ alkynyl, C₁₋₁₂ haloalkyl, C₆₋₁₂ carbocyclyl, C₅₋₁₂ heterocyclyl, OR³⁸, SR³⁸, NO₂, CN, NR³⁸₂, NR³⁸CONR³⁸₂, NR³⁸COR³⁸, NR³⁸CO₂R³⁸, (CH₂)ₙOR³⁸, (CH₂)ₙNR³⁸₂, CO₂R³⁸, COR³⁸, CONR³⁸₂, S(O)₂R³⁸, SONR³⁸₂, S(O)R³⁸, SO₂NR³⁸₂, or NR³⁸S(O)₂R³⁸; and each saturated carbon in the optional fused ring is further optionally and independently substituted by =O, =S, NNR³⁹₂, =N-OR³⁹, =NNR³⁹COR³⁹, =NNR³⁹CO₂R³⁹, =NNSO₂R³⁹, or =NR³⁹; and each substitutable nitrogen atom in R⁴ is optionally substituted by R⁴⁰, COR⁴⁰, SO₂R⁴⁰ or CO₂R⁴⁰;
wherein n is 1 to 6, preferably n is 1, 2 or 3;
wherein R³⁸ is hydrogen, C₁₋₁₂ alkyl, C₆₋₁₂ carbocyclyl or C₅₋₁₂ heterocyclyl, optionally substituted by one or more of C₁₋₆ alkyl, halogen, C₁₋₆ haloalkyl, OR⁴¹, SR⁴¹, NO₂, CN, NR⁴¹R⁴¹, NR⁴¹CONR⁴¹R⁴¹, NR⁴¹COR⁴¹, NR⁴¹CO₂R⁴¹, CO₂R⁴¹, COR⁴¹, CONR⁴¹₂, S(O)₂R⁴¹, SONR⁴¹₂, S(O)R⁴¹, SO₂NR⁴¹R⁴¹, NR⁴¹S(O)₂R⁴¹, wherein the C₁₋₂ alkyl group optionally incorporates one or two insertions selected from the group consisting of -O-, -N(R⁴¹)-, -S(O)- and - S(O₂)-, wherein each R⁴¹ may be the same or different and is as defined below;
wherein R³⁹ is hydrogen, C₁₋₁₂ alkyl, carbocyclyl or heterocyclyl, optionally substituted by one or more of C₁₋₆ alkyl, halogen, C₁₋₆ haloalkyl, OR⁴¹, SR⁴¹, NO₂, CN, NR⁴¹R⁴¹, NR⁴¹COR⁴¹, NR⁴¹CONR⁴¹R⁴¹, NR⁴¹COR⁴¹, NR⁴¹CO₂R⁴¹, CO₂R⁴¹, COR⁴¹, CONR⁴¹₂, S(O)R⁴¹, S(O)R⁴¹, SO₂NR⁴¹R⁴¹, NR⁴¹S(O)R⁴¹, wherein the C₁₋₁₂ alkyl group optionally incorporates one or two insertions selected from the group consisting of -O-, -N(R⁴¹)-, -S(O)- and -S(O₂)-, wherein each R⁴¹ may be the same or different and is as defined below;
wherein R⁴⁰ is hydrogen, C₁₋₆ alkyl, C₁₋₆ haloalkyl or C₆₋₁₂ aryl.
wherein R⁴¹ is hydrogen, C₁₋₆ alkyl, or C₁₋₆ haloalkyl.

9. A compound as claimed in claim 8 wherein R⁴ is furan, imidazole, isoxazole, isothiazole, oxazole, oxadiazole, oxatriazole, pyrazole, pyrrole, tetrazole, thiophene, thiadiazole, thiatriazole, thiazole or triazole; and R³⁴, R³⁵, R³⁶ or R³⁷ are independently selected from a lone electron pair, hydrogen, halogen, C₁₋₆ alkyl, C₁₋₆ haloalkyl, OR⁴², SR⁴², CN, NR⁴²₂, NR⁴²COR⁴², CO₂R₄₂, COR⁴², CONR⁴²₂, S(O)₂R⁴², or S(O)R⁴²;
wherein R⁴² is hydrogen, C₁₋₄ alkyl, preferably methyl or ethyl or carbocyclyl, preferably phenyl.

10. A compound as claimed in any one of claims 1 to 9 selected from

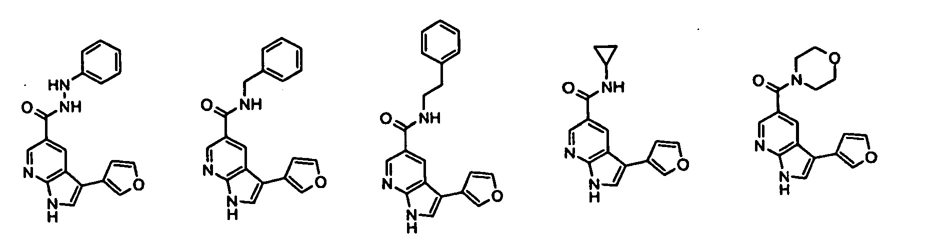

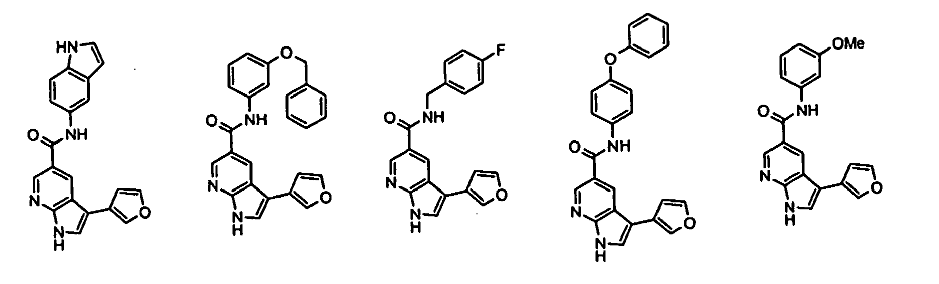

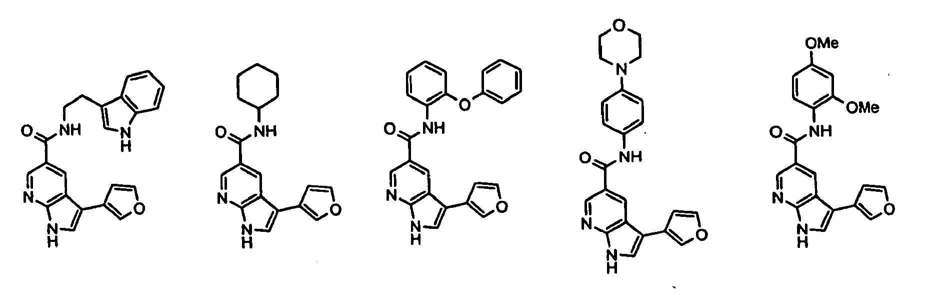

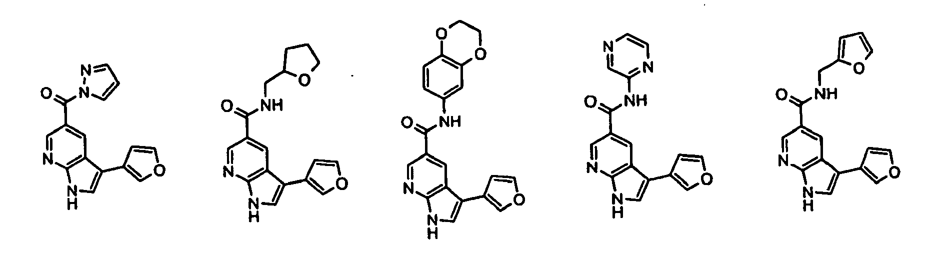

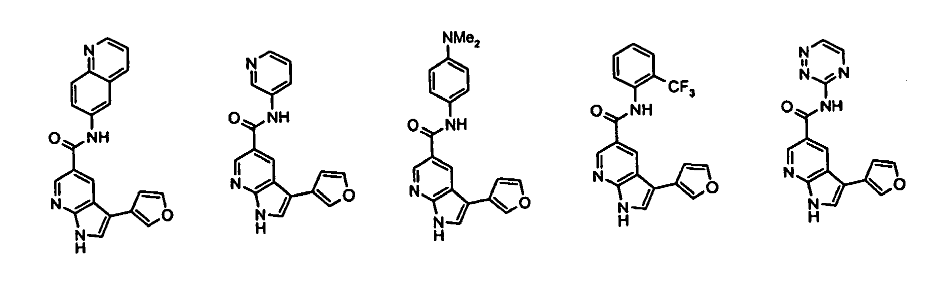

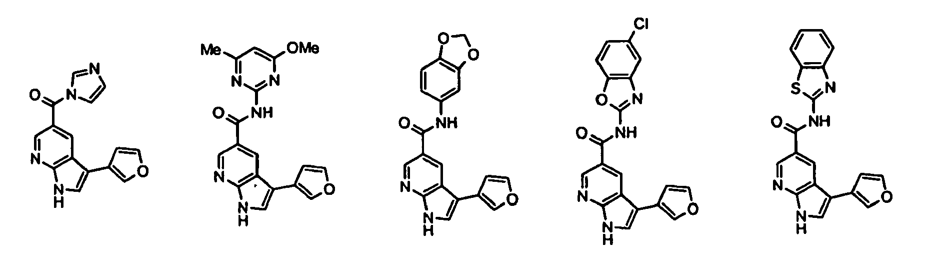

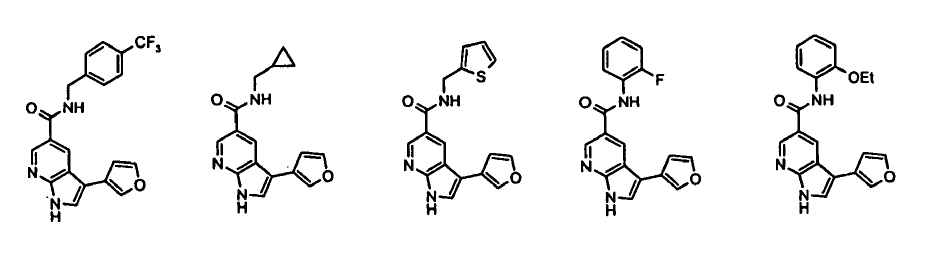

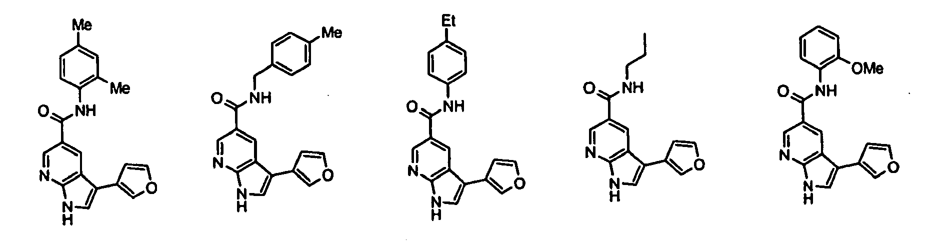

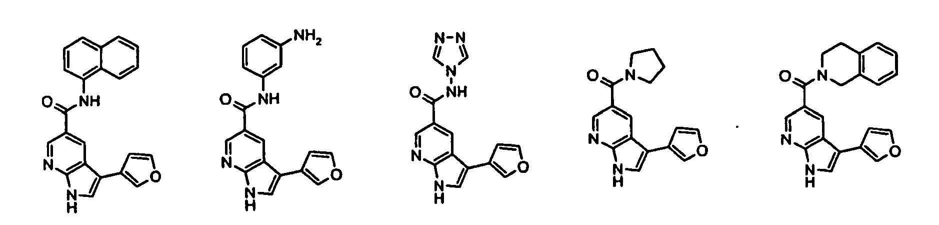

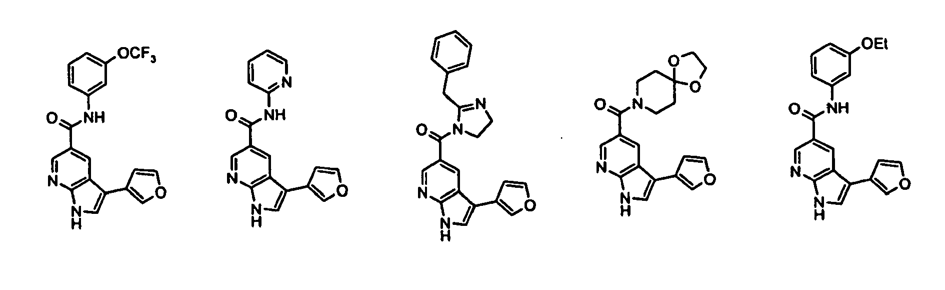

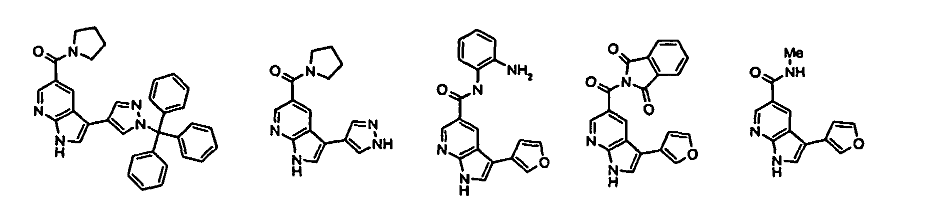

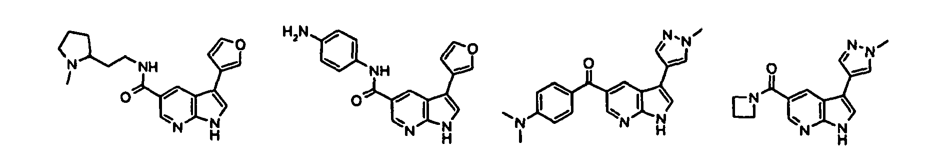

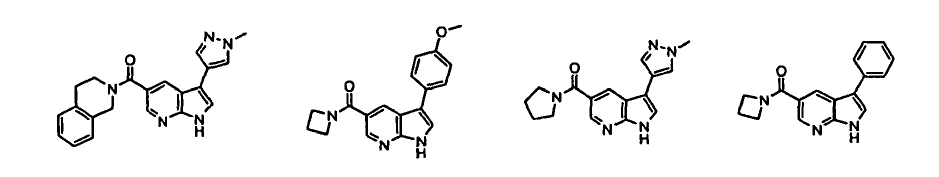

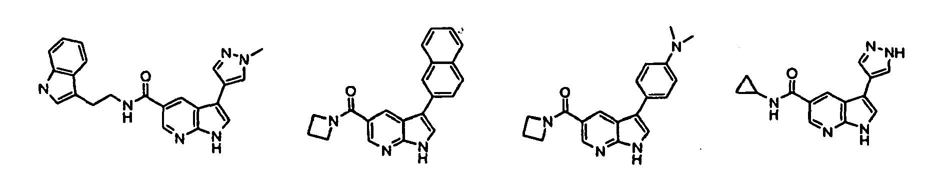

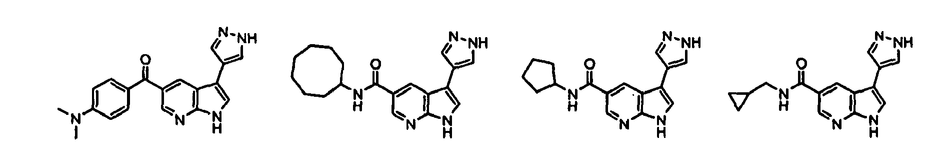

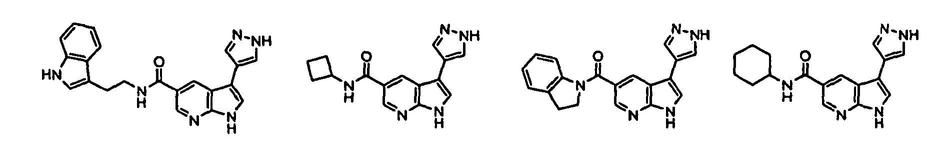

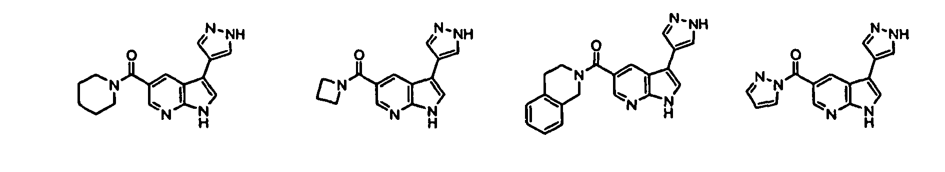

11. A process for the manufacture of a compound of formula (I) wherein R¹ is a group of formula (II) as defined in the any one of claims 1 to 10 of the invention comprising the condensation of an intermediate (III) with an intermediate (IV). 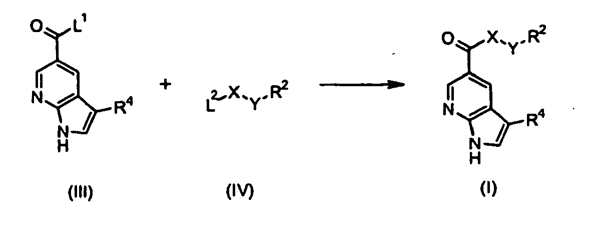 wherein R² and R⁴ are as defined in any one of claims 1 to 10; and wherein each of L¹ and L² is independently a leaving group wherein L¹ and L² together form a condensation product.

12. A process as claimed in claim 11 wherein L¹ is OH, OR⁵⁰ OM, Cl, Br or I wherein R⁵⁰ is C₁₋₆ alkyl, preferably methyl or ethyl and M is Na, Li, K, Ca, Mg or Ba, and L² is hydrogen or M.

13. A pharmaceutical composition comprising a compound as claimed in any one of claims 1 to 10 in combination with a pharmaceutically acceptable carrier, diluent or excipient.

14. A composition as claimed in claim 13, additionally comprising one or more of an anti-inflammatory agent, an AMPA receptor antagonist, a chemotherapeutic agent and/or an antiproliferative agent.

15. A process for the manufacture of a composition according to claim 13 or claim 14, comprising combining a compound according to any one of claims 1 to 10 of the invention with a pharmaceutically acceptable carrier or diluent and optionally with any one or more of additional agents of claim 14.

16. A compound as claimed in any one of claims 1 to 10, or a composition as claimed in claim 13 or claim 14, for use in medicine.

17. A compound as defined in any of claims 1-10, or a composition as defined in any of claims 13 or 14, for inhibiting JNK.

18. A compound as defined in any of claims 1-10, or a composition as defined in any of claims 13 or 14, for selectively inhibiting JNK3.

19. A compound as defined in any of claims 1-10, or a composition as defined in any of claims 13 or 14, for use in the prevention or treatment of a JNK-mediated disorder.

20. A compound or a composition as claimed in claim 19, wherein the disorder is a neurodegenerative disorder (including dementia), inflammatory disease, a disorder linked to apoptosis, particularly neuronal apoptosis, autoimmune disease, destructive bone disorder, proliferative disorder, cancer, infectious disease, allergy, ischemia reperfusion injury, heart attack, angiogenic disorder, organ hypoxia, vascular hyperplasia, cardiac hypertrophy, thrombin induced platelet aggregation and/or any condition associated with prostaglandin endoperoxidase synthase-2.

21. A compound or composition as claimed in claim 20, wherein the neurodegenerative disorder results from apoptosis and/or inflammation.

22. A compound or composition as claimed in claim 20 or 21, wherein the neurodegenerative disorder is: dementia; Alzheimer's disease; Parkinson's disease; Amyotrophic Lateral Sclerosis; Huntington's disease; senile chorea; Sydenham's chorea; hypoglycemia; head and spinal cord trauma including traumatic head injury; acute and chronic pain; epilepsy and seizures; olivopontocerebellar dementia; neuronal cell death; hypoxia-related neurodegeneration; acute hypoxia; glutamate toxicity including glutamate neurotoxicity; cerebral ischemia; dementia linked to meningitis and/or neurosis; cerebrovascular dementia; or dementia in an HIV-infected patient.

23. A compound or composition as claimed in claim 20 or 21, wherein the neurodegenerative disorder is a peripheral neuropathy, including mononeuropathy, multiple mononeuropathy or polyneuropathy, such as may be found in diabetes mellitus, Lyme disease or uremia; peripheral neuropathy caused by a toxic agent; demyelinating disease such as acute or chronic inflammatory polyneuropathy, leukodystrophies or Guillain-Barré syndrome; multiple mononeuropathy secondary to a collagen vascular disorder; multiple mononeuropathy secondary to sarcoidosis; multiple mononeuropathy secondary to a metabolic disease; or multiple mononeuropathy secondary to an infectious disease.

24. A compound or composition as claimed in claim 20, wherein the disorder is inflammatory bowel disorder; bronchitis; asthma; acute pancreatitis; chronic pancreatitis; allergies of various types; Alzheimer's disease; autoimmune disease such as rheumatoid arthritis, systemic lupus erythematosus, glomerulonephritis, scleroderma, chronic thyroiditis, Graves's disease, autoimmune gastritis, diabetes, autoimmune haemolytis anaemia, autoimmune neutropaenia, thrombocytopenia, atopic dermatitis, chronic active hepatitis, myasthenia gravis, multiple sclerosis, ulcerative colitis, Crohn's disease, psoriasis or graft vs host disease.

25. Use of a compound as defined in claim 1-10 in the manufacture of a medicament for the prevention or treatment of a JNK-mediated disorder.

## Patentansprüche

1. Verbindung der Formel (I): 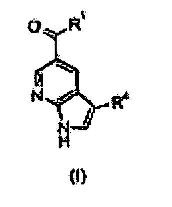 und der pharmazeutisch akzeptablen Salze;
wobei R¹ eine C₃₋₁₂-Carbocyclyl- oder eine C₃₋₁₂-Heterocyclyl-Gruppe ist, die gegebenenfalls mit einem partiell gesättigtem, ungesättigtem oder vollständig gesättigtem fünf- bis siebengliedrigem Ring fusioniert ist, der null bis drei Heteroatome enthält, und jedes substituierbare Kohlenstoffatom in R¹, einschließlich des gegebenen fusionierten Rings, gegebenenfalls und unabhängig durch ein oder mehrere von Halogen, C₁₋₁₂-Alkyl, C₂₋₁₂-Alkenyl, C₂₋₁₂-Alkynyl, Halo-C₁₋₁₂-Alkyl, C₃₋₁₂-Carbocyclyl, C₃₋₁₂-Heterocyclyl, (CH₂)ₙOR⁵, (CH₂)ₙNR⁵₂(CH₂)ₙSR⁵, OR⁵, SR⁵, NO₂, CN, NR⁵₂, NR⁵COR⁵, NR⁵CONR⁵₂, NR⁵COR⁵, NR⁵CO₂R⁵, CO₂R⁵, COR⁵, CONR⁵₂, S(O)₂R⁵, SONR⁵₂, S(O)R⁵, SO₂NR⁵₂ oder NR⁵S(O)₂R⁵ substituiert ist, wobei die C₁₋₁₇-Alkyl-Gruppe gegebenenfalls ein oder mehrere Insertionen ausgewählt aus -O-, -N(R⁵)--S-, -S(O) - und -S(O₂)- enthält; und jeder gesättigte Kohlenstoff im gegebenen fusionierten Ring des Weiteren gegebenenfalls und unabhängig durch =O, =S, NNR⁶₂, =N-OR⁶, =NNR⁶COR⁶, =NNR⁶CO₂R⁶, =NNSO₂R⁶ oder =NR⁶ substituiert ist; und jedes substituierbare Stickstoffatom in R¹ gegebenenfalls durch R⁷ COR⁷, SO₂R⁷ oder CO₂R⁷ substituiert ist;
wobei n 1 bis 6 ist, vorzugsweise ist n 1, 2 oder 3;
wobei R⁵ Wasserstoff, C₁₋₁₂-Alkyl, C₃₋₁₂-Carbocyclyl oder C₃₋₁₂-Heterocyclyl ist, gegebenenfalls substituiert durch ein oder mehrere von C₁₋₆-Alkyl, C₃₋₁₂-Carbocyclyl, C₃₋₁₂-Heterocyclyl, Halogen, C₁₋₆-Haloalkyl, OR⁸, SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S(O)₂R⁸, SONR⁸₂, S(O)R⁸, SO₂NR⁸R⁸, NR⁸S(O)₂R⁸, wobei die C₁₋₁₂-Alkyl-Gruppe gegebenenfalls ein oder zwei Insertionen ausgewählt aus der Gruppe bestehend aus -O-, -N(R⁸)-, -S(O)- und -S(O₂)- inkorporiert, wobei jedes R⁸ gleich oder verschieden sein kann und wie unten definiert ist;
wobei zwei R⁵ in NR⁵₂ gegebenenfalls einen partiell gesättigten, ungesättigten oder vollständig gesättigten drei- bis sieben-gliedrigen Ring bilden können, der ein bis drei Heteroatome enthält, gegebenenfalls und unabhängig substituiert durch ein oder mehrere von C₁₋₆-Alkyl, Halogen, C₁₋₆-Haloalkyl, OR⁸, SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S(O)₂R⁸, SONR⁸₂, S(O)R⁸, SO₂NR⁸R⁸, NR⁸S(O)₂R⁸;
wobei die C₁₋₆-Alkyl-Gruppe gegebenenfalls ein oder zwei Insertionen ausgewählt aus der Gruppe bestehend aus -O-, -N(R⁸)-, - S(O) - und -S(O₂)- inkorporiert, wobei jedes R⁸ gleich oder verschieden sein kann und wie unten definiert ist;
wobei R⁶ Wasserstoff, C₁₋₁₂-Alkyl, C₁₋₁₂-Carbocyclyl oder C₁₋₁₂-Heterocyclyl ist, gegebenenfalls substituiert durch ein oder mehrere von C₁₋₆-Alkyl, Halogen, C₁₋₆-Haloalkyl, OR⁸, SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S(O)₂R⁸, S(O)R⁸, SO₂NR⁸R⁸, NR⁸S (O)₂R⁸, wobei die C₁₋₁₂-Alkyl-Gruppe gegebenenfalls ein oder zwei Insertionen ausgewählt aus der Gruppe bestehend aus -O-, -N(R⁸)-, -S(O)- und -S(O₂)- inkorporiert, wobei jedes R⁸ gleich oder verschieden sein kann und wie unten definiert ist;
wobei R⁷ Wasserstoff, C₆₋₁₂-Aryl, C₁₋₆-Alkyl oder C₁₋₆-Haloalkyl ist;
wobei R⁸ Wasserstoff, C₁₋₆-Alkyl oder C₁₋₆-Haloalkyl ist, oder wobei R¹ eine Gruppe der Formel (II) ist 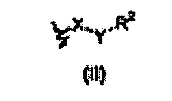
wobei X NR³, 0, S oder (CR²²R²²)ₙ ist, Y nicht vorhanden oder NR²³, O oder (CR²³R²³) ist, wobei jedes R²³ H, C₁₋₄-Alkyl, C₁₋₄-Alkoxy oder C₁₋₄-Haloalkyl ist;
und n 1 bis 6 ist, vorzugsweise ist n 1, 2, 3 oder 4; und
R² gegebenenfalls substituiertes C₁₋₁₂-Alkyl, C₂₋₁₂-Alkenyl, C₂₋₁₂-alkenyl, C₃₋₁₂-Carbocyclyl oder C₃₋₁₂-Heterocyclyl ist, wobei die gegebenenfalls substituierte Carbocyclyl- oder Heterocyclyl-Gruppe gegebenenfalls mit einem bis drei ungesättigten, partiell ungesättigten oder vollständig gesättigten fünf- bis sieben-gliedrigen Ringen fusioniert ist, die null bis drei Heteroatome enthalten;
jedes substituierbare Kohlenstoffatom in R², einschließlich des gegebenen kondensierten Rings, gegebenenfalls und unabhängig durch ein oder mehrere von C₁₋₁₂-Alkyl, C₃₋₁₂-Cycloalkyl, C₃₋₁₂-Hetrocycloalkyl, C₃₋₁₂-Aryl, C₃₋₁₂-Heteroaryl-Halogen, C₁₋₁₂-Haloalkyl, OR⁹, SR⁹, NO₂, CN, NR⁹R⁹, NR⁹COR⁹, NR⁹CONR⁹R⁹, NR⁹COR¹, NR⁹CO₂R⁹, CO₂R⁹, COR⁹, CONR⁹R⁹, S(O)²R⁹, SONH₂, S(O)R⁹, SO₂NR⁹R⁹, NR⁹S(O)²R⁹ substituiert ist, wobei jedes R⁹ gleich oder verschieden sein kann und wie unten definiert ist und wobei:
die C₁₋₁₂-Alkyl-Gruppe gegebenenfalls ein oder zwei Insertionen ausgewählt aus der Gruppe bestehend aus -O-, -C(O)-, N(R⁹)-, -S(O)- und -S(O₂)- inkorporiert, wobei jedes R⁹ gleich oder verschieden sein kann und wie oben definiert ist;
die C₁₋₁₂-Alkyl-, C₃₋₁₂-Cycloalkyl, C₃₋₁₂-Hetrocycloalkyl, C₃₋₁₂-Aryl oder C₃₋₁₂-Heteroaryl-Gruppen gegebenenfalls durch ein oder mehrere von Halogen, C₁₋₁₂-Haloalkyl, OR⁹, SR⁹, NO₂, CN, NR⁹R⁹, NR⁹-COR⁹, NR⁹CONR⁹R⁹, NR⁹COR⁹, NR⁹CO₂R⁹, CO₂R⁹, COR⁹, CONR⁹R⁹, S(O)₂R⁹, SONH₂, S(O)R⁹, SO₂NR⁹R⁹, NR⁹S(O)₂R⁹ substituiert sind, wobei jedes R⁹ gleich oder verschieden sein kann und wie unten definiert ist; und
die C₃₋₁₂-Cycloalkyl-, C₃₋₁₂-Hetrocycloalkyl-, C₃₋₁₂-Aryl- oder C₃₋₁₂-Heteroaryl-Gruppen gegebenenfalls durch ein oder mehrere C₁₋₁₂-Alkyl-Gruppen substituiert sind;
jeder gesättigte Kohlenstoff in R², einschließlich des gegebenen fusionierten Rings, des Weiteren gegebenenfalls und unabhängig durch =O, =S, NNR⁹R⁹, =N-OR⁹, =NNHCOR⁹, =NNHCO₂R⁹, =NNSO₂R⁹ oder =NR⁹ substituiert ist, wobei jedes R⁹ gleich oder verschieden sein kann und wie unten definiert ist; und
jedes substituierbare Stickstoffatom in R² gegebenenfalls durch R¹⁰, COR⁹, SO₂R⁹ oder CO₂R⁹ substituiert ist, wobei jedes R⁹ und R¹⁰ gleich oder verschieden sein kann und wie unten definiert ist;
wobei zwei R⁹ in NR⁹₂ gegebenenfalls einen partiell gesättigten, ungesättigten oder vollständig gesättigten drei- bis sieben-gliedrigen Ring bilden können, der ein bis drei Heteroatome enthält, gegebenenfalls und unabhängig substituiert durch ein oder mehrere von C₁₋₆-Alkyl, Halogen, C₁₋₆-Haloalkyl, OR¹¹, SR¹¹, NO₂, CN, NR¹¹R¹¹, NR¹¹COR¹¹, NR¹¹CONR¹¹R¹¹, NR¹¹COR¹¹, NR¹¹CO₂R¹¹, CO₂R¹¹, COR¹¹, CONR¹¹₂, S(O)₂R¹¹, SONR¹¹₂, S(O)R¹¹, SO₂NR¹¹R¹¹, NR¹¹S(O)₂R¹¹, wobei die C₁₋₆-Alkyl-Gruppe gegebenenfalls ein oder zwei Insertionen ausgewählt aus der Gruppe bestehend aus -O-, -N(R¹¹)-, -S(O)- und -S(O₂)- inkorporiert, wobei jedes R¹¹ gleich oder verschieden sein kann und wie unten definiert ist;
wobei R¹¹ Wasserstoff, C₁₋₆-Alkyl oder C₁₋₆-Haloalkyl ist;
wobei R⁹ Wasserstoff, C₁₋₁₂-Alkyl oder C₃₋₁₂-Aryl ist, gegebenenfalls substituiert durch ein oder mehrere von C₁₋₄-Alkyl, Halogen, C₁₋₄-Haloalkyl, OR¹², SR¹², NO₂, CN, NR¹²R¹², NR¹²COR¹², NR¹²CONR¹²R¹², NR¹²COR¹², NR¹²CO₂R¹², CO₂R¹², COR¹², CONR¹²₂, S(O)₂R¹², SONH₂, S (0) R¹², SO₂NR¹²R¹² , NR¹²S (O)₂R¹², wobei die C₁₋₁₂-Alkyl-Gruppe gegebenenfalls ein oder zwei Insertionen ausgewählt aus der Gruppe bestehend aus -O-, N(R¹²)-, -S(O)- und -S(O₂)- inkorporiert, wobei jedes R¹² gleich oder verschieden sein kann und wie unten definiert ist;
wobei R¹⁰ C₁₋₁₂-Alkyl oder C₃₋₁₂-Aryl ist, gegebenenfalls substituiert durch ein oder mehrere von C₁₋₄-Alkyl, Halogen, C₁₋₄-Haloalkyl, OR¹², SR¹², NO₂, CN, NR¹²R¹², NR¹²COR¹², NR¹²CONR¹²R¹², NR¹²COR¹², NR¹²CO₂R¹², CO₂R¹², COR¹², CONR¹²₂, S(O)₂R¹², SONH₂, S(O)R¹², SO₂NR¹²R¹², NR¹²S(O)₂R¹², wobei die C₁₋₁₂-Alkyl-Gruppe gegebenenfalls ein oder zwei Insertionen ausgewählt aus der Gruppe bestehend aus -O-, -N(R¹²)-, -S(O)- und S(O₂)- inkorporiert, wobei jedes R¹² gleich oder verschieden sein kann und wie unten definiert ist;
wobei R¹² Wasserstoff, C₁₋₄-Alkyl oder C₁₋₄-Haloalkyl ist;
wobei X NR³, 0, S oder (CR²²-R²²) ist, wobei R²² unabhängig ein oder mehrere von Halogen, C₁₋₁₂-Alkyl, C₂₋₁₂-Alkenyl, C₂₋₁₂-Alkynyl, C₁₋₁₂-Haloalkyl, C₆₋₁₂-Carbocyclyl, C₅₋₁₂-Heterocyclyl, (CH₂)ₙOR⁵, (CH₂)ₙNR⁵₂, OR⁵, SR⁵, NO₂, CN, NR⁵₂, NR⁵COR⁵, NR⁵CONR⁵₂, NR⁵COR⁵, NR⁵-CO₂R⁵, CO₂R⁵, COR⁵, CONR⁵₂, S(O)₂R⁵, SONR⁵₂, S(O)R⁵, SO₂NR⁵₂ oder NR⁵S(O)₂R⁵ ist, wobei jedes R⁵ gleich oder verschieden sein kann und wie oben definiert ist; und
wobei n 1 bis 6 ist, vorzugsweise ist n 1, 2, 3 oder 4;
und wobei R³ ein einzelnes Elektronenpaar, Wasserstoff, C₁₋₁₂-Alkyl, C₂₋₁₂-Alkenyl, C₂₋₁₂-Alkynyl, C₃₋₁₂-Carbocyclyl oder C₃₋₁₂-Heterocyclyl ist, wobei jedes davon gegebenenfalls substituiert wird, wobei:
die gegebenenfalls substituierte Carbocyclyl- oder Heterocyclyl-Gruppe gegebenenfalls mit einem bis drei ungesättigten, partiell ungesättigten oder vollständig gesättigten fünf- bis sieben-gliedrigen Ringen fusioniert ist, die null bis drei Heteroatome enthalten;
jedes substituierbare Kohlenstoffatom in R³, einschließlich des gegebenen fusionierten Rings, gegebenenfalls und unabhängig durch ein oder mehrere von C₁₋₁₂-Alkyl, C₃₋₁₂-Cycloalkyl, C₃₋₁₂-Heterocycloalkyl, C₃₋₁₂-Aryl, C₃₋₁₂-Heteroaryl, Halogen, C₁₋₁₂-Haloalkyl, OR¹³, SR¹³ NO₂, CN, NR¹³R¹³, NR¹³COR¹³, NR¹³CONR¹³R¹³, NR¹³COR¹³, NR¹³CO₂R¹³, CO₂R¹³, COR¹³, CONR¹³R¹³, S(O)₂R¹³, SONH₂, S(O)R¹³, SO₂NR¹³R¹³, NR¹³S(O)₂R¹³ substituiert ist, wobei jedes R¹³ gleich oder verschieden sein kann und wie oben definiert ist und wobei:
die C₁₋₁₂-Alkyl-Gruppe gegebenenfalls ein oder zwei Insertionen ausgewählt aus der Gruppe bestehend aus -O-, -C(O)-, -N(R¹³)-, -S(O)- und -S(O₂)- inkorporiert, wobei jedes R¹³ gleich oder verschieden sein kann und wie oben definiert ist;
die C₁₋₁₂-Alkyl-, C₃₋₁₂-Cycloalkyl-, C₃₋₁₂-Heterocycloalkyl-, C₃₋₁₂-Aryl- oder C₃₋₁₂-Heteroaryl-Gruppen gegebenenfalls durch ein oder mehrere von Halogen, C₁₋₁₂-Haloalkyl, OR¹³, SR¹³ NO₂, CN, NR¹³R¹³, NR¹³COR¹³, NR¹³CONR¹³R¹³, NR¹³COR¹³, NR¹³CO₂R¹³, CO₂R¹³, COR¹³, CONR¹³R¹³, S(O)₂R¹³, SONH₂, S(O)R¹³, SO₂NR¹³R¹³, NR¹³S(O)₂R¹³ substituiert sind, wobei jeder R¹³ gleich oder verschieden sein kann und wie unten definiert ist; und
die C₃₋₁₂-Cycloalkyl-, C₃₋₁₂-Heterocycloalkyl-, C₃₋₁₂-Aryl- oder C₃₋₁₂-Heteroaryl-Gruppen gegebenenfalls durch ein oder mehrere C₁₋₁₂-Alkyl-Gruppen substituiert sind;
jeder gesättigte Kohlenstoff in R², einschließlich des gegebenen fusionierten Rings, des Weiteren gegebenenfalls und unabhängig durch =O, =S, NNR¹³R¹³, =N-OR¹³, =NNHCOR¹³, =NNHCO₂R¹³, =NNSO₂R¹³ oder =NR¹³ substituiert ist, wobei jedes R¹³ gleich oder verschieden sein kann und wie unten definiert ist; und
jedes substituierbare Stickstoffatom in R³ gegebenenfalls durch R¹⁴, COR¹³, SO₂R¹³ oder CO₂R¹³ substituiert ist, wobei jedes R¹³ und R¹⁴ gleich oder verschieden sein kann und wie unten definiert ist;
wobei zwei R¹³ in NR¹³₂ gegebenenfalls einen partiell gesättigten, ungesättigten, oder vollständig gesättigten drei- bis sieben-gliedrigen Ring bilden können, der ein bis drei Heteroatome enthält, gegebenenfalls und unabhängig substituiert durch ein oder mehrere von C₁₋₆-Alkyl, Halogen, C₁₋₆-Haloalkyl, OR¹⁵, SR¹⁵, NO₂, CN, NR¹⁵R¹⁵, NR¹⁵SCOR¹⁵, NR¹⁵CONR¹⁵R¹⁵, NR¹⁵COR¹⁵, NR¹⁵CO₂R¹⁵, CO₂R¹⁵, COR¹⁵, CONR¹⁵₂, S(O)₂R¹⁵, SONR¹⁵₂, S(O)R¹⁵, SO₂NR¹⁵R¹⁵, NR¹⁵S(O)₂R¹⁵, WObei die C₁₋₆-Alkyl-Gruppe gegebenenfalls ein oder zwei Insertionen ausgewählt aus der Gruppe bestehend aus -O-, -N(R¹⁵)-, -S(O)- und -S(O₂)- inkorporiert, wobei jedes R¹⁵ gleich oder verschieden sein kann und wie unten definiert ist;
wobei R¹⁵ Wasserstoff, C₁₋₆-Alkyl oder C₁₋₆-Haloalkyl ist;
wobei R¹³ Wasserstoff, C₁₋₁₂-Alkyl oder C₃₋₁₂-Aryl ist, gegebenenfalls substituiert durch ein oder mehrere von C₁₋₄-Alkyl, Halogen, C₁₋₄-Haloalkyl, OR¹⁶, SR¹⁶, NO₂, CN, NR¹⁶R¹⁶, NR¹⁶COR¹⁶, NR¹⁶CONR¹⁶R¹⁶, NR¹⁶COR¹⁶, NR¹⁶CO₂R¹⁶, CO₂R¹⁶, COR¹⁶, CONR¹⁶₂, S(O)₂R¹⁶, SONH₂, S(O)R¹⁶, SO₂NR¹⁶R¹⁶, NR¹⁶S(O)₂R¹⁶, wobei die C₁₋₁₂-Alkyl-Gruppe gegebenenfalls ein oder zwei Insertionen ausgewählt aus der Gruppe bestehend aus -O-, -N(R¹⁶) -, -S(O)- und -S(O₂) - inkorporiert, wobei jedes R¹⁶ gleich oder verschieden sein kann und wie unten definiert ist;
wobei R¹⁴ C₁₋₁₂-Alkyl oder C₃₋₁₂-Aryl ist, gegebenenfalls substituiert durch ein oder mehrere von C₁₋₄-Alkyl, Halogen, C₁₋₄-Haloalkyl, OR¹⁶, SR¹⁶, NO₂, CN, NR¹⁶R¹⁶, NR¹⁶COR¹⁶, NR¹⁶CONR¹⁶R¹⁶, NR¹⁶COR¹⁶, NR¹⁶CO₂R¹⁵, CO₂R¹⁶, COR¹⁶, CONR¹⁶₂, S(O)₂R¹⁶, SONH₂, S(O)R¹⁶, SO₂NR¹⁶R¹⁶, NR¹⁶S (O)₂R¹⁶, wobei die C₁₋₁₂-Alkyl-Gruppe gegebenenfalls ein oder zwei Insertionen ausgewählt aus der Gruppe bestehend aus -O-, -N(R¹⁶)-, -S(O)- und -S(O₂)- inkorporiert, wobei jedes R¹⁶ gleich oder verschieden sein kann und wie unten definiert ist;
wobei R¹⁶ Wasserstoff, C₁₋₄-Alkyl oder C₁₋₄-Haloalkyl ist;
wobei wenn X NR² ist, R² und R³ einen 3- bis 12-gliedrigen Heterocyclyl-Ring bilden können, stärker bevorzugt einen 5-, 6-, 7-, 8-, 9-, 10-, 11- oder 12-gliedrigen Ring, wobei besagter Ring partiell gesättigt, ungesättigt oder vollständig gesättigt sein kann und ein bis drei Heteroatome enthält; wobei die durch R² und R³ gebildete heterocyclylische Gruppe gegebenenfalls mit einem bis drei ungesättigten, partiell gesättigten oder vollständig gesättigten 5- bis 7-gliedrigen Ringen, die null bis drei Heteroatome enthalten, fusioniert sein kann, wobei jeder dieser Ringe gegebenenfalls und unabhängig durch ein oder mehrere von C₁₋₆-Alkyl, Halogen, C₁₋₆-Haloalkyl, OR²², SR²², NO₂, CN, NR²²R²², NR²²COR²², NR²²CONR²²R²², NR²²COR²², NR²²CO₂R²²O, CO₂R²², COR²², CONR²²₂, S(O)₂R²², SONR²²₂, S(O)R²², SO₂NR²²R²² oder NR²²S(O)₂R²² substituiert ist, wobei die C₁₋₆-Alkyl-Gruppe gegebenenfalls ein oder zwei Insertionen aus -O-, -N(R²²)-, -S(O)- und -S(O₂)- inkorporiert und wobei jedes R²² gleich oder verschieden sein kann;
wobei R⁴ eine sechs-gliedrige Carbocyclyl-Gruppe oder eine fünf- bis sechs-gliedrige Heterocyclyl-Gruppe ist, die zwischen 1 bis 4 Heteroatome enthält, welche unabhängig aus N, S oder O ausgewählt werden, wobei die gegebenenfalls substituierte sechs-gliedrige Carbocyclyl- oder fünf- bis sechs-gliedrige Heterocyclyl-Gruppe gegebenenfalls mit einem partiell gesättigtem, ungesättigtem oder vollständig gesättigtem fünf- bis siebengliedrigem Ring fusioniert ist, der null bis drei Heteroatome enthält, und jedes substituierbare Kohlenstoff- oder Heteroatom in R⁴, einschließlich des gegebenen fusionierten Rings, gegebenenfalls und unabhängig durch ein oder mehrere von Halogen, C₁₋₁₂-Alkyl, C₂₋₁₂-Alkenyl, C₂₋₁₂-Alkynyl, C₁₋₁₂-Haloalkyl, C₃₋₁₂-Carbocyclyl, C₃₋₁₂-Heterocyclyl, (CH₂)ₙOR¹⁷, (CH₂)ₙNR¹⁷₂, OR¹⁷, SR¹⁷, NO₂, CN, NR¹⁷₂, NR¹⁷COR¹⁷, NR¹⁷CONR¹⁷₂, NR¹⁷COR¹⁷, NR¹⁷CO₂R¹⁷, CO₂R¹⁷, COR¹⁷, CONR¹⁷₂, S(O)₂R¹⁷, SONR¹⁷₂, S(O)R¹⁷, SO₂NR¹⁷₂ oder NR¹⁷S(O)₂R¹⁷ substituiert ist, wobei die C₁₋₁₂-Alkyl-Gruppe gegebenenfalls ein oder mehrere Insertionen ausgewählt aus -O-, -N(R¹²)--S, -S(O)- und -S(O₂)- enthält; und jeder gesättigte Kohlenstoff im gegebenen fusionierten Ring des Weiteren gegebenenfalls und unabhängig durch =O, =S, NNR¹⁸₂, =N-OR¹⁸, =NNR¹⁸COR¹⁸, =NNR¹⁸CO₂R¹⁸, =NNSO₂R¹⁸ oder =NR¹⁸ substituiert ist; und jedes substituierbare Stickstoffatom in R⁴ gegebenenfalls durch R¹⁹, COR¹⁹, SO₂R¹⁹ oder CO₂R¹⁹ substituiert ist;
wobei n 1 bis 6 ist, vorzugsweise ist n 1, 2 oder 3; vorzugsweise, wobei jedes substituierbare Kohlenstoff- oder Heteroatom in R⁴ gegebenenfalls und unabhängig durch ein oder mehrere von C₁₋₆-Alkyl, OR²⁰, SR²⁰, NO₂, CN, NR²⁰₂, NR²⁰COR²⁰, NR²⁰CONR²⁰₂, NR²⁰COR²⁰, NH-CO₂R²⁰, CO₂R²⁰, COR²⁰, CONR²⁰₂, S(O)₂R²⁰, SONR²⁰₂, S(O)R²⁰, SO₂NR²⁰2 oder NR²⁰S (O)₂R²⁰ substituiert ist;
wobei R²⁰ Wasserstoff, C₁₋₆-Alkyl oder C₁₋₆-Haloalkyl ist;
wobei R¹⁷ Wasserstoff, C₁₋₁₂-Alkyl, C₃₋₁₂-Carbocyclyl oder C₃₋₁₂-Heterocyclyl ist, gegebenenfalls substituiert durch ein oder mehrere von C₁₋₆-Alkyl, C₃₋₁₂-Carbocyclyl, C₃₋₁₂-Heterocyclyl, Halogen, C₁₋₆-Haloalkyl, OR²¹, SR²¹, NO₂, CN, NR²¹R²¹, NR²¹COR²¹, NR²¹-CONR²¹R²¹, NR²¹COR²¹, NR²¹CO₂R²¹, CO₂R²¹, COR²¹, CONR²¹, S(O)₂R²¹, SONR²¹₂, S(O)R²¹, SO₂NR²¹R²¹, NR²¹S (O)₂R²¹, wobei die C₁₋₁₂-Alkyl-Gruppe gegebenenfalls ein oder zwei Insertionen ausgewählt aus der Gruppe bestehend aus -O-, -N(R²¹)-, -S(O)- und -S(O₂)- inkorporiert, wobei jedes R²¹ gleich oder verschieden sein kann und wie unten definiert ist;
wobei zwei R¹⁷ in NR¹⁷₂ gegebenenfalls einen partiell gesättigten, ungesättigten, oder vollständig gesättigten drei- bis sieben-gliedrigen Ring bilden können, der ein bis drei Heteroatome enthält, gegebenenfalls und unabhängig substituiert durch ein oder mehrere von C₁₋₆-Alkyl, Halogen, C₁₋₆-Haloalkyl, OR²¹, SR²¹, NO₂, CN, NR²¹R²¹, NR²¹COR²¹, NR²¹CONR²¹R²¹, NR²¹COR²¹, NR²¹CO₂R²¹, CO₂R²¹, COR²¹, CONR²¹₂, S(O)₂R²¹, SONR²¹₂, S(O)R²¹, SO₂NR²¹R²¹, NR²¹S(O)₂R²¹, wobei die C₁₋₆-Alkyl-Gruppe gegebenenfalls ein oder zwei Insertionen ausgewählt aus der Gruppe bestehend aus -O-, -N(R²¹)-, -S(O)- und -S(O₂)- inkorporiert, wobei jedes R²¹ gleich oder verschieden sein kann und wie unten definiert ist;
wobei R¹⁸ Wasserstoff, C₁₋₁₂-Alkyl, C₃₋₁₂-Carbocyclyl oder C₃₋₁₂-Heterocyclyl ist, gegebenenfalls substituiert durch ein oder mehrere von C₁₋₆-Alkyl, Halogen, C₁₋₆-Haloalkyl, OR²¹, SR²¹, NO₂, CN, NR²¹R²¹, NR²¹COR²¹, NR²¹CONR²¹R²¹, NR²¹COR²¹, NR²¹CO₂R²¹, CO₂R²¹, COR²¹, CONR²¹₂, S(O)₂R²¹, S(O)R²¹, SO₂NR²¹R²¹, NR²¹S(O)₂R²¹, wobei die C₁-₁₂-Alkyl-Gruppe gegebenenfalls ein oder zwei Insertionen ausgewählt aus der Gruppe bestehend aus -O-, -N(R²¹)-, -S(O) - und -S(O₂)- inkorporiert, wobei jedes R²¹ gleich oder verschieden sein kann und wie unten definiert ist;
wobei R¹⁹ Wasserstoff, C₆₋₁₂-Aryl, C₁₋₆-Alkyl oder C₁₋₆-Haloalkyl ist;
wobei R²¹ Wasserstoff, C₁₋₆-Alkyl oder C₁₋₆-Haloalkyl ist.

2. Verbindung nach Anspruch 1, wobei R¹ eine gegebenenfalls substituierte fünf- oder sechs-gliedrige Carbocyclyl- oder Heterocyclyl-Gruppe ist, ausgewählt aus gegebenenfalls substituiertem Phenyl, Acridin, Benzimidazol, Benzofuran, Benzothiophen, Benzoxazol, Benzothiazol, Cyclohexyl-Furan, Imidazol, Indol, Isoindol, Isochinolin, Isoxazol, Isothiazol, Morpholin, Naphthalin, Oxazol, Phenazin, Phenothiazin, Phenoxazin, Piperazin, Piperidin, Pyrazol, Pyridazin, Pyridin, Pyrrol, Chinolin, Chinolizin, Tetrahydrofuran, Tetrazin, Tetrazol, Thiophen, Thiazol, Thiomorpholin, Thianaphthalin, Thiopyran, Triazin, Triazol oder Trithian.

3. Verbindung nach Anspruch 1 oder Anspruch 2, wobei R¹ eine Gruppe der Formel (II) ist, wobei X eine Gruppe NR³ ist, Y fehlt und ein oder mehrere von R² und R³ Wasserstoff, Alkyl oder Cycloalkyl sind.

4. Verbindung nach Anspruch 3, wobei die Gruppe der Formel (II) eine Alkylamino- oder Cycloalkylamino-Gruppe ist, vorzugsweise ausgewählt aus gegebenenfalls substituiertem Methylamino, Ethylamino, Propylamino, Isoprypylamino, Butylamino, Cyclobutylamino, Pentylamino, Cyclopentylamino, Hexylamino, Cyclohexylamino, Heptylamino, Cycloheptylamino, Octylamino und Cyclooctylamino.

5. Verbindung nach einem der Ansprüche 1 bis 4, wobei R¹ durch ein oder mehrere von OR²⁴, Halogen C₁₋₆-Alkyl, C₂₋₆-Alkenyl, C₂₋₆-Alkynyl, C₁₋₆-Haloalkyl, C₁₋₆-Alkylaryl, C₁₋₆-Alkylheterocyclyl, (CH₂)ₙOR²⁴, (CH₂)ₙNR²⁴₂, SR²⁴, NO₂, CN, NR²⁴₂, CO₂R²⁴, NR²⁴C(O)R²⁴, NR²⁴S (O)₂R²⁴, COR²⁴, CONR²⁴₂, S (O)₂R²⁴, S (O) R²⁴ oder SO₂NR²⁴₂ substituiert ist;
wobei R²⁴ Wasserstoff, C₁₋₄-Alkyl oder C₆₋₁₂-Aryl, vorzugsweise Phenyl, oder C₅₋₁₂-Heterocyclyl, vorzugsweise Pyridin, ist und n 1, 2, 3, 4, 5 oder 6 ist;
wobei zwei R²⁴₂ in NR²⁴ gegebenenfalls einen partiell gesättigten, ungesättigten oder vollständig gesättigten drei- bis sieben-gliedrigen Ring bilden können, der ein bis drei Heteroatome enthält, wobei dieser Ring vorzugsweise unabhängig durch ein oder mehrere von Halogen, C₁₋₁₂-Alkyl, C₂₋₁₂-Alkenyl, C₂₋₁₂-Alkynyl, C₁₋₁₂-Haloalkyl, C₃₋₁₂Carbocyclyl, C₃₋₁₂-Heterocyclyl, OR²⁵, SR²⁵, NO₂, CN, NR²⁵₂, NR²⁵COR²⁵, NR²⁵CONR²⁵₂, NR²⁵COR²⁵, NR²⁵CO₂R²⁵, CO₂R²⁵, COR²⁵, CONR²⁵₂, S(O)₂R²⁵, SONR²⁵₂, S(O)R²⁵, SO₂NR²⁵₂ oder NR²⁵S(O)₂R²⁵ substituiert ist; und jeder gesättigte Kohlenstoff im gegebenen Ring des Weiteren gegebenenfalls und unabhängig durch =O, =S, NNR²⁶₂, =N-OR²⁶, =NNR²⁶COR²⁶, =NNR²⁶CO₂R²⁶, =NNSO₂R²⁶ oder =NR²⁶ substituiert ist; und jedes substituierbare Stickstoffatom gegebenenfalls durch R²⁷, COR²⁷, SO₂R²⁷ oder CO₂R²⁷ substituiert ist;
wobei R²⁵ Wasserstoff, C₁₋₁₂-Alkyl, C₆₋₁₂-Carbocyclyl oder C₅₋₁₂-Heterocyclyl ist, gegebenenfalls substituiert durch ein oder mehrere von C₁₋₆-Alkyl, Halogen, C₁₋₆-Haloalkyl, OR²⁸, SR²⁸, NO₂, CN, NR²⁸R²⁸, NR²⁸COR²⁸, NR²⁸CONR²⁸R²⁸, NR²⁸COR²⁸, NR²⁸CO₂R²⁸, CO₂R²⁸, COR²⁸, CONR²⁸₂, S(O)₂R²⁸, SONR²⁸₂, S(O)R²⁸, SO₂NR²⁸R²⁸, NR²¹S (O)₂R²⁸, wobei die C₁₋₁₂-Alkyl-Gruppe gegebenenfalls ein oder zwei Insertionen ausgewählt aus der Gruppe bestehend aus -O-, -N(R²⁸)-, -S(O)- und S(O₂)- inkorporiert, wobei jedes R²⁸ gleich oder verschieden sein kann und wie unten definiert ist;
wobei R²⁶ Wasserstoff, C₁₋₁₂-Alkyl, C₆₋₁₂-Carbocyclyl oder C₅₋₁₂-Heterocyclyl ist, gegebenenfalls substituiert durch ein oder mehrere von C₁₋₆-Alkyl, Halogen, C₁₋₆-Haloalkyl, OR²⁸, SR²⁸, NO₂, CN, NR²⁸R²⁸, NR²⁸COR²⁸, NR²⁸CONR²⁸R²⁸, NR²⁸COR²⁸, NR²⁸CO₂R²⁸, CO₂R²⁸, COR²⁸, CONR²⁸₂, S(O)₂R²⁸, S(O)R²⁸, SO₂NR²⁸R²⁸, NR²⁸S(O)₂R²⁸, wobei die C₁₋₁₂-Alkyl-Gruppe gegebenenfalls ein oder zwei Insertionen ausgewählt aus der Gruppe bestehend aus -O-, -N(R²⁸)-, -S(O)- und S(O₂)- inkorporiert, wobei jedes R²⁸ gleich oder verschieden sein kann und wie unten definiert ist;
wobei R²⁷ Wasserstoff, C₁₋₆-Alkyl, C₁₋₆-Haloalkyl oder C₆₋₁₂-Aryl ist;
wobei R²⁸ Wasserstoff, C₁₋₆-Alkyl oder C₁₋₆-Haloalkyl ist.

6. Verbindung nach einem der Ansprüche 1 bis 5, wobei R⁴ aus Phenyl, Cyclohexyl, Acridin, Benzimidazol, Benzofuran, Benzothiophen, Benzoxazol, Benzothiazol, Indol, Isoindol, Indolizin, Indazol, Isoindol, Isochinolin, Morpholin, Naphthalen, Phenazin, Phenothiazin, Phenoxazin, Piperazin, Piperidin, Pyridazin, Pyridin, Pyrimidinyl, Pyrazinyl, Chinolin, Chinolizin, Tetrazin, Thiomorpholin, Thianaphthalin, Thiopyran, Triazin, Trithian, Furan, Imidazol, Isoxazol, Isothiazol, Oxazol, Oxadiazol, Oxathiazol, Pyrazol, Pyrrol, Tetrazol, Thiophen, Thiadiazol, Thiatriazol, Thiazol oder Triazol ausgewählt wird, wobei jedes substituierbare Kohlenstoff- oder Heteroatom in R⁴ gegebenenfalls und unabhängig durch ein oder mehrere von C₁₋₆-Alkyl, OR²⁰, SR²⁰, NO₂, CN, NR²⁰₂, NR²⁰COR²⁰, NR²⁰CONR²⁰₂, NR²⁰COR²⁰, NHCO₂R²⁰, CO₂R²⁰, COR²⁰, CONR²⁰₂, S(O)₂R²⁰, SONR²⁰₂, S(O)R²⁰, SO₂NR²⁰₂ oder NR²⁰S (O)₂R²⁰ substituiert ist;
wobei R²⁰ Wasserstoff, C₁₋₆-Alkyl oder C₁₋₆-Haloalkyl ist.

7. Verbindung nach einem der Ansprüche 1 bis 6, wobei R⁴ eine sechs-gliedrige Carbocyclyl- oder Heterocyclyl-Gruppe ist, die gegebenenfalls durch ein oder mehrere von OR²⁹, NR²⁹₂, SR²⁹, (CH₂)ₙOR²⁹, (CH₂)ₙNR²⁹₂, Halogen, C₁₋₆-Alkyl, C₂₋₆-Alkenyl, C₂₋₆-Alkynyl, Haloalkyl, NO₂, CN, NR²⁹C(O)R²⁹, NR²⁹S(O)₂R²⁹, CO₂R²⁹, COR²⁹, CONR²⁹₂, S(O)₂R²⁹, S(O)R²⁹ oder SO₂NR²⁹₂ substituiert ist;
wobei R²⁹ Wasserstoff, C₁₋₄-Alkyl, C₅₋₁₂-Heterocyclyl oder C₆₋₁₂-Aryl, vorzugsweise Phenyl, ist und n 1, 2, 3, 4, 5 oder 6 ist;
wobei zwei R²⁹ in NR²⁹₂ gegebenenfalls einen partiell gesättigten, ungesättigten oder vollständig gesättigten fünf- bis sieben-gliedrigen Ring bilden können, der ein bis drei Heteroatome enthält, gegebenenfalls und unabhängig substituiert durch ein oder mehrere von Halogen, C₁₋₁₂-Alkyl, C₂₋₁₂-Alkenyl, C₂₋₁₂-Alkynyl, C₁₋₁₂-Haloalkyl, C₆₋₁₂-Carbocyclyl, C₅₋₁₂-Heterocyclyl, OR³⁰, SR³⁰, NO₂, CN, NR³⁰₂, NR³⁰COR³⁰, NR³⁰CONR³⁰₂, NR³⁰COR³⁰, NR³⁰CO₂R^{3O}, CO₂R³⁰, COR³⁰, CONR³⁰₂, S(O)₂R³⁰, SONR³⁰₂, S(O)R³⁰, SO₂NR³⁰₂ oder NR³⁰S (O)₂R³⁰; und jeder gesättigte Kohlenstoff im gegebenen Ring des Weiteren gegebenenfalls und unabhängig durch =O, =S, NNR³¹₂, =N-OR³¹, =NNR³¹-COR³¹, =NNR³¹CO₂R³¹, =NNSO₂R³¹ oder =NR³¹ substituiert ist; und jedes substituierbare Stickstoffatom gegebenenfalls durch R³², COR³², SO₂R³² oder CO₂R¹² substituiert ist;
wobei R³⁰ Wasserstoff, C₁₋₁₂-Alkyl, C₆₋₁₂-Carbocyclyl oder C-₅₋₁₂-Heterocyclyl ist, gegebenenfalls substituiert durch ein oder mehrere von C₁₋₆-Alkyl, Halogen, C₁₋₆-Haloalkyl, OR³³, SR³³, NO₂, CN, NR³³R³³, NR³³COR³³, NR³³CONR³³R³³, NR³³COR³³, NR³³CO₂R³³, CO₂R³³, COR³³, CONR³³₂, S(O)₂R³³, SONR³³₂, S(O)R³³, SO₂NR³³R³³, NR³³S(O)₂R³³, wobei die C₁₋₁₂-Alkyl-Gruppe gegebenenfalls ein oder zwei Insertionen ausgewählt aus der Gruppe bestehend aus -O-, -N(R³³)-, -S(O)- und -S(O₂)- inkorporiert, wobei jedes R³³ gleich oder verschieden sein kann und wie unten definiert ist;
wobei R³¹ Wasserstoff, C₁₋₁₂-Alkyl, C₁₋₆-Carbocyclyl oder C₅₋₁₂-Heterocyclyl ist, gegebenenfalls substituiert durch ein oder mehrere von C₁₋₆-Alkyl, Halogen, C₁₋₆-Haloalkyl, OR³³, SR³³, NO₂, CN, NR³³R³³, NR³³COR³³, NR³³CONR³³R³³, NR³³COR³³, NR³³CO₂R³³, CO₂R³³, COR³³, CONR³³₂, S(O)₂R³³, S(O)R³³, SO₂NR³³R³³, NR³³S(O)₂R³³, wobei die C₁₋₁₂-Alkyl-Gruppe gegebenenfalls ein oder zwei Insertionen ausgewählt aus der Gruppe bestehend aus -O-, -N(R³³)-, -S(O)- und -S(O₂) - inkorporiert, wobei jedes R²¹ gleich oder verschieden sein kann und wie unten definiert ist;
wobei R³² Wasserstoff, C₆₋₁₂-Aryl, C₁₋₆-Alkyl oder C₁₋₆-Haloalkyl ist;
wobei R³³ Wasserstoff, C₁₋₆-Alkyl oder C₁₋₆-Haloalkyl ist.

8. Verbindung nach einem der Ansprüche 1 bis 7, wobei R⁴ ein fünf-gliedriges Heterocyclyl ist 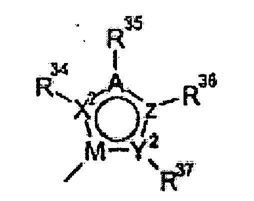
wobei A, X², Y² oder Z unabhängig aus N, 0, C, S ausgewählt werden und M C oder N ist, wobei ein, zwei, drei oder vier von A X², Y², Z und M anders als C ist;
R³⁴, R³⁵, R³⁶ oder R³⁷ unabhängig aus einem einzelnen Elektronenpaar, Wasserstoff, Halogen C₁₋₁₂-Alkyl, C₁₋₁₂-Haloalkyl, OR³⁸, SR³⁸, NO₂, CN, NR³⁸₂, NR³⁸COR³⁸, NR³⁸CONR³⁸₂, NR³⁸COR³⁸, NR³⁸CO₂R³⁸, (CH₂)ₙOR³⁸, (CH₂)ₙNR³⁸₂, CO₂R³⁸, COR³⁸, CONR³⁸₂, S(O)₂R³¹, SONR³⁸₂, S(O)R³⁸, SO₂NR³⁸₂ oder NHS (O)₂R³⁸ ausgewählt werden;
wobei n 1 bis 6 ist, vorzugsweise ist n 1, 2 oder 3;
oder wobei zwei beliebige R³⁴, R³⁵, R³⁶ oder R³⁷ gegebenenfalls einen partiell gesättigten, ungesättigten oder vollständig gesättigten fünf- bis sieben-gliedrigen Ring bilden können, der null bis drei Heteroatome enthält, wobei jeder gesättigter Kohlenstoff im gegeben fusionierten Ring des Weiteren gegebenenfalls und unabhängig durch ein oder mehrere von Halogen, C₁₋₁₂-Alkyl, C₂₋₁₂-Alkenyl, C₂₋₁₂-Alkynyl, C₁₋₁₂-Haloalkyl, C₆₋₁₂-Carbocyclyl, C₅₋₁₂-Heterocyclyl, OR³⁸, SR³⁸, NO₂, CN, NR³⁸₂, NR³⁸CONR³⁸₂, NR³⁸-COR³⁸, NR³⁸CO₂R³⁸, (CH₂)ₙOR³⁸, (CH₂)ₙNR³⁸₂, CO₂R³⁸, COR³⁸, CONR³⁸₂, S(O)₂R³⁸, SONR³⁸₂, S(O)R³⁸, SO₂NR³⁸₂ oder NR³⁸S (O)₂R³⁸ substituiert ist; und jeder gesättigte Kohlenstoff im gegebenen fusionierten Ring des Weiteren gegebenenfalls und unabhängig durch =O, =S, NNR³⁹₂, =N-OR³⁹, =NNR³⁵COR³⁹, =NNR³⁹CO₂R³⁹, =NNSO₂R³⁹ oder =NR³⁹ substituiert ist; und jedes substituierbare Stickstoffatom in R⁴ gegebenenfalls durch R⁴⁰, COR¹⁰, SO₂R⁴⁰ oder CO₂R⁴⁰ substituiert ist;
wobei n 1 bis 6 ist, vorzugsweise ist n 1, 2, oder 3;
wobei R³⁸ Wasserstoff, C₁₋₁₂-Alkyl, C₆₋₁₂-Carbocyclyl oder C₅₋₁₂-Heterocyclyl ist, gegebenenfalls substituiert durch ein oder mehrere von C₁₋₆-Alkyl, Halogen C₁₋₆-Haloalkyl, OR⁴¹, SR⁴¹, NO₂, CN, NR⁴¹R⁴¹, NR⁴¹CONR⁴¹R⁴¹, NR⁴¹COR⁴¹, NR⁴¹CO₂R⁴¹, CO₂R⁴¹, COR⁴¹, CONR⁴¹₂, S(O)₂R⁴¹, SONR⁴¹₂, S(O)R⁴¹, SO₂NR⁴¹R⁴¹, NR⁴¹S(O)₂R⁴¹, wobei die C₁₋₁₂-Alkyl-Gruppe gegebenenfalls ein oder zwei Insertionen ausgewählt aus der Gruppe bestehend aus -O-, -N(R⁴¹)-, -S(O)- und S(O₂)- inkorporiert, wobei jedes R⁴¹ gleich oder verschieden sein kann und wie unten definiert ist;
wobei R³⁹ Wasserstoff, C₁₋₁₂-Alkyl, Carbocyclyl oder Heterocyclyl ist, gegebenenfalls substituiert durch ein oder mehrere von C₁₋₆-Alkyl, Halogen, C₁₋₆-Haloalkyl, OR⁴¹, SR41, NO₂, CN, NR⁴¹R⁴¹, NR⁴¹-COR⁴¹, NR⁴¹CONR⁴¹R⁴¹, NR⁴¹COR⁴¹, NR⁴¹CO₂R⁴¹, CO₂R⁴¹, COR⁴¹, CONR⁴¹₂, S (O)₂R⁴¹, S (O) R⁴¹, SO₂NR⁴¹R⁴¹, NR⁴¹S(O)₂R⁴¹, wobei die C₁₋₁₂-Alkyl-Gruppe gegebenenfalls ein oder zwei Insertionen ausgewählt aus der Gruppe bestehend aus -O-, -N(R⁴¹)-, -S(O)- und S(O₂) - inkorporiert, wobei jedes R⁴¹ gleich oder verschieden sein kann und wie unten definiert ist;
wobei R⁴⁰ Wasserstoff, C₁₋₆-Alkyl, C₁₋₆-Haloalkyl oder C₆₋₁₂-Aryl ist;
wobei R⁴¹ Wasserstoff, C₁₋₆-Alkyl oder C₁₋₆-Haloalkyl ist.

9. Verbindung nach Anspruch 8, wobei R⁴ Furan, Imidazol, Isoxazol, Isothiazol, Oxazol, Oxadiazol, Oxatriazol, Pyrazol, Pyrrol, Tetrazol, Thiophen, Thiadiazol, Thiatriazol, Thiazol oder Triazol ist; und R³⁴, R³⁵, R³⁶ oder R³⁷ unabhängig aus einem einzelnen Elektronenpaar, Wasserstoff, Halogen, C₁₋₆-Alkyl, C₁₋₆-Haloalkyl, OR⁴², SR⁴², CN, NR⁴²₂, NR⁴²COR⁴², CO₂R⁴², COR⁴², CONR⁴²₂, S(O)₂R⁴² oder S(O)R¹² ausgewählt werden;
wobei R⁴² Wasserstoff, C₁₋₄-Alkyl, vorzugsweise Methyl oder Ethyl, oder Carbocyclyl, vorzugsweise Phenyl, ist.

10. Verbindung nach einem der Ansprüche 1 bis 9, ausgewählt aus

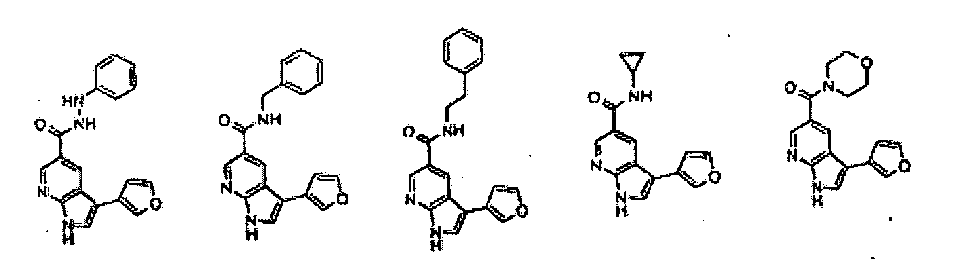

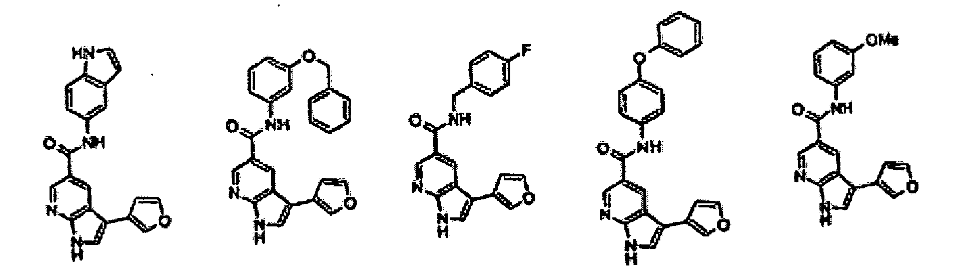

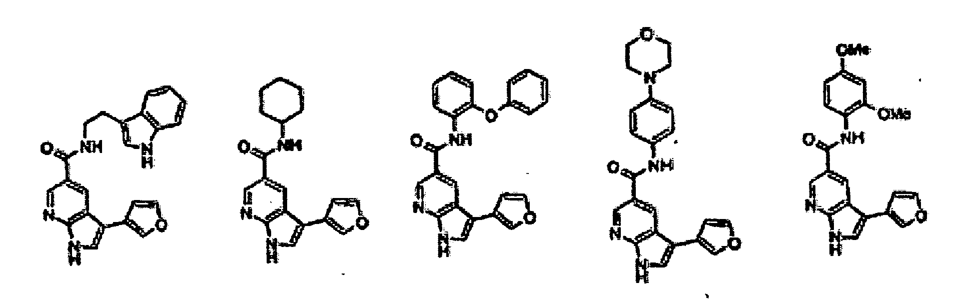

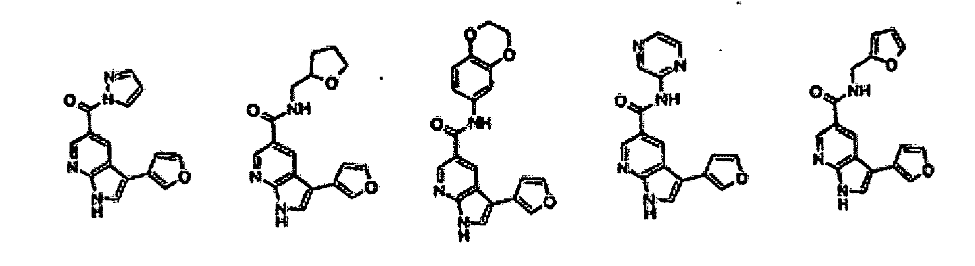

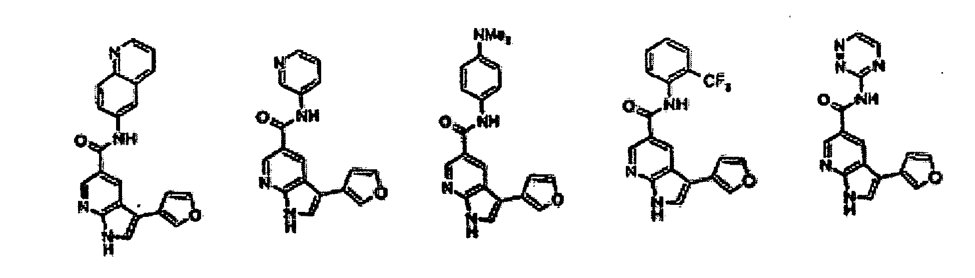

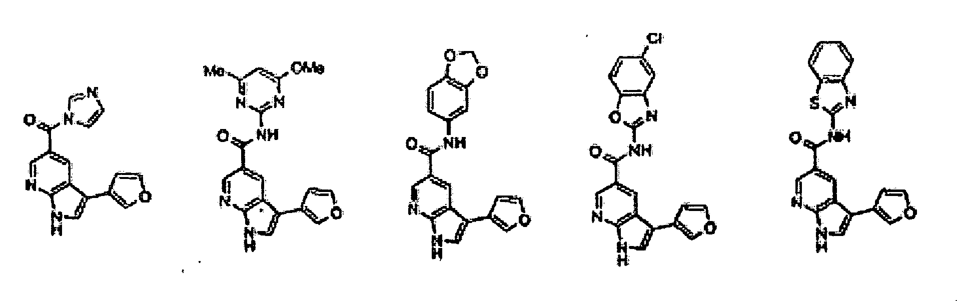

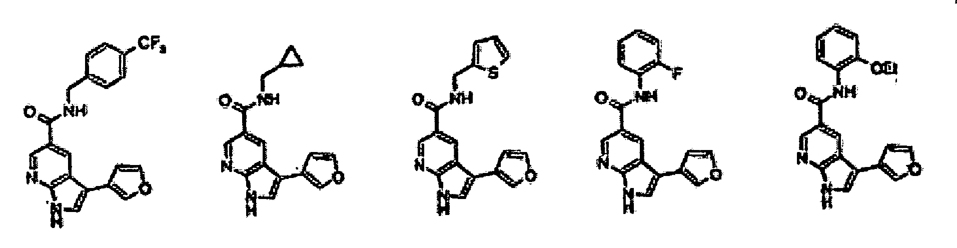

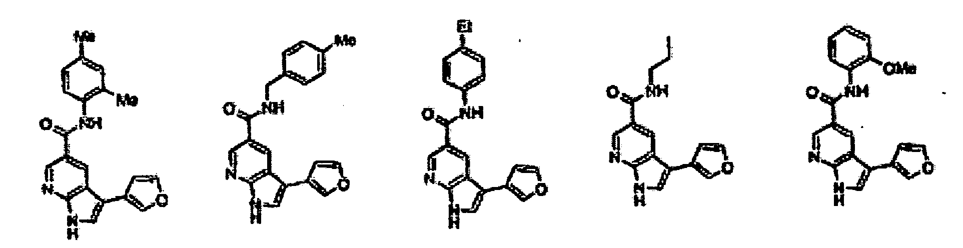

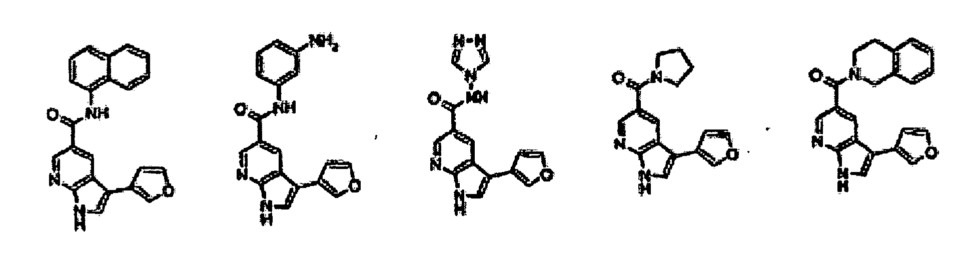

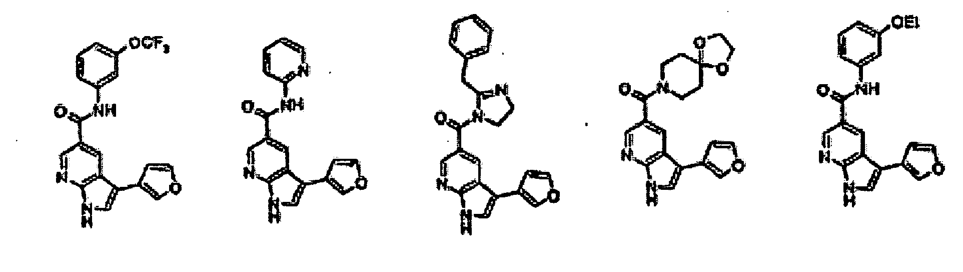

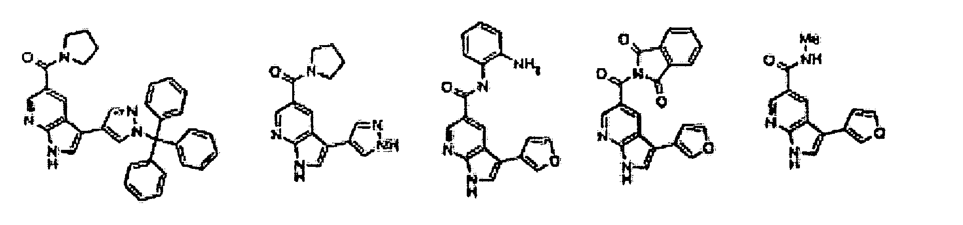

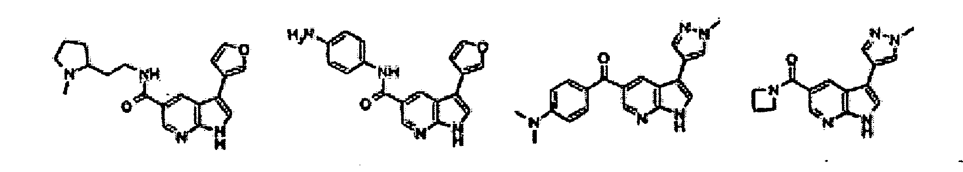

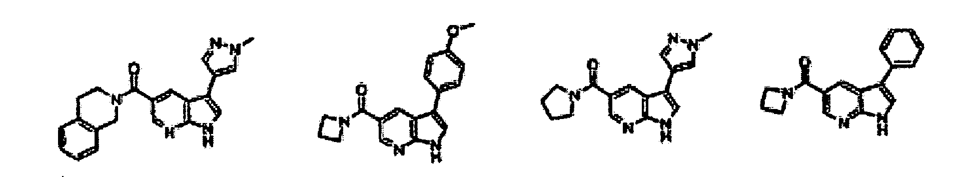

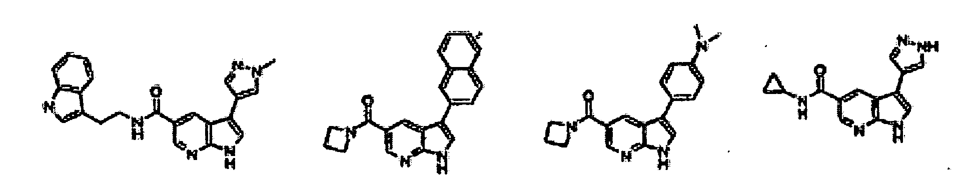

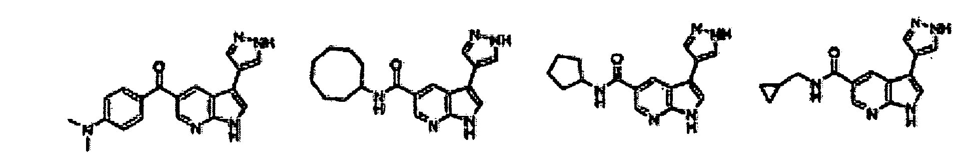

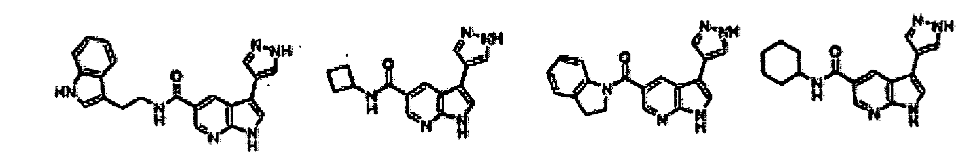

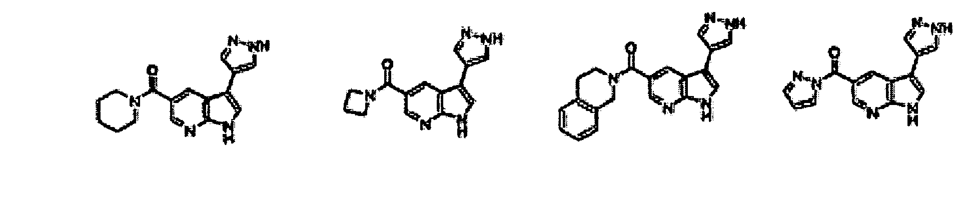

11. Verfahren zur Herstellung einer Verbindung der Formel (I), wobei R¹ eine Gruppe der Formel (II), wie sie in einem der Ansprüche 1 bis 10 der Erfindung definiert wird, ist, welches die Kondensation eines Zwischenprodukts (III) mit einem Zwischenprodukt (IV) umfasst, 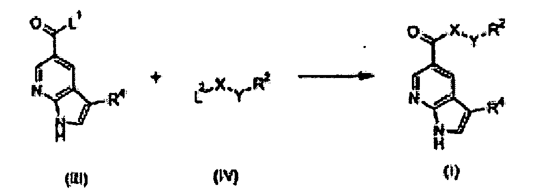 wobei R² und R⁴ sind, wie sie in einem der Ansprüche 1 bis 10 definiert werden; und wobei jedes L¹ und L² unabhängig eine Abgangsgruppe ist, wobei L¹ und L² zusammen ein Kondensationsprodukt bilden.

12. Verfahren nach Anspruch 11, wobei L¹ OH, OR⁵⁰, OM, CI, Br oder I ist, wobei R⁵⁰ C₁₋₆-Alkyl ist, vorzugsweise Methyl oder Ethyl und M Na, Li, K, Ca, Mg oder Ba ist und L² Wasserstoff oder M ist.

13. Pharmazeutische Zusammensetzung, die eine Verbindung nach einem der Ansprüche 1 bis 10 in Kombination mit einem pharmazeutisch akzeptablen Träger, Verdünnungsmittel oder Hilfsstoff umfasst.

14. Zusammensetzung nach Anspruch 13, die zusätzlich ein oder mehrere von einem entzündungshemmendem Wirkstoff, einem AMPA-Rezeptor-Antagonisten, einem Chemotherapeutikum und/oder einem antiproliferativem Mittel umfasst.

15. Verfahren zur Herstellung einer Zusammensetzung gemäß Anspruch 13 oder 14, welches Kombinieren einer Verbindung gemäß einem der Ansprüche 1 bis 10 der Erfindung mit einem pharmazeutisch akzeptablen Träger oder Verdünnungsmittel und gegebenenfalls mit einem oder mehreren beliebigen zusätzlichen Wirkstoffen aus Anspruch 14 umfasst.

16. Verbindung nach einem der Ansprüche 1 bis 10, oder Zusammensetzung nach Anspruch 10 oder Anspruch 14, für die medizinische Verwendung.

17. Verbindung, wie sie in einem der Ansprüche 1 bis 10 definiert wird, oder Zusammensetzung, wie sie in einem der Ansprüche 13 oder 14 definiert wird, zur JNK-Hemmung.

18. Verbindung, wie sie in einem der Ansprüche 1 bis 10 definiert wird, oder Zusammensetzung, wie sie in einem der Ansprüche 13 oder 14 definiert wird, zur selektiven JNK3-Hemmung.

19. Verbindung, wie sie in einem der Ansprüche 1-10 definiert wird, oder Zusammensetzung, wie sie in einem der Ansprüche 13 oder 14 definiert wird, für die Verwendung bei der Prävention oder Behandlung einer durch JNK ausgelösten Erkrankung.

20. Verbindung oder Zusammensetzung nach Anspruch 19, wobei es sich bei der Erkrankung um eine neurodegenerative Erkrankung (einschließlich Demenz), Entzündungserkrankung, eine Erkrankung, die mit Apoptose im Zusammenhang steht, insbesondere neuronale Apoptose, eine Autoimmunerkrankungen, destruktive Knochenerkrankung, proliferative Erkrankung, Krebs, Infektionskrankheit, Allergie, Ischämie-Reperfusionsverletzung, Herzinfarkt, angiogene Erkrankung, Organ-Hypoxie, vaskuläre Hyperplasie, Herzhypertrophie, thrombininduzierte Plättchenaggregation und/oder jeden Zustand, der mit Prostaglandin-Endoperoxidase-Synthase-2 in Verbindung gebracht wird, handelt.

21. Verbindung oder Zusammensetzung nach Anspruch 20, wobei die Ursache der neurodegenerativen Erkrankung Apoptose und/oder eine Entzündung ist.

22. Verbindung oder Zusammensetzung nach Anspruch 20 oder 21, wobei es sich bei der neurodegenerativen Erkrankung um Demenz; Alzheimer-Krankheit; Parkinson-Krankheit; amyotrophische Lateralsklerose; Huntington-Krankheit; senile Chorea; Sydenham-Chorea; Hypoglykämie; Kopf- und Rückenmarks-Trauma, einschließlich traumatischer Kopfverletzung; akute und chronische Schmerzen; Epilepsie und Krampfanfälle; olivopontocerebellare Demenz; neuronalen Zelltod; mit Hypoxie zusammenhängende Neurodegeneration; akute Hypoxie; Glutamat-Toxizität, einschließlich Glutamat-Neurotoxizität; cerebrale Ischämie; Demenz, die mit Meningitis und/oder Neurose im Zusammenhang steht; oder Demenz bei HIV-infizierten Patienten, handelt.

23. Verbindung oder Zusammenstellung nach Anspruch 20 oder 21, wobei es sich bei der neurodegenerativen Erkrankung um eine periphere Neuropathie, einschließlich Mononeuropathie, multiple Mononeuropathie oder Polyneuropathie, wie sie bei Diabetes mellitus, Lyme-Krankheit oder Urämie auftreten kann; periphere Neuropathie verursacht durch einen toxischen Wirkstoff; eine demyelinisierende Krankheit wie zum Beispiel akute oder chronische entzündliche Polyneuropathie, Leukodystrophien oder Guillain-Barre-Syndrom; multiple Mononeuropathie sekundär zu einer kollagen-vaskulären Krankheit; multiple Mononeuropathie sekundär zu Sarkoidose; multiple Mononeuropathie sekundär zu einer Stoffwechselkrankheit; oder multiple Mononeuropathie sekundär zu einer Infektionskrankheit, handelt.

24. Verbindung oder Zusammenstellung nach Anspruch 20, wobei es sich bei der Erkrankung um eine entzündliche Darmerkrankung, Bronchitis, Asthma, akute Pankreatitis, chronische Pankreatitis, Allergien verschiedenster Art, Alzheimer-Krankheit, oder eine Autoimmunerkrankung handelt, wie zum Beispiel rheumatoide Arthritis, systemischen Lupus erythematodes, Glomerulonephritis, Sklerodermie, chronische Thyroiditis, Graves-Krankheit, autoimmune Gastritis, Diabetes, autoimmunhämolytische Anämie, autoimmune Neutropenie, Thrombozytopenie, atopische Dermatitis, chronisch-aktive Hepatitis, Myasthenia gravis, Multiple Sklerose, Colitis ulcerosa, Morbus Crohn, Psoriasis oder Graft-versus-Host-Krankheit.

25. Verwendung einer Verbindung, wie sie in Anspruch 1-10 definiert wird, für die Herstellung eines Medikaments zur Prävention oder für die Behandlung einer durch JNK hervorgerufenen Erkrankung.

## Revendications

1. Composé de formule (I) 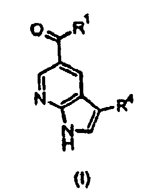 et ses sels pharmaceutiquement acceptables ;
où R¹ est un carbocyclyle en C₃₋₁₂ ou un groupe hétérocyclyle en C₃₋₁₂, éventuellement fondu en un cycle à cinq à sept éléments partiellement saturé, insaturé ou totalement saturé contenant zéro à trois hétéroatomes, et chaque atome de carbone substituable en R¹, comprenant le cycle éventuellement fondu, est éventuellement et indépendamment substitué par un ou plusieurs atomes d'halogène, alkyle en C₁₋₁₂, alcényle en C₂₋₁₂, alcynyle en C₂₋₁₂, haloalkyle en C₁₋₁₂, carbocyclyle en C₃₋₁₂, hétérocyclyle en C₃₋₁₂, (CH₂)ₙOR⁵, (CH₂)ₙNR⁵₂ (CH₂)ₙSR⁵, OR⁵, SR⁵, NO₂, CN, NR⁵₂, NR⁵COR⁵, NR⁵CONR⁵₂, NR⁵COR⁵, NR⁵CO₂R⁵, CO₂R⁵, COR⁵, CONR⁵₂, S(O)₂R⁵, SONR⁵₂, S(O)R⁵, SO₂NR⁵₂, ou NR⁵S(O)₂R⁵, où le groupe alkyle en C₁₋₁₂ contient éventuellement une ou plusieurs insertions choisies parmi -O-, un -N(R⁵)- -S-, -S(O)- et -S(O₂) - ; et chaque atome de carbone saturé dans le cycle fondu éventuel est en outre éventuellement et indépendamment substitué par =O, =S, NNR⁶₂, =N-OR⁶, =NNR⁶COR⁶, =NNR⁶CO₂R⁶, =NNSO₂R⁶, ou =NR⁶, et chaque atome d'azote substituable en R¹ est éventuellement substitué par R⁷, COR⁷, SO₂R⁷ ou CO₂R⁷ ;
où n est égal à 1 à 6, de préférence n est égal à 1, 2 ou 3 ;
où R⁵ est un atome d'hydrogène, un alkyle en C₁₋₁₂, un carbocyclyle en C₃₋₁₂, ou un hétérocyclyle en C₃₋₁₂, éventuellement substitué par un ou plusieurs éléments parmi un alkyle en C₁₋₆, un carbocyclyle en C₃₋₁₂, un hétérocyclyle en C₃₋₁₂, un halogène, un haloalkyle en C₁₋₆, un OR⁸, SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S (O)₂R⁸, SONR⁸₂, S(O)R⁸, SO₂NR⁸R⁸, NR⁸S(O)₂R⁸, où le groupe alkyle en C₁₋₁₂ comprend éventuellement une ou deux insertions choisies dans le groupe constitué de -O-, -N(R⁸)-, -S(O)- et -S(O₂)-, où chaque R⁸ peut être identique ou différent et est comme défini ci-dessous ;
où deux R⁵ dans NR⁵₂ peuvent éventuellement former un cycle à trois à sept éléments partiellement saturés, insaturés ou totalement saturés contenant un à trois hétéroatomes, éventuellement et indépendamment substitués par un ou plusieurs éléments parmi un alkyle en C₁₋₆, un halogène, un haloalkyle en C₁₋₆, un OR⁸, un SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S(O)₂R⁸, SONR⁸₂, S(O)R⁸, SO₂NR⁸R⁸, NR⁸S(O)₂R⁸,
où le groupe alkyle en C₁₋₆ comprend éventuellement une ou deux insertions choisies dans le groupe constitué de -O-, -N(R⁸)-, -S(O)- et -S(O₂)-, où chaque R⁸ peut être identique ou différent et est comme défini ci-dessous ;
où R⁶ est un hydrogène, un alkyle en C₁₋₁₂, un carbocyclyle en C₃₋₁₂, ou un hétérocyclyle en C₃₋₁₂, éventuellement substitué par un ou plusieurs éléments parmi un alkyle en C₁₋₆, un halogène, un haloalkyle en C₁₋₆, un OR⁸, un SR⁸, NO₂, CN, NR⁸R⁸, NR⁸COR⁸, NR⁸CONR⁸R⁸, NR⁸COR⁸, NR⁸CO₂R⁸, CO₂R⁸, COR⁸, CONR⁸₂, S(O)₂R⁸, S(O)R⁸, SO₂NR⁸R⁸, NR⁸S(O)₂R⁸, où le groupe alkyle en C₁₋₁₂ comprend éventuellement une ou deux insertions choisies dans le groupe constitué de -O-, -N(R⁸)-, -S(O)- et -S(O₂)-, où chaque R⁸ peut être identique ou différent et est comme défini ci-dessous ;
où R⁷ est un hydrogène, un aryle en C₆₋₁₂, un alkyle en C₁₋₆ ou un haloalkyle en C₁₋₆ ;
où R⁸ est un hydrogène, un alkyle en C₁₋₆ ou un haloalkyle en C₁₋₆ ou où R¹ est un groupe de formule (II) 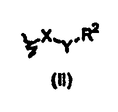
où X est un NR³, O, S, ou (CR²²R²²)ₙ, Y est absent ou est un NR²³, O ou (CR²³R²³)ₙ où chaque R²³ est un H, un alkyle en C₁₋₄, un alcoxy en C₁₋₄, ou un haloalkyle en C₁₋₄ ;
et n est égal à 1 à 6, de préférence n est égal à 1, 2, 3 ou 4 ; et
R² est un alkyle en C₁₋₁₂ éventuellement substitué, un alcényle en C₁₋₁₂, un alcynyle en C₂₋₁₂, un carbocyclyle en C₃₋₁₂ ou un hétérocyclyle en C₃₋₁₂, dans lequel le groupe carbocyclyle ou hétérocyclyle éventuellement substitué est éventuellement fondu à un à trois cycles à cinq à sept éléments insaturés, partiellement saturés ou totalement saturés contenant zéro à trois hétéroatomes ;
chaque atome de carbone substituable en R², y compris le cycle éventuellement fondu, est éventuellement et indépendamment substitué par un ou plusieurs éléments parmi un alkyle en C₁₋₁₂, un cycloalkyle en C₃₋₁₂, un hétérocycloalkyle en C₃₋₁₂, un aryle en C₃₋₁₂, un hétéroaryle halogène en C₃₋₁₂, un haloalkyle en C₁₋₁₂, un OR⁹, un SR⁹, NO₂, CN, NR⁹R⁹, NR⁹COR⁹, NR⁹CONR⁹R⁹, NR⁹COR⁹, NR⁹CO₂R⁹, CO₂R⁹, COR⁹, CONR⁹R⁹, S(O)₂R⁹, SONH₂, S(O)R⁹, SO₂NR⁹R⁹, NR⁹S (O)₂R, où chaque R⁹ peut être identique ou différent et est comme défini ci-dessous et où :
le groupe alkyle en C₁₋₁₂ comprend éventuellement une ou deux insertions choisies dans le groupe constitué de -O-, -C(O)-, -N(R⁹)-, -S(O)- et - S(O₂)-, où chaque R⁹ peut être identique ou différent et est comme défini ci-dessous ;
les groupes alkyle en C₁₋₁₂, cycloalkyle en C₃₋₁₂, hétérocycloalkyle en C₃₋₁₂ , aryle en C₃₋₁₂ ou hétéroaryle en C₃₋₁₂ sont éventuellement substitués par un ou plusieurs éléments parmi un halogène, un haloalkyle en C₁₋₁₂, un OR⁹, un SR⁹, NO₂, CN, NR⁹R⁹, NR⁹COR⁹, NR⁹CONR⁹R⁹, NR⁹COR⁹, NR⁹CO₂R⁹, CO₂R⁹, COR⁹, CONR⁹R⁹, S(O)₂R⁹, SONH₂, S(O)R⁹, SO₂NR⁹R⁹, NR⁹S(O)₂R⁹, où chaque R⁹ peut être identique ou différent et est comme défini ci-dessous et
les groupes cycloalkyle en C₃₋₁₂, hétérocycloalkyle en C₃₋₁₂, aryle en C₃₋₁₂ ou hétéroaryle en C₃₋₁₂ sont éventuellement substitués par un ou plusieurs éléments parmi les groupes alkyle en C₁₋₁₂ ;
chaque atome de carbone saturé en R², y compris le cycle fondu optionnel, est en outre éventuellement et indépendamment substitué par =O, =S, NNR⁹R⁹, =N-OR⁹, =NNHCOR⁹, =NNHCO₂R⁹, =NNSO₂R⁹, ou =NR⁹, où chaque R⁹ peut être identique ou différent et est comme défini ci-dessous ; et
chaque atome d'azote substituable en R² est éventuellement substitué par R¹⁰, COR⁹, SO₂R⁹, ou CO₂R⁹, où chaque R⁹ et R¹⁰ peut être identique ou différent et est comme défini ci-dessous ;
où deux R⁹ dans NR⁹₂ peuvent éventuellement former un cycle à trois à sept éléments partiellement saturé, insaturé ou totalement saturé contenant un à trois hétéroatomes, éventuellement et indépendamment substitués par un ou plusieurs alkyle en C₁₋₆, un halogène, un haloalkyle en C₁₋₆, un OR¹¹, SR¹¹, NO₂, CN, NR¹¹R¹¹, NR¹¹COR¹¹, NR¹¹CONR¹¹R¹¹, NR¹¹COR¹¹, NR¹¹CO₂R¹¹, CO₂R¹¹, COR¹¹, CONR¹¹₂, S(O)₂R¹¹, SONR¹¹₂, S(O)R¹¹, SO₂NR¹¹R¹¹, NR¹¹S(O)₂R¹¹,
où le groupe alkyle en C₁₋₆ comprend éventuellement une ou deux insertions choisies dans le groupe constitué de -O-, -N(R¹¹)-, -S(O)-, et -S(O₂) - où chaque R¹¹ peut être identique ou différent et est comme défini ci-dessous ;
où R¹¹ est un hydrogène, un alkyle en C₁₋₆, ou un haloalkyle en C₁₋₆ ;
où R⁹ est un hydrogène, un alkyle en C₁₋₁₂ ou un aryle en C₃₋₁₂, éventuellement substitué par un ou plusieurs éléments parmi un alkyle en C₁₋₄, un halogène, un haloalkyle en C₁₋₄, un OR¹², SR¹², NO₂, CN, NR¹²R¹², NR¹²COR¹², NR¹²CONR¹²R¹², NR¹²COR¹², NR¹²CO₂R¹², CO₂R¹², COR¹², CONR¹²₂, S(O)₂R¹², SONH₂, S(O)R¹², SO₂NR¹²R¹², NR¹²S (O)₂R¹², où le groupe alkyle en C₁₋₁₂ comprend éventuellement une ou deux insertions choisies dans le groupe constitué de -O-, -N(R¹²)-, -S(O)-, et -S(O₂)- où chaque R¹² peut être identique ou différent et est comme défini ci-dessous ;
où R¹⁰ est un alkyle en C₁₋₁₂ ou un aryle en C₃₋₁₂ éventuellement substitué par un ou plusieurs éléments parmi un alkyle en C₁₋₄, un halogène, un haloalkyle en C₁₋₄, un OR¹², SR¹², NO₂, CN, NR¹²R¹², NR¹²COR¹², NR¹²CONR¹²R¹² , NR¹²COR¹², NR¹²CO₂R¹², CO₂R¹², COR¹², CONR¹²₂, S(O)2R¹², SONH₂, S(O)R¹², SO₂NR¹²R¹², NR¹²S(O)₂R¹², où le groupe alkyle en C₁₋₁₂ comprend éventuellement une ou deux insertions choisies dans le groupe constitué de -O-, -N(R¹²) -, -S(O)-, et -S(O₂)- où chaque R¹² peut être identique ou différent et est comme défini ci-dessous ;
où R¹² est un hydrogène, un alkyle en C₁₋₄ ou un haloalkyle en C₁₋₄ ;
où X est un NR³ , O, S ou (CR²²-R²²)ₙ où R²² est indépendamment un élément parmi un halogène, un alkyle en C₁₋₁₂, un alcényle en C₁₋₁₂, un alcynyle en C₁₋₁₂, un haloalkyle en C₁₋₁₂, un carbocyclyle en C₆₋₁₂, un hétérocyclyle en C₅₋₁₂ , un (CH₂)ₙOR⁵, un (CH₂)ₙNR⁵_{2,} OR⁵, SR⁵ , NO₂, CN, NR⁵₂, NR⁵COR⁵, NR⁵CONR⁵₂, NR⁵COR⁵, NR⁵CO₂R⁵, CO₂R⁵ , COR⁵, CONR⁵₂, S(O)₂R⁵, SONR⁵2, S(O)R⁵, SO₂NR⁵2, ou NR⁵S(O)₂R⁵ où chaque R⁵ peut être identique ou différent et est comme défini ci-dessus ; et
dans lequel n est égal à 1 à 6, de préférence n est égal à 1, 2, 3 ou 4 ;
et dans lequel R³ est une paire d'électrons célibataire, un hydrogène, un alkyle en C₁₋₁₂, un alcényle en C₂₋₁₂, un alcynyle en C₂₋₁₂, un carbocyclyle en C₃₋₁₂ ou un hétérocyclyle en C₃₋₁₂, chacun d'eux étant éventuellement substitué, dans lequel :
le groupe carbocyclyle ou hétérocyclyle éventuellement substitué est éventuellement fondu à un à trois cycles à cinq à sept éléments, insaturés, partiellement insaturés ou totalement saturés, contenant zéro à trois hétéroatomes,
chaque atome de carbone substituable en R³ comprenant le cycle fondu optionnel, est éventuellement et indépendamment substitué par un ou plusieurs éléments parmi un alkyle en C₁₋₁₂, un cycloalkyle en C₃₋₁₂, un hétérocycloalkyle en C₃₋₁₂, un aryle en C₃₋₁₂, un hétéroaryle halogène en C₃₋₁₂, un haloalkyle en C₁₋₁₂, un OR¹³, SR¹³, NO₂ , CN, NR¹³R¹³, NR¹³COR¹³, NR¹³CONR¹³R¹³ , NR¹³COR¹³, NR¹³CO₂R¹³, CO₂R¹³, COR¹³, CONR¹³R¹³, S(O)₂R¹³, SON₂, S(O)R¹³, SO₂NR¹³R¹³, NR¹³S(O)₂R¹³ , où chaque R¹³ peut être identique ou différent et est comme défini ci-dessus et dans lequel :
le groupe alkyle en C₁₋₁₂ comprend éventuellement une ou deux insertions choisies dans le groupe constitué de -O-, -C(O)-, -N(R¹³)-, -S(O)-, et -S(O₂)- où chaque R¹³ peut être identique ou différent et est comme défini ci-dessus ;
les groupes alkyle en C₁₋₁₂, cycloalkyle en C₃₋₁₂, hétérocycloalkyle en C₃₋₁₂, aryle en C₃₋₁₂, ou hétéroaryle en C₃₋₁₂ sont éventuellement substitués par un ou plusieurs éléments parmi un halogène, un haloalkyle en C₁₋₁₂, un OR¹³, SR¹³ , NO₂, CN, NR¹³R¹³ , NR¹³COR¹³ , NR¹³CONR¹³R¹³, NR¹³COR¹³ , NR¹³CO₂R¹³ , CO₂R¹³, COR¹³, CONR¹³R¹³, S(O)₂R¹³, SONH₂, S(O)R¹³ , SO₂NR¹3R¹³, NR¹³S(O)₂R¹³, où chaque R¹³ peut être identique ou différent et est comme défini ci-dessous et
les groupes cycloalkyle en C₃₋₁₂. hétérocycloalkyle en C₃₋₁₂, aryle en C₃₋₁₂, ou hétéroaryle en C₃₋₁₂ sont éventuellement substitués par un ou plusieurs groupes alkyle en C₁₋₁₂ ;
chaque atome de carbone saturé en R², comprenant le cycle fondu optionnel, est en outre éventuellement et indépendamment substitué par =O, =S, NNR¹³R¹³, =N-OR¹³, =NNHCOR¹³, =NNHCO₂R¹³, =NNSO₂R¹³, ou =NR¹³, où chaque R¹³ peut être identique ou différent et est comme défini ci-dessous ; et
chaque atome d'azote substituable en R³ est éventuellement substitué par R¹⁴, COR¹³, SO₂R¹³, ou CO₂R¹³, où chaque R¹³ et R¹⁴ peut être identique ou différent et est comme défini ci-dessous ;
où deux R¹³ dans NR¹³₂ peuvent éventuellement former un cycle à trois à sept éléments partiellement saturé, insaturé ou totalement saturé contenant un à trois hétéroatomes, éventuellement et indépendamment substitués par un ou plusieurs éléments parmi un alkyle en C₁₋₆, un halogène, un haloalkyle en C₁₋₆, un OR¹⁵, SR¹⁵, NO₂, CN, NR¹⁵R¹⁵, NR¹⁵COR¹⁵ , NR¹⁵CONR¹⁵R¹⁵, NR¹⁵COR¹⁵ , NR¹⁵CO₂R¹⁵, CO₂R¹⁵, COR¹⁵, CONR¹⁵, S(O)₂R¹⁵, SONR¹⁵₂, S(O)R¹⁵, SO₂NR¹⁵R¹⁵, NR¹⁵S(O)₂R¹⁵,
où le groupe alkyle en C₁₋₆ comprend éventuellement une ou deux insertions choisies dans le groupe constitué de -O-, -N(R¹⁵)-, -S(O)-, et -S(O₂)- où chaque R¹⁵ peut être identique ou différent et est comme défini ci-dessous ;
où R¹⁵ est un hydrogène, un alkyle en C₁₋₆, ou un haloalkyle en C₁₋₆ ;
où R¹³ est un hydrogène, un alkyle en C₁₋₁₂ ou un aryle en C₃₋₁₂, éventuellement substitué par un ou plusieurs éléments parmi un alkyle en C₁₋₄, un halogène, un haloalkyle en C₁-₄, un OR¹⁶, SR¹⁶, NO₂, CN, NR¹⁶R¹⁶, NR¹⁶COR¹⁶ , NR¹⁶CONR¹⁶R¹⁶, NR¹⁶COR¹⁶, NR¹⁶CO₂R¹⁶ , CO₂R¹⁶, COR¹⁶ , CONR¹⁶₂, S(O)₂R¹⁶, SONH₂, S(O)R¹⁶, SO₂NR¹⁶R¹⁶, NR¹⁶S(O)₂R¹⁶, où le groupe alkyle en C₁₋₁₂ comprend éventuellement une ou deux insertions choisies dans le groupe constitué de -O-, -N(R¹⁶)-, -S(O)-, et -S(O₂)- où chaque R¹⁶ peut être identique ou différent et est comme défini ci-dessous ;
où R¹⁴ est un alkyle en C₁₋₁₂ ou un aryle en C₃₋₁₂, éventuellement substitué par un ou plusieurs éléments parmi un alkyle en C₁₋₄, un halogène, un haloalkyle en C₁₋₄, un OR¹⁶, SR¹⁶, NO₂, CN, NR¹⁶R¹⁶, NR¹⁶COR¹⁶ , NR¹⁶CONR¹⁶R^{16,} NR¹⁶COR¹⁶, NR¹6CO₂R¹⁶, CO₂R¹⁶, COR¹⁶, CONR¹⁶₂, S(O)₂R¹⁶, SONH₂, S(O)R¹⁶, SO₂NR¹⁶R¹⁶, NR¹⁶S(O)₂R¹⁶, où le groupe alkyle en C₁₋₁₂ comprend éventuellement une ou deux insertions choisies dans le groupe constitué de -O-, -N(R¹⁶)-, -S(O)-, et -S(O₂)- où chaque R¹⁶ peut être identique ou différent et est comme défini ci-dessous ;
où R¹⁶ est un hydrogène, un alkyle en C₁₋₄ ou un haloalkyle en C₁₋₄ ;
où, quand X est un NR², R² et R³ peuvent former un cycle hétérocyclyle à 3 à 12 éléments, de préférence un cycle à 5, 6, 7, 8, 9, 10, 11 ou 12 éléments, dans lequel ledit cycle peut être partiellement saturé, insaturé ou totalement saturé, contenant un à trois hétéroatomes ; dans lequel le groupe hétérocyclique formé par R² et R³ peut être éventuellement fondu à un à trois cycles à 5 à 7 éléments, insaturés, partiellement saturés ou totalement insaturés contenant zéro à trois hétéroatomes, l'un quelconque desdits cycles étant éventuellement et indépendamment substitué avec un ou plusieurs éléments parmi un alkyle en C₁₋₆, un halogène un haloalkyle en C₁₋₆, OR²², SR²², NO₂, CN, NR²²R²², NR²²COR²², NR²²CONR²²R²², NR²²COR²², NR²²CO₂R²², CO₂R²², COR²², CONR²²₂, S(O)₂R²², SONR²²2, S(O)R²² , SO₂NR²²R²², NR²²S(O)₂R²², où le groupe alkyle en C₁₋₆ comprend éventuellement une ou deux insertions choisies dans le groupe constitué de -O-, -N(R²²)-, -S(O)-, et -S(O₂)- et où chaque R²² peut être identique ou différent ;
où R⁴ est un groupe carbocyclyle à six éléments ou un groupe hétérocyclyle à cinq ou six éléments contenant de 1 à 4 hétéroatomes indépendamment choisis parmi un N, S ou O, dans lequel le groupe carbocyclyle à six éléments éventuellement substitué ou un groupe hétérocyclyle à cinq ou six éléments est éventuellement fondu à un cycle à cinq à sept éléments partiellement saturés, insaturés ou totalement saturés, contenant zéro à trois hétéroatomes, et chaque atome de carbone ou hétéroatome dans R⁴ comprenant le cycle fondu éventuel, est éventuellement et indépendamment substitué par un ou plusieurs éléments parmi un halogène, alkyle en C₁₋₁₂, un alcényle en C₂₋₁₂, un alcynyle en C₂₋₁₂, un haloalkyle en C₁₋₁₂, un carbocyclyle en C₃₋₁₂, un hétérocyclyle en C₃₋₁₂, un (CH₂)ₙOR¹⁷, CH₂)ₙNR¹⁷₂, OR¹⁷, SR¹⁷, NO₂, CN, NR¹⁷2, NR¹⁷COR¹⁷, NR¹⁷CONR¹⁷₂, NR¹⁷COR¹⁷, NR¹⁷CO₂R¹⁷ CO₂R¹⁷, COR¹⁷, CONR¹⁷₂, S(O)₂R¹⁷, SONR¹⁷₂, S(O)R¹⁷, SO₂NR¹⁷₂, NR¹⁷S(O)₂R¹⁷, où le groupe alkyle en C₁₋₁₂ comprend éventuellement une ou deux insertions choisies dans le groupe constitué de -O-, -N(R¹²)--S-, -S(O)-, et -S(O₂) - ; et chaque atome de carbone saturé dans le cycle éventuellement fondu est en outre éventuellement et indépendamment substitué par =O, =S, NNR¹⁸₂, =N-OR¹⁸, =NNR¹⁸COR¹⁸, =NNR¹⁸CO₂R¹⁸, =NNS_{O}R¹⁸, ou =NR¹⁸ ; et chaque atome d'azote substituable en R⁴ est éventuellement substitué par R¹⁹, COR¹⁹, SO₂R¹⁹ ou CO₂R¹⁹, où n est égal à 1 à 6, de préférence n est égal à 1, 2 ou 3 ; de préférence, dans lequel chaque atome de carbone substituable ou hétéroatome en R⁴ est éventuellement et indépendamment substitué par un ou plusieurs éléments parmi un alkyle en C₁₋₆, OR²⁰, SR²⁰, NO₂ CN, NR²⁰₂, NR²⁰COR²⁰, NR²⁰CONR²⁰₂, NR²⁰COR²⁰, NHCO₂R²⁰, CO₂R²⁰, COR²⁰, CONR²⁰₂, S(O)₂R²⁰, SONR²⁰₂, S(O)R²⁰, SO₂NR²⁰₂, NR²⁰S(O) ₂R²⁰;
où R²⁰ est un hydrogène, un alkyle en C₁₋₆, ou un haloalkyle en C₁₋₆ ;
où R¹⁷ est un hydrogène, un alkyle en C₁₋₁₂, un carbocyclyle en C₃₋₁₂ ou un hétérocyclyle en C₃₋₁₂, éventuellement substitué par un ou plusieurs éléments parmi un alkyle en C₁₋₆, un carbocyclyle en C₃₋₁₂, un hétérocyclyle en C₃₋₁₂, un halogène, un haloalkyle en C₁₋₆, un OR²¹, SR²¹, NO₂, CN, NR²¹R²¹, NR²¹COR²¹, NR²¹CONR²¹R²¹, NR²COR²¹, NR²¹CO₂R²¹, CO₂R²¹, COR²¹, CONR²¹₂, S(O)₂R²¹, SONR²¹₂, S(O)R²¹, SO₂NR²¹R²¹, NR²¹S(O) ₂R²¹, où le groupe alkyle en C₁₋₁₂ comprend éventuellement une ou deux insertions choisies dans le groupe constitué de -O-, -N(R²¹)-, -S(O)-, et -S(O₂)- où chaque R²¹ peut être identique ou différent et est comme défini ci-dessous ;
dans lequel deux R¹⁷ dans NR¹⁷₂ peuvent éventuellement former un cycle à trois à sept éléments partiellement saturé, insaturé ou totalement saturé contenant un à trois hétéroatomes, éventuellement et indépendamment substitués par un ou plusieurs éléments parmi un alkyle en C₁₋₆, un halogène, un haloalkyle en C₁₋₆, un OR²¹, SR²¹, NO₂, CN, NR²¹_{R}²¹, NR²¹COR²¹, NR²¹CONR²¹R²¹, NR²¹COR²¹, NR²¹CO₂R²¹, CO₂R²¹, COR²¹, CONR²¹₂, S(O)₂R²¹, SONR²¹₂, S(O)R²¹, SO₂NR²¹R²¹, NR²¹S (O)₂R²¹, où le groupe alkyle en C₁₋₆ comprend éventuellement une ou deux insertions choisies dans le groupe constitué de -O-, -N(R²¹)-, -S(O)-, et -S(O₂) - où chaque R²¹ peut être identique ou différent et est comme défini ci-dessous ;
où R¹⁸ est un hydrogène, un alkyle en C₁₋₁₂, un carbocyclyle en C₃₋₁₂ ou un hétérocyclyle en C₃₋₁₂, éventuellement substitué par un ou plusieurs éléments parmi un alkyle en C₁₋₆, un halogène, un haloalkyle en C₁₋₆, un OR²¹, SR²¹, NO₂, CN, NR²¹R²¹, NR²¹COR²¹, NR²¹CONR²¹R²¹, NR²¹COR²¹, NR²¹CO₂R²¹, CO₂R²¹, COR²¹, CONR²¹₂, S(O)₂R²¹, S(O)R²¹, SO₂NR²¹R²¹, NR²¹S(O)₂R²¹, où le groupe alkyle en C₁₋₁₂ comprend éventuellement une ou deux insertions choisies dans le groupe constitué de -O-, -N(R²¹)-, -S(O)-, et -S(O₂)- où chaque R²¹ peut être identique ou différent et est comme défini ci-dessous ;
où R¹⁹ est un hydrogène, un aryle en C₆₋₁₂, un alkyle en C₁₋₆, ou un haloalkyle en C₁₋₆ ;
où R²¹ est un hydrogène, un alkyle en C₁₋₆, ou un haloalkyle en C₁₋₆.

2. Composé selon la revendication 1, dans lequel R¹ est un groupe carbocyclyle ou hétérocyclyle à cinq à six éléments éventuellement substitué, choisi parmi un phényle éventuellement substitué, l'acridine, le benzimidazole, le benzofurane, le benzothiophène, le benzoxazole, le benzothiazole, le cyclohexyl furane, l'imidazole, l'indole, l'isoindole, l'isoquinoline, l'isoxazole, l'isothiazole, la morpholine, la naphtaline, l'oxazole, la phénazine, la phénothiazine, la phénoxazine, la pipérazine, la pipéridine, le pyrazole, la pyridazine, la pyridine, le pyrrole, la quinoline, la quinolizine, le tétrahydrofurane, la tétrazine, le tétrazole, le thiophène, le thiazole, la thiomorpholine, le thianaphtalène, le thiopyrane, la triazine, le triazole ou le trithiane.

3. Composé selon la revendication 1 ou la revendication 2, dans lequel R¹ est un groupe de formule (II), où X est un groupe NR³, Y est absent et un ou plusieurs éléments parmi R² et R³ est (sont) un hydrogène, un alkyle ou un cycloalkyle.

4. Composé selon la revendication 3, dans lequel le groupe de formule (II) est un groupe alkylamino ou cycloalkylamino de préférence choisi parmi un méthylamino, un éthylamino, un propylamino, un isopropylamino, un butylamino, un cyclobutylamino, un pentylamino, un cyclopentylamino, un hexylamino, un cyclohexylamino, un heptylamino, un cycloheptylamino, un octylamino et un cyclooctylamino.

5. Composé selon l'une quelconque des revendications 1 à 4, dans lequel R¹ est substitué par un ou plusieurs éléments parmi OR²⁴, un halogène, un alkyle en C₁₋₆, un alcényle en C₁₋₆, un alcynyle en C₁₋₆, un haloalkyle en C₁₋₆, un alkylaryle en C₁₋₆, un alkylhétérocyclyle en C₁₋₆, un (CH₂)ₙOR²⁴, un (CH₂)ₙNR²⁴₂, un SR²⁴, NO₂, CN, NR²⁴₂, CO₂R²⁴, NR²⁴C(O)R²⁴, NR²⁴S(O)₂R²⁴, COR²⁴, CONR²⁴₂, S(O)₂R²⁴, S(O)R²⁴, ou SO₂NR²⁴₂ ;
où R²⁴ est un hydrogène, un alkyle en C₁₋₄, ou un aryle en C₆₋₁₂, de préférence un phényle, ou un hétérocyclyle en C₅₋₁₂. de préférence la pyridine, et n est égal à 1, 2, 3, 4, 5 ou 6 ;
où deux R²⁴ dans NR²⁴2 peuvent éventuellement former un cycle à trois à sept éléments partiellement saturé, insaturé ou totalement saturé contenant un à trois hétéroatomes, ledit cycle étant de préférence indépendamment substitué par un ou plusieurs éléments parmi un halogène, un alkyle en C₁₋₁₂, un alcényle en C₂₋₁₂, un alcynyle en C₂₋₁₂, un haloalkyle en C₁₋₁₂, un carbocyclyle en C₃₋₁₂, un hétérocyclyle en C₃₋₁₂, un OR²⁵, SR²⁵, NO₂, CN, NR²⁵₂, NR²⁵COR²⁵, NR²⁵CONR²⁵₂, NR²⁵COR²⁵, NR²⁵CO₂R²⁵ , CO_{2R}²⁵, COR²⁵ , CONR²⁵₂, S(O)₂R²⁵ , SONR²⁵₂, S(O)R²⁵, SO₂NR²⁵2, ou NR²⁵S(O)₂R²⁵; et chaque atome de carbone saturé dans le cycle optionnel est en outre éventuellement et indépendamment substitué par =O, =S, NNR²⁶2, =N-OR²⁶, =NNR²⁶COR²⁶, =NNR²⁶CO₂R²⁶ , =NNSO₂R²⁶ , ou =NR²⁶ ; et chaque atome d'azote substituable est éventuellement substitué par R²⁷, COR²⁷, SO₂R²⁷, ou CO₂R²⁷;
où R²⁵ est un hydrogène, un alkyle en C₁₋₁₂, un carbocyclyle en C₆₋₁₂ ou un hétérocyclyle en C₅₋₁₂, éventuellement substitué par un ou plusieurs éléments parmi un alkyle en C₁₋₆, un halogène, un haloalkyle en C₁₋₆, un OR²⁸, SR²⁸, NO₂, CN, NR²⁸R²⁸, NR²⁸COR²⁸, NR²⁸CONR²⁸R²⁸, NR²⁸COR²⁸, NR²⁸CO₂R²⁸, CO₂R²⁸, COR²⁸, CONR²⁸₂, S(O)₂R²⁸, SONR²⁸₂, S(O)R²⁸, SO₂NR²⁸R²⁸, NR²⁸S(O)₂R²⁸, dans lequel le groupe alkyle en C₁₋₁₂ comprend éventuellement une ou deux insertions choisies dans le groupe constitué de -O-, -N(R²⁸)-, -S(O)- et -S(O₂)-, où chaque R²⁸ peut être identique ou différent et est comme défini ci-dessous ;
où R²⁶ est un hydrogène, un alkyle en C₁₋₁₂, un carbocyclyle en C₆₋₁₂ ou un hétérocyclyle en C₅₋₁₂, éventuellement substitué par un ou plusieurs éléments parmi un alkyle en C₁₋₆, un halogène, un haloalkyle en C₁-6, un OR²⁸, SR²⁸, NO₂, CN, NR²⁸R²⁸, NR²⁸COR²⁸, NR²⁸CONR²⁸R²⁸, NR²⁸COR²⁸, NR²⁸CO₂R²⁸, CO₂R²⁸, COR²⁸, CONR²⁸₂, S(O)₂R²⁸, S(O)R²⁸ ,SO₂NR²⁸R²⁸, NR²⁸S(O)₂R²⁸, dans lequel le groupe alkyle en C₁₋₁₂ comprend éventuellement une ou deux insertions choisies dans le groupe constitué de -O-, -N(R²⁸)-, -S(O)- et -S(O₂)-, où chaque R²⁸ peut être identique ou différent et est comme défini ci-dessous ;
où R²⁷ est un hydrogène, un alkyle en C₁₋₆, un haloalkyle en C₁₋₆, ou un aryle en C₆₋₁₂ ;
où R²⁸ est un hydrogène, un alkyle en C₁₋₆, ou un haloalkyle en C₁₋₆.

6. Composé selon l'une quelconque des revendications 1 à 5, dans lequel R⁴ est choisi parmi un phényle, un cyclohexyle, l'acridine, le benzimidazole, le benzofurane, le benzothiophène, le benzoxazole, le benzothiazole, l'indole, l'isoindole, l'indolizine, l'indazole, l'isoindole, l'isoquinoline, la morpholine, le naphtalène, la phénazine, le phénothiazine, la phénoxazine, la pipérazine, la pipéridine, la pyridazine, la pyridine, le pyrimidinyle, le pyrazinyle, la quinoline, la quinolizine, la tétrazine, la thiomorpholine, le thianaphtalène, le thiopyrane, la triazine, le trithiane, le furane, l'imidazole, l'isoxazole, l'isothiazole, l'oxazole, l'oxadiazole, l'oxathiazole, le pyrazole, le pyrrole, le tétrazole, le thiophène, le thiadiazole, le thiatriazole, le thiazole ou le triazole, dans lequel chaque atome de carbone substituable ou hétéroatome dans R⁴ est éventuellement et indépendamment substitué par un ou plusieurs éléments parmi un alkyle en C₁₋₆, un OR²⁰, SR²⁰, NO₂, CN, NR²⁰2, NR²⁰COR²⁰, NR²⁰CONR²⁰₂, NR²⁰COR²⁰, NHCO₂R²⁰, CO₂R²⁰, COR²⁰, CONR²⁰₂, S(O)₂R²⁰, SONR²⁰₂, S(O)R²⁰, SO₂NR²⁰2 ou NR²⁰S(O)₂R²⁰ ;
où R²⁰ est un hydrogène, un alkyle en C₁₋₆ ou un haloalkyle en C₁₋₆.

7. Composé selon l'une quelconque des revendications 1 à 6, dans lequel R⁴ est un groupe carbocyclyle ou hétérocyclyle à six éléments éventuellement substitué par un ou plusieurs éléments parmi un OR²⁹ , NR²⁹ , SR²⁹ , (CH₂)ₙOR²⁹ , (CH₂)ₙNR²⁹₂ , un halogène, un alkyle en C₁₋₆, un alcényle en C₂₋₆, un alcynyle en C₂₋₆, un haloalkyle, un NO₂, un CN, NR²⁹C(O)R²⁹, NR²⁹S(O)₂R²⁹, CO₂R²⁹, COR²⁹, CONR²⁹₂, S(O)₂R²⁹, S(O)R²⁹ ou SO₂NR²⁹₂ ;
où R²⁹ est un hydrogène, un alkyle en C₁₋₄, un hétérocyclyle en C₅₋₁₂, ou un aryle en C₆₋₁₂ de préférence un phényle, et n est égal à 1, 2, 3, 4, 5 ou 6 ;
où deux R²⁹ dans NR²⁹₂ peuvent éventuellement former un cycle à cinq à sept éléments partiellement saturé, insaturé ou totalement saturé contenant un à trois hétéroatomes, éventuellement et indépendamment substitués par un ou plusieurs éléments parmi un halogène, un alkyle en C₁₋₁₂, un alcényle en C₂₋₁₂, un alcynyle en C₂₋₁₂, un haloalkyle en C₁₋₁₂, un carbocyclyle en C₆₋₁₂, un hétérocyclyle en C₁₋₁₂, un OR³⁰, SR³⁰, NO₂, CN, NR³⁰₂, NR³⁰COR³⁰, NR³⁰CONR³⁰₂, NR³⁰COR³⁰, NR³⁰CO₂R³⁰, CO₂R³⁰, COR³⁰, CONR³⁰₂, S(O)₂R³⁰, SONR³⁰₂, S(O)R³⁰, SO₂NR³⁰₂, ou NR³⁰S(O)₂R³⁰ ; et chaque atome de carbone saturé dans le cycle optionnel est en outre éventuellement et indépendamment substitué par =O, =S , NNR³¹2 , =N-OR³¹, =NNR³¹COR³¹, =NNR³¹CO₂R³¹, =NNSO₂R³¹, ou =NR³¹ ; et chaque atome d'azote substituable est éventuellement substitué par R³², COR³², SO₂R³², ou CO₂R32 ;
où R³⁰ est un hydrogène, un alkyle en C₁₋₁₂, un carbocyclyle en C₆₋₁₂ ou un hétérocyclyle en C₅₋₁₂, éventuellement substitué par un ou plusieurs éléments parmi un alkyle en C₁₋₆, un halogène, un haloalkyle en C₁₋₆, un OR³³ , SR³³ , NO₂ , CN, NR³³COR³³ , NR³³CONR³³R³³, NR³³COR³³ , NR³³CO₂R³³ , CO₂R³³, COR³³ , CONR³³₂ , S(O)₂R³³, SONR³³₂, S(O)R³³ , SO₂NR³³R³³, NR³³S(O)₂R³³ , dans lequel le groupe alkyle en C₁₋₁₂ comprend éventuellement une ou deux insertions choisies dans le groupe constitué de -O-, -N(R³³)-, -S(O)- et -S(O₂)-, où chaque R³³ peut être identique ou différent et est comme défini ci-dessous ;
où R³¹ est un hydrogène, un alkyle en C₁₋₁₂, un carbocyclyle en C₆₋₁₂ ou un hétérocyclyle en C₅₋₁₂, éventuellement substitué par un ou plusieurs éléments parmi un alkyle en C₁₋₆, un halogène, un haloalkyle en C₁₋₆, un OR³³, SR³³, NO₂, CN, NR³³R³³, NR³³COR³³, NR³³CONR³³R³³, NR³³COR³³, NR³³CO₂R³³, CO₂R³³, COR³³, CONR³³₂, S(O)₂R³³ , S(O)R³³, SO₂NR³³R³³, NR³³S(O)₂R³³, dans lequel le groupe alkyle en C₁₋₁₂ comprend éventuellement une ou deux insertions choisies dans le groupe constitué de -O-, -N(R³³), -S(O)- et -S(O₂)-, où chaque R²¹ peut être identique ou différent et est comme défini ci-dessous ;
où R³² est un hydrogène, un aryle en C₆₋₁₂, un alkyle en C₁₋₆, ou un haloalkyle en C₁₋₆ ;
où R³³ est un hydrogène, un alkyle en C₁₋₆, ou un haloalkyle en C₁₋₆.

8. Composé selon l'une quelconque des revendications 1 à 7, dans lequel R⁴ est un hétérocyclyle à cinq éléments 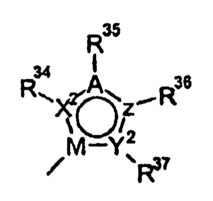
où A, X², Y² ou Z sont indépendamment choisis parmi un N, O, C, S et M est un C ou N, où un, deux, trois ou quatre éléments parmi A, X², Y², Z et M sont différents de C ; R³⁴, R³⁵, R³⁶ ou R³⁷ sont indépendamment choisis parmi une paire d'électrons célibataire, un hydrogène, un halogène, un alkyle en C₁₋₁₂, un haloalkyle en C₁₋₁₂, un OR³⁸, SR³⁸, NO₂, CN, NR³⁸₂ ; NR³⁸COR³⁸, NR³⁸CONR³⁸₂, NR³⁸COR³⁸, NR³⁸CO₂R³⁸, (CH₂)ₙOR³⁸, (CH₂) ₙNR³⁸₂, CO₂R³⁸, COR³⁸, CONR³⁸2, S(O)₂R³⁸, SONR³⁸₂, S(O)R³⁸, SO₂NR³⁸₂, ou NHS(O)2R³⁸ ;
où n est égal à 1 à 6, de préférence n est égal à 1, 2 ou 3 ;
ou dans lequel deux éléments quelconques parmi R³⁴, R³⁵, R³⁶ ou R³⁷ peuvent éventuellement former un cycle à cinq à sept éléments partiellement saturé, insaturé ou totalement saturé, contenant zéro à trois hétéroatomes, chaque atome de carbone saturé dans le cycle fondu optionnel étant en outre éventuellement et indépendamment substitué par un ou plusieurs éléments parmi un halogène, un alkyle en C₁₋₁₂, un alcényle en C₂₋₁₂, un alcynyle en C_{2-12,} un haloalkyle en C₁₋₁₂, un carbocyclyle en C₆₋₁₂, un hétérocyclyle en C₅₋₁₂, un OR³⁸, SR³⁸, NO₂, CN, NR³⁸₂, NR³⁸CONR³⁸2, NR³⁸COR³⁸, NR³⁸CO₂R³⁸, (CH₂)ₙOR³⁸, (CH₂)ₙNR³⁸₂, CO₂R³⁸ , CONR³⁸₂ , SONR³⁸₂, S(O)R³⁸ , SO₂NR³⁸₂ , ou NR³⁸S(O)₂R³⁸ ; et chaque atome de carbone saturé dans le cycle fondu optionnel est en outre éventuellement et indépendamment substitué par un =O, =S, NNR³⁹₂, =N-OR³⁹, =NNR³⁹COR³⁹, =NNR³⁹CO₂R³⁹, =NNSO₂R³⁹, ou =NR³⁹ ; et chaque atome d'azote substituable dans R⁴ est éventuellement substitué par R⁴⁰, COR⁴⁰, SO₂R⁴⁰, ou CO₂R⁴⁰ ;
où n est égal à 1 à 6, de préférence n est égal à 1, 2 ou 3 ;
où R³⁸ est un hydrogène, un alkyle en C₁₋₁₂, un carbocyclyle en C₆₋₁₂, ou un hétérocyclyle en C₅₋₁₂, éventuellement substitué par un ou plusieurs éléments parmi un alkyle en C₁₋₆, un halogène, un haloalkyle en C₁₋₆, un OR⁴¹ , SR⁴¹ , NO₂ , CN, NR⁴¹R⁴¹ , NR⁴¹CONR⁴¹R⁴¹ , NR⁴¹COR⁴¹ , NR⁴¹CO₂R⁴¹, CO₂R⁴¹, COR ⁴¹, CONR⁴¹₂, S(O)₂R⁴¹, SONR⁴¹_{2 ,} S(O)₂R⁴¹, SO₂NR⁴¹R⁴¹, ou NR⁴¹S(O)₂R⁴¹, où le groupe alkyle en C₁₋₁₂ comprend éventuellement une ou deux insertions choisies dans le groupe constitué de -O-, -N(R⁴¹)-, -S(O)- et -S(O₂)-, où chaque R⁴¹ peut être identique ou différent et est comme défini ci-dessous ;
où R³⁹ est un hydrogène, un alkyle en C₁₋₁₂, un carbocyclyle ou hétérocyclyle, éventuellement substitué par un ou plusieurs éléments parmi un alkyle en C₁₋₆, un halogène, un haloalkyle en C₁₋₆, un OR⁴¹, SR⁴¹, NO₂, CN, NR⁴¹R⁴¹ , NR⁴¹COR⁴¹ , NR⁴¹CONR⁴¹R⁴¹ , NR⁴¹COR⁴¹ , NR⁴¹CO₂R⁴¹ , CO₂R⁴¹, COR⁴¹, CONR⁴¹₂ , S(O)₂R⁴¹, S(O)R⁴¹, SO₂NR⁴¹R⁴¹, NR⁴¹ S(O)₂R⁴¹, où le groupe alkyle en C₁₋₁₂ comprend éventuellement une ou deux insertions choisies dans le groupe constitué de -O-, -N(R⁴¹) -, -S(O)- et -S(O₂)-, où chaque R⁴¹ peut être identique ou différent et est comme défini ci-dessous ;
où R⁴⁰ est un hydrogène, un alkyle en C₁₋₆, un haloalkyle en C₁₋₆ ou un aryle en C₆₋₁₂ ;
où R⁴¹ est un hydrogène, un alkyle en C₁₋₆ ou un haloalkyle en C₁₋₆.

9. Composé selon la revendication 8, dans lequel R⁴ est un furane, imidazole, isoxazole, isothiazole, oxazole, oxadiazole, oxatriazole, pyrazole, pyrrole, tétrazole, thiophène, thiadiazole, thiatriazole, thiazole ou triazole ; et R³⁴, R³⁵, R³⁶ ou R³⁷ sont indépendamment choisis parmi une paire d'électrons célibataires, un hydrogène, un halogène, un alkyle en C₁₋₆, un haloalkyle en C₁₋₆, un OR⁴², SR⁴², CN, NR⁴²₂, NR⁴²COR⁴², CO₂R⁴², COR⁴², CONR⁴²₂, S(O)₂R⁴² ou S(O)R⁴²;
où R⁴² est un hydrogène, un alkyle en C₁₋₄, de préférence un méthyle ou éthyle ou carbocyclyle, de préférence un phényle.

10. Composé selon l'une quelconque des revendications 1 à 9, choisi parmi

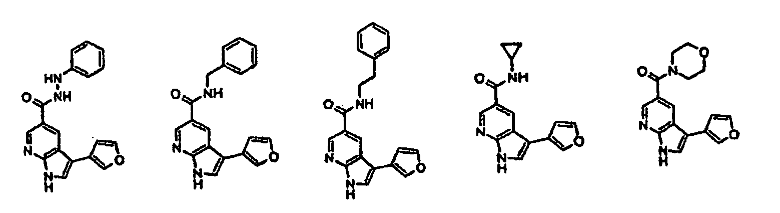

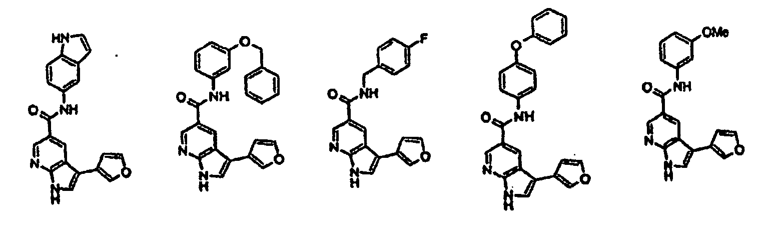

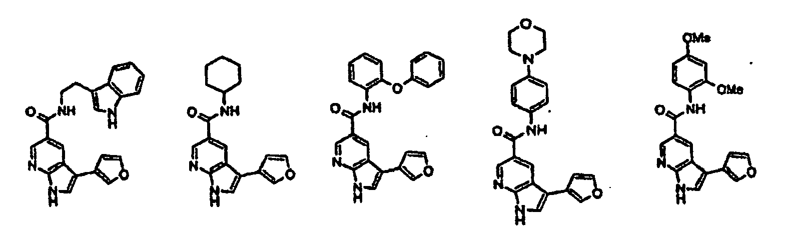

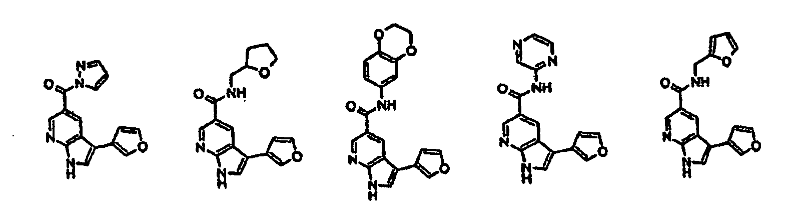

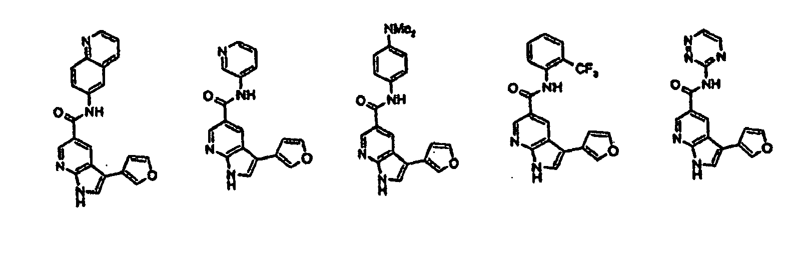

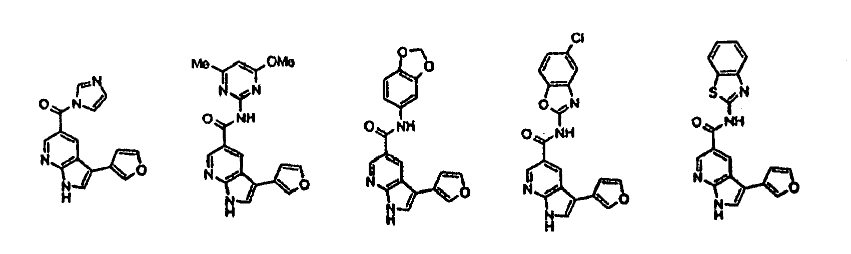

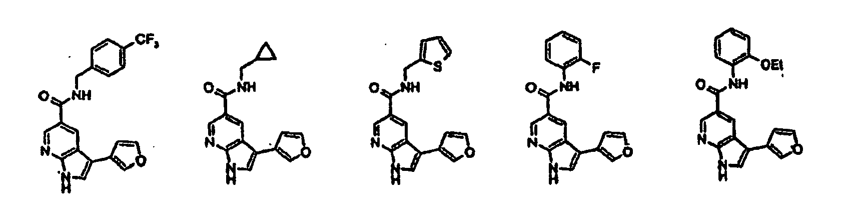

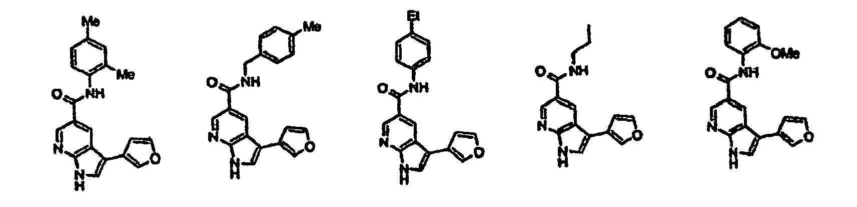

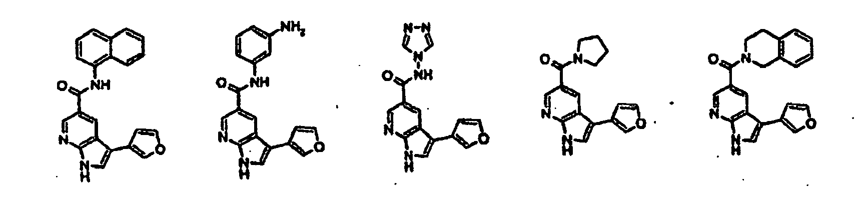

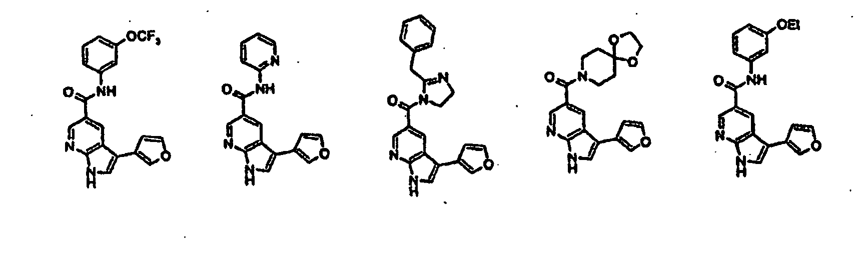

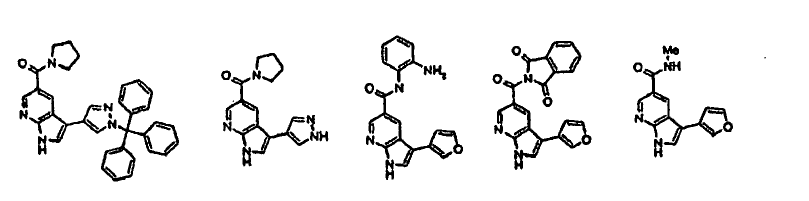

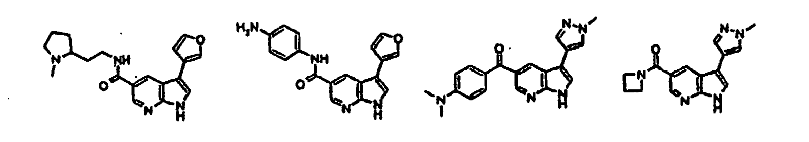

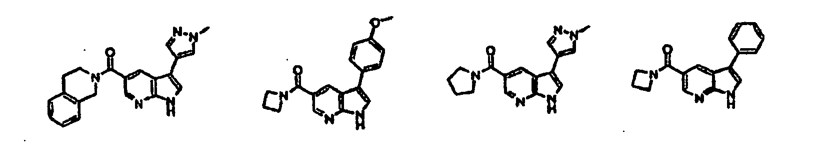

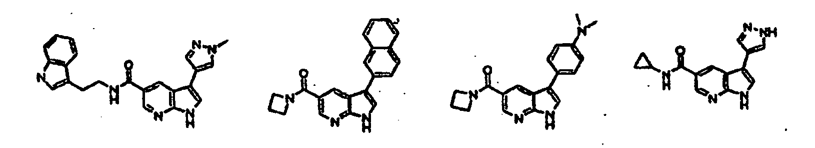

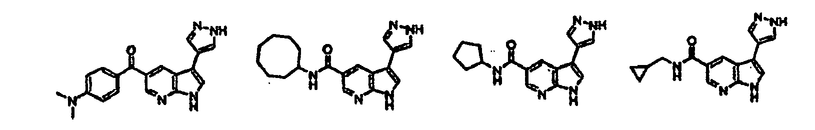

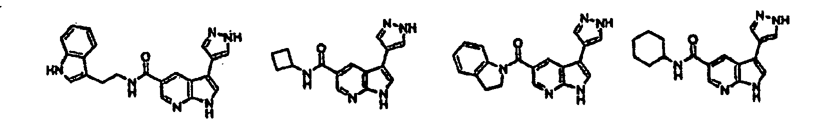

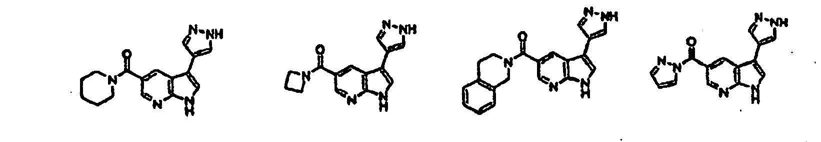

11. Procédé pour la fabrication d'un composé de formule (I) dans lequel R¹ est un groupe de formule (II), comme défini dans l'une quelconque des revendications 1 à 10 de l'invention comprenant la condensation d'un intermédiaire (III) avec un intermédiaire (IV) 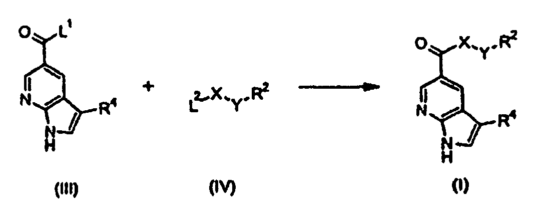 où R² et R⁴ sont comme définis dans l'une quelconque des revendications 1 à 10 ; et dans lequel chacun des L¹ et L² est indépendamment un groupe labile où L¹ et L² forment ensemble un produit de condensation.

12. Procédé selon la revendication 11 dans lequel L¹ est un OH, OR⁵⁰, OM, Cl, Br, ou I où R⁵⁰ est un alkyle en C₁₋₆, de préférence un méthyle ou un éthyle et M est un Na, Li, K, Ca, Mg ou Ba, et L² est un hydrogène ou M.

13. Composition pharmaceutique comprenant un composé selon l'une quelconque des revendications 1 à 10, en combinaison avec un support, diluant ou excipient pharmaceutiquement acceptable.

14. Composition selon la revendication 13, comprenant en outre un ou plusieurs éléments parmi un agent anti-inflammatoire, un antagoniste de récepteur AMPA, un agent chimiothérapeutique et/ou un agent antiprolifératif.

15. Procédé pour la fabrication d'une composition selon la revendication 13 ou la revendication 14, comprenant la combinaison d'un composé selon l'une quelconque des revendications 1 à 10 de l'invention avec un support ou diluant pharmaceutiquement acceptable et éventuellement avec un ou plusieurs des agents additionnels selon la revendication 14.

16. Composé selon l'une quelconque des revendications 1 à 10, ou composition selon la revendication 13 ou la revendication 14, à utiliser en médecine.

17. Composé selon l'une quelconque des revendications 1 à 10, ou composition selon l'une quelconque des revendications 13 ou 14, pour inhiber la JNK.

18. Composé selon l'une quelconque des revendications 1 à 10, ou composition selon l'une quelconque des revendications 13 ou 14, pour inhiber sélectivement la JNK3.

19. Composé selon l'une quelconque des revendications 1 à 10, ou composition selon l'une quelconque des revendications 13 ou 14, à utiliser dans la prévention ou le traitement d'un trouble provoqué par la JNK.

20. Composé ou composition selon la revendication 19, dans lequel le trouble est un trouble neurodégénératif (y compris la démence), une maladie inflammatoire, un trouble lié à l'apoptose, en particulier l'apoptose neuronale, une maladie autoimmune, un trouble osseux destructif, un trouble prolifératif, un cancer, une maladie infectieuse, une allergie, une lésion de reperfusion d'ischémie, une attaque cardiaque, un trouble angiogénique, une hypoxie organique, une hyperplasie vasculaire, une hypertrophie cardiaque, une agrégation plaquettaire provoquée par la thrombine, et/ou toute condition associée à l'endoperoxydase synthase-2 de prostaglandine.

21. Composé ou composition selon la revendication 20, dans lequel le trouble neurodégénératif résulte d'une apoptose et/ou d'une inflammation.

22. Composé ou composition selon la revendication 20 ou 21, dans lequel le trouble neurodégénératif est : la démence, la maladie d'Alzheimer, la maladie de Parkinson, la sclérose amyotrophique latérale, la maladie de Huntington, la chorée sénile, la chorée de Sydenham, l'hypoglycémie, le traumatisme crânien et de la moelle épinière comprenant une lésion par traumatisme crânien, une douleur aiguë et chronique, l'épilepsie et les attaques ; la démence olivo-ponto-cérébelleuse ; la mort des neurones ; la neurodégénérescence liée à l'hypoxie ; l'hypoxie aiguë ; la toxicité du glutamate comprenant la neurotoxicité de glutamate ; l'ischémie cérébrale ; la démence liée à la méningite et/ou la névrose ; la démence cérébrovasculaire ; ou la démence chez un patient infecté par le HIV.

23. Composé ou composition selon la revendication 20 ou 21, dans lequel le trouble neurodégénératif est une névropathie périphérique, comprenant la mononeuropathie, la mononeuropathie multiple ou la polyneuropathie, comprenant la mononeuropathie, une mononeuropathie ou polyneuropathie multiple, comme cela peut être trouvé dans le diabète sucré, la maladie de Lyme ou une urémie ; la névropathie périphérique provoquée par un agent toxique ; la maladie de démyélinisation comme une polyneuropathie inflammatoire aiguë ou chronique, les leucodystrophies ou syndrome de Guillain-Barré ; la mononeuropathie inflammatoire secondaire à un trouble vasculaire de collagène ; la mononeuropathie multiple secondaire à la sarcoïdose ; la mononeuropathie multiple secondaire à une maladie métabolique ; ou la mononeuropathie multiple secondaire à une maladie infectieuse.

24. Composé ou composition selon la revendication 20, dans lequel le trouble est une inflammation des intestins, une bronchite, l'asthme, la pancréatite aiguë, la pancréatite chronique, les allergies de différentes sortes, la maladie d'Alzheimer, une maladie auto-immune comme la polyarthrite rhumatoïde, le lupus érythémateux systémique, la glomérulonéphrite, la sclérodermie, la thyroïdite chronique, la maladie de Graves, la gastrite autoimmune, le diabète, l'anémie hémolytique auto-immune, la neutropénie auto-immune, la thrombocytopénie, la dermatite atopique, l'hépatite active chronique, la myasthénie grave, la sclérose en plaques, la rectocolite hémorragique, la maladie de Crohn, le psoriasis ou la maladie du greffon contre l'hôte.

25. Utilisation d'un composé selon la revendication 1 à 10, dans la fabrication d'un médicament pour la prévention ou le traitement d'un trouble provoqué par la JNK.
